# EUROPEAN PATENT APPLICATION

(11) **EP 4 592 383 A2**
(43) Date of publication of application: **30.07.2025**
(21) Application number: 25169778.5
(22) Date of filing: 28.06.2021
(51) Int. Cl.: C12N 5/0783

(54) **POLY-DONOR CD4+ T CELLS EXPRESSING IL-10 AND USES THEREOF**

(30) Priority: 30.06.2020 WO PCT/US2020/040372
(62) Divisional of application: 21748708.1
(71) Applicant: TR1X, Inc., San Diego, CA 92121 (US)
(72) Inventor: RONCAROLO, Maria, Grazia, La Jolla, 92037 (US); DE VRIES, Jan Egbert, La Jolla, 92037 (US)
(74) Representative: Mathys & Squire

(57) **Abstract**

The present disclosure provides a population of poly-donor CD4^{IL-10} cells generated by genetically modifying CD4⁺ T cells from at least three different T cell donors. Further provided are methods of generating the poly-donor CD4^{1L-10} cells and methods of using the poly-donor CD4^{IL-10} cells for immune tolerization, treating GvHD, cell and organ transplantation, cancer, and other immune disorders.

## Description

### 1. CROSS REFERENCE TO RELATED APPLICATIONS

The instant application claims priority to PCT application, PCT/US2020/040372 filed on June 30, 2020, which has been incorporated by reference in its entirety herein.

### 2. SEQUENCE LISTING

The instant application contains a Sequence Listing with 5 sequences.

### 3. BACKGROUND

Regulatory T cells belong to a small but important subset of T cells which maintain immunological tolerance to self and non-pathogenic antigens and maintain immune homeostasis. There are two major populations of regulatory T cells - CD4⁺, Foxp3⁺ CD25⁺ T cells (Foxp3⁺ cells) and type 1 regulatory T (Tr1) cells Both Foxp3⁺ and Tr1 cells downregulate pathogenic T-cell responses in various preclinical models for organ and pancreatic islet transplantation, GvHD and various autoimmune and inflammatory diseases.

Tr1 cells have shown to be effective in clinical studies. Administration of cloned, antigen-specific, autologous Tr1 cells to patients with ongoing moderate to severe Crohn's disease resulted in objective, transient remissions (Desreumaux et al., Gastroenterology. 2012;143(5):1207-1217.e2.). In addition, adoptive transfer of donor-derived allo-specific CD4⁺ T cell populations enriched for Tr1 cells to leukemia patients following allogeneic HSCT resulted in a rapid reconstitution of the immune system and protection against microbial and viral infections, without severe GvHD. In the responder patients, long term remissions and tolerance (> 7 years) resulting in cures were achieved (Bacchetta et al., Front Immunol. 2014;5:16).

Despite these encouraging results, the production of donor-derived or autologous Tr1 cells for large scale therapy for patients with high unmet medical needs is not always feasible, is very cumbersome, and also does not allow for the generation of large quantities of pure Tr1 cells.

Recently, Locafaro and colleagues circumvented some of these problems by transducing purified CD4⁺ T cells from a single donor with a bidirectional lentiviral vector containing a human IL-10 gene. The resulting single-donor CD4^{IL-10} populations shared the major functions of naturally occurring Tr1 cells. Like Tr1 cells, single-donor CD4^{IL-10} cells produce high levels of IL-10 and downregulate the proliferation of both allogeneic CD4⁺ and CD8⁺ T cells. In addition, they are cytotoxic for both normal myeloid cells (including antigen presenting cells, APC) and myeloid leukemia cells. These single-donor CD4^{IL-10} cells were shown to be effective in reducing GvHD in a humanized xeno-GvHD model while retaining graft-versus-leukemia (GvL) activity. *See* Locafaro et al. Mol Ther. 2017;25(10):2254-2269 and WO 2016/146,542.

Although it is possible to produce highly purified single-donor CD4^{IL-10} cells for therapeutic use, there are still significant limitations, because of qualitative and quantitative differences between the various individual batches, which most likely are related to intrinsic differences between the various donors in addition to variations in the quality of buffy coats.

### 4. SUMMARY

The present disclosure provides a new Tr1-based therapy using a population of polydonor CD4^{IL-10} cells. Poly-donor CD4^{IL-10} cells refer to CD4⁺ T cells obtained from at least three different T cell donors and then genetically modified to comprise an exogenous polynucleotide encoding IL-10. The T cell donors are third party donors who are neither a host to be treated with the poly-donor CD4^{IL-10} cells nor an HSC or organ transplant donor. The poly-donor CD4^{IL-10} cells are not alloantigen-specific, i.e., they have not been primed or stimulated with cells from the host before administration.

Applicant demonstrated that the poly-donor CD4^{IL-10} cells have cytokine production profiles, immune suppressive- and cytotoxic capabilities comparable to those of single-donor CD4^{1L-10} cells. In addition, *in vivo,* they are more effective in preventing xeno GvHD mediated by CD4⁺ T cells than single-donor CD4^{IL-10} cells, while they do not induce GvHD by themselves. Overall, the functional properties of these poly-donor CD4^{IL-10} cells both *in vitro* and *in vivo* were comparable to or better than those of single donor CD4^{IL-10} cells.

Based on these results, Applicant claims that poly-donor allogeneic CD4^{IL-10} cells can be used for therapeutic purposes in GvHD, cell and organ transplantation, autoimmune- and inflammatory diseases.

Further, by using third party T cells and eliminating the requirement of allo-specificity, poly-donor CD4^{IL-10} cells makes the Tr1-based cell therapy available to a larger population of patients with various genetic backgrounds.

Accordingly, the present disclosure provides a population of CD4⁺ T cells that have been genetically modified to comprise an exogenous polynucleotide encoding IL-10, wherein the CD4⁺ T cells were obtained from at least three different T cell donors (poly-donor CD4^{IL-10} cells).

In some embodiments, the CD4⁺ T cells were obtained from three, four, five, six, seven, eight, nine, or ten different T cell donors. In some embodiments, the CD4⁺ T cells in the population collectively have six, seven, eight, nine, ten, eleven, twelve, or more different HLA haplotypes.

In some embodiments, all the CD4⁺ T cells in the population have at least 5/10, 6/10, 7/10, 8/10, or 9/10 match at the HLA-A, HLA-B, HLA-C, HLA-DRB1, and HLA-DQB1 loci to each other. In some embodiments, all the CD4⁺ T cells in the population have at least 4/8, 5/8, 6/8, 7/8, or 8/8 match at the HLA-A, HLA-B, HLA-C, and HLA-DRB1 loci to each other. In some embodiments, all the CD4⁺ T cells in the population have 2/2 match at the HLA-A locus to each other. In some embodiments, all the CD4⁺ T cells in the population have 2/2 match at the HLA-B locus to each other. In some embodiments, all the CD4⁺ T cells in the population have 2/2 match at the HLA-C locus to each other. In some embodiments, all the CD4⁺ T cells in the population have at least 3/4 or 4/4 match at the HLA-DRB1 and HLA-DQB1 loci with each other. In some embodiments, all the CD4⁺ T cells in the population have an A*02 allele.

In some embodiments, none of the CD4⁺ T cells is immortalized. In some embodiments, the exogenous polynucleotide comprises an IL-10-encoding polynucleotide segment operably linked to expression control elements. In some embodiments, the IL-10 is a human IL-10. In some embodiments, the IL-10 is a viral IL-10. In some embodiments, the IL-10-encoding polynucleotide segment encodes a protein having the sequence of SEQ ID NO: 1. In some embodiments, the IL-10-encoding polynucleotide segment has the sequence of SEQ ID NO:2. In some embodiments, the expression control elements drive constitutive expression of the encoded IL-10. In some embodiments, the expression control elements drive expression of IL-10 in activated CD4⁺ T cells. In some embodiments, the expression control elements drive tissue-specific or CD4⁺ T cell-specific expression.

In some embodiments, the exogenous polynucleotide further comprises a sequence encoding a selection marker. In some embodiments, the selection marker is ΔNGFR. In some embodiments, the ΔNGFR has the sequence of SEQ ID NO: 3. In some embodiments, the exogenous polynucleotide comprises a sequence of SEQ ID NO:4. In some embodiments, the exogenous polynucleotide having a sequence of SEQ ID NO: 5.

In some embodiments, the selection marker is a truncated EGFR polypeptide. In some embodiments, the selection marker is a truncated human EGFR polypeptide.

In some embodiments, the exogenous polynucleotide is integrated into the T cell nuclear genome. In some embodiments, the exogenous polynucleotide is not integrated into the T cell nuclear genome. In some embodiments, the exogenous polynucleotide further comprises lentiviral vector sequences. In some embodiments, the exogenous polynucleotide is not integrated into the T cell nuclear genome.

In some embodiments, at least 90% of the CD4⁺ T cells within the population express IL-10. In some embodiments, at least 95% of the CD4⁺ T cells within the population express IL-10. In some embodiments, at least 98% of the CD4⁺ T cells within the population express IL-10.

In some embodiments, the genetically modified CD4⁺ T cells constitutively express at least 100pg IL-10 per 10⁶ of the CD4⁺ T cells/ml of culture medium. In some embodiments, the genetically modified CD4⁺ T cells constitutively express at least 200pg, 500pg, 1ng, 5ng, 10ng, or 50ng IL-10 per 10⁶ of the CD4⁺ T cells/ml. In some embodiments, the genetically modified CD4⁺ T cells express at least 1or 2ng IL-10 per 10⁶ of the CD4⁺ T cells/ml after activation with anti-CD3 and anti-CD28 antibodies. In some embodiments, the genetically modified CD4⁺ T cells express at least 2ng, 5ng, 10ng, 100ng, 200ng, or 500ng IL-10 per 10⁶ of the CD4⁺ T cells/ml after activation with anti-CD3 and anti-CD28 antibodies. In some embodiments, the genetically modified CD4⁺ T cells express IL-10 at a level at least 5-fold higher than unmodified CD4⁺ T cells. In some embodiments, the genetically modified CD4⁺ T cells express IL-10 at a level at least 10-fold higher than unmodified CD4⁺ T cells.

In some embodiments, at least 90% of the CD4⁺ T cells within the population express the selection marker from the exogenous polynucleotide. In some embodiments, at least 95% of the CD4⁺ T cells within the population express the selection marker from the exogenous polynucleotide. In some embodiments, at least 98% of the CD4⁺ T cells within the population express the selection marker from the exogenous polynucleotide.

In some embodiments, the genetically modified CD4⁺ T cells express CD49b. In some embodiments, the genetically modified CD4⁺ T cells express LAG-3. In some embodiments, the genetically modified CD4⁺ T cells express TGF-β. In some embodiments, the genetically modified CD4⁺ T cells express IFNγ. In some embodiments, the genetically modified CD4⁺ T cells express GzB. In some embodiments, the genetically modified CD4⁺ T cells express perforin. In some embodiments, the genetically modified CD4⁺ T cells express CD18. In some embodiments, the genetically modified CD4⁺ T cells express CD2. In some embodiments, the genetically modified CD4⁺ T cells express CD226. In some embodiments, the genetically modified CD4⁺ T cells express IL-22.

In some embodiments, the CD4⁺ T cells have not been anergized in the presence of peripheral blood mononuclear cells (PBMCs) from a host. In some embodiments, the CD4⁺ T cells have not been anergized in the presence of recombinant IL-10 protein, wherein the recombinant IL-10 protein is not expressed from the CD4⁺ T cells. In some embodiments, the CD4⁺ T cells have not been anergized in the presence of DC10 cells from a host.

In some embodiments, the CD4⁺ T cells are in a frozen suspension. In some embodiments, the CD4⁺ T cells are in a liquid suspension. In some embodiments, the liquid suspension has previously been frozen.

In another aspect of the present disclosure provides a pharmaceutical composition comprising:
(i) the population of CD4⁺ T cells of any one of the above claims; suspended in
(ii) a pharmaceutically acceptable carrier.

In yet another aspect, the present disclosure provides a method of making poly-donor CD4^{IL-10} cells, comprising the steps of:
(i) pooling primary CD4⁺ T cells obtained from at least three different T cell donors; and
(ii) modifying the pooled CD4⁺ T cells by introducing an exogenous polynucleotide encoding IL-10,
thereby obtaining the genetically-modified CD4⁺ T cells.

In one aspect, the present disclosure provides a method of making poly-donor CD4^{IL-10} cells, comprising the steps of:
(i) obtaining primary CD4⁺ T cells from at least three different T cell donors; and
(ii) separately modifying each donor's CD4⁺ T cells by introducing an exogenous polynucleotide encoding IL-10, and then
(iii) pooling the genetically modified CD4⁺ T cells,
thereby obtaining the genetically-modified CD4⁺ T cells.

In some embodiments, the method further comprises the step, after step (i) and before step (ii), after step (ii), after step (ii) and before step (iii), or after step (iii), of:
incubating the primary CD4⁺ T cells in the presence of an anti-CD3 antibody, and anti-CD28 antibody or anti-CD3 antibody and CD28 antibody coated beads.

In some embodiments, the method comprises incubating the primary CD4⁺ T cells further in the presence of IL-2. In some embodiments, the exogenous polynucleotide is introduced into the primary CD4⁺ T cells using a viral vector. In some embodiments, the viral vector is a lentiviral vector. In some embodiments, the exogenous polynucleotide comprises a segment encoding IL-10 having the sequence of SEQ ID NO:1. In some embodiments, the IL-10-encoding polynucleotide segment has the sequence of SEQ ID NO:2.

In some embodiments, the exogenous polynucleotide further comprises a segment encoding a selection marker. In some embodiments, the encoded selection marker is ΔNGFR. In some embodiments, the encoded selection marker has the sequence of SEQ ID NO:3. In some embodiments, the encoded selection marker is a truncated EGFR polypeptide. In some embodiments, the encoded selection marker is a truncated human EGFR polypeptide.

In some embodiments, the method further comprises the step, after step (ii), of:
isolating the genetically-modified CD4⁺ T cells expressing the selection marker, thereby generating an enriched population of genetically-modified CD4⁺ T cells.

In some embodiments, at least 90% or at least 95% of the genetically-modified CD4⁺ T cells in the enriched population express IL-10. In some embodiments, at least 98% of the genetically-modified CD4⁺ T cells in the enriched population express IL-10. In some embodiments, at least 90% or at least 95% of the genetically-modified CD4⁺ T cells in the enriched population express the selection marker. In some embodiments, at least 98% of the genetically-modified CD4⁺ T cells in the enriched population express the selection marker.

In some embodiments, the method further comprises the step of incubating the enriched population of genetically-modified CD4⁺ T cells. In some embodiments, the step of incubating the enriched population of genetically-modified CD4⁺ T cells is performed in the presence of anti-CD3 antibody and anti-CD28 antibody or CD3 antibody and CD28 antibody coated beads in the presence of IL-2.

In some embodiments, the method further comprises the later step of freezing the genetically-modified CD4⁺ T cells. In some embodiments, in step (i), the primary CD4⁺ T cells are obtained from three, four, five, six, seven, eight, nine, or ten different T cell donors. In some embodiments, the at least three T cell donors have at least 5/10, 6/10, 7/10, 8/10, or 9/10 match at the HLA-A, HLA-B, HLA-C, HLA-DRB1, and HLA-DQB1 loci to each other. In some embodiments, the at least three T cell donors have at least 4/8, 5/8, 6/8, 7/8, or 8/8 match at the HLA-A, HLA-B, HLA-C, and HLA-DRB1 loci to each other. In some embodiments, the at least three T cell donors have 2/2 match at the HLA-A locus to each other. In some embodiments, the at least three T cell donors have 2/2 match at the HLA-B locus to each other. In some embodiments, the at least three T cell donors have 2/2 match at the HLA-C locus to each other. In some embodiments, the at least three T cell donors have at least 3/4 or 4/4 match at the HLA-DRB1 and HLA-DQB1 loci to each other. In some embodiments, each of the at least three T cell donors has an A*02 allele.

In some embodiments, in step (i), the primary CD4⁺T cells are obtained from one or more frozen stocks. In some embodiments, in step (i), the primary CD4⁺T cells are obtained from unfrozen peripheral blood mononuclear cells of the at least three different T cell donors.

In some embodiments, the method further comprises the step of isolating CD4⁺ T cells from the peripheral blood mononuclear cells. In some embodiments, the peripheral blood mononuclear cells are obtained from buffy coat or apheresis.

In another aspect, the present disclosure provides method of treating a patient, comprising the step of:
administering the poly-donor CD4^{IL-10} cells or the pharmaceutical composition of the present disclosure to a patient in need of immune tolerization.

In some embodiments, the method further comprises the preceding step of thawing a frozen suspension of poly-donor CD4^{IL-10} cells.

In some embodiments, the poly-donor CD4^{IL-10} cells or the pharmaceutical composition prevents or reduces severity of pathogenic T cell response in the patient.

In some embodiments, the method further comprises the step of administering mononuclear cells from a hematopoietic stem cells (HSC) donor to the patient. In some embodiments, the poly-donor CD4^{IL-10} cells or the pharmaceutical composition and the mononuclear cells from a HSC donor are administered concurrently. In some embodiments, the mononuclear cells from a HSC is administered either prior to or subsequent to administration of the poly-donor CD4^{IL-10} cells or the pharmaceutical composition. In some embodiments, the mononuclear cells are in the PBMC. In some embodiments, the mononuclear cells are in the bone marrow. In some embodiments, the mononuclear cells are in the cord blood. In some embodiments, the mononuclear cells have been isolated from the PBMC, bone marrow or cord blood.

In some embodiments, the method further comprises the step of:
administering hematopoietic stem cells (HSC) of an HSC donor to the patient either prior to or subsequent to administration of the poly-donor CD4^{IL-10} cells or pharmaceutical composition.

In some embodiments, the HSC donor is partially HLA-mismatched to the patient. In some embodiments, the HSC donor has less than 5/10, 6/10, 7/10, 8/10, 9/10 or 10/10 match at the HLA-A, HLA-B, HLA-C, HLA-DRB1, and HLA-DQB1 loci to the patient. In some embodiments, the HSC donor has less than 4/8, 5/8,6/8, 7/8, or 8/8 match at the HLA-A, HLA-B, HLA-C, and HLA-DRB1 loci to the patient. In some embodiments, the HSC donor has less than 2/2 match at the HLA-A, HLA-B, or HLA-C locus to the patient. In some embodiments, the HSC donor has less than 3/4 or 4/4 match at the HLA-DRB1 and HLA-DQB1 loci to the patient.

In some embodiments, one or more of the T cell donors are HLA-mismatched or partially HLA-mismatched to the patient. In some embodiments, one or more of the T cell donors have less than 5/10, 6/10, 7/10, 8/10, 9/10 or 10/10 match at the HLA-A, HLA-B, HLA-C, HLA-DRB1, and HLA-DQB1 loci to the patient. In some embodiments, one or more of the T cell donors have less than 4/8, 5/8, 6/8, 7/8, or 8/8 match at the HLA-A, HLA-B, HLA-C, and HLA-DRB1 loci to the patient. In some embodiments, one or more of the T cell donors have less than 2/2 match at the HLA-A, HLA-B, or HLA-C locus to the patient. In some embodiments, one or more of the T cell donors have less than 2/4, 3/4 or 4/4 match at the HLA-DRB1 and HLA-DQB1 loci to the patient. In some embodiments, one or more of the T cell donors are HLA-mismatched or partially HLA-mismatched with the HSC donor. In some embodiments, one or more of the T cell donors have less than 5/10, 6/10, 7/10, 8/10, 9/10 or 10/10 match at the HLA-A, HLA-B, HLA-C, HLA-DRB1, and HLA-DQB1 loci to the HSC donor. In some embodiments, one or more of the T cell donors have less than 4/8, 5/8, 6/8, 7/8, or 8/8 match at the HLA-A, HLA-B, HLA-C, and HLA-DRB1 loci to the HSC donor. In some embodiments, one or more of the T cell donors have less than 2/2 match at the HLA-A, HLA-B, or HLA-C locus to the HSC donor. In some embodiments, one or more of the T cell donors have less than 3/4 or 4/4 match at the HLA-DRB1 and HLA-DQB1 loci to the HSC donor.

In some embodiments, the poly-donor CD4^{IL-10} cells or the pharmaceutical composition prevents or reduces severity of GvHD by the transplanted hematopoietic stem cells.

In some embodiments, the poly-donor CD4^{IL-10} cells or the pharmaceutical composition prevents or reduces severity of pathogenic response of lymphoid cells from the transplanted hematopoietic stem cells.

In some embodiments, the patient has a cancer. In some embodiments, the patient has neoplastic cells. In some embodiments, the neoplastic cells express CD13, HLA-class I and CD54. In some embodiments, the neoplastic cells express CD112, CD58, or CD155.

In some embodiments, the patient has a cancer. In some embodiments, the cancer is a solid or hematological neoplasm. In some embodiments, the patient has a cancer selected from the group consisting of: Adrenal Cancer, Anal Cancer, Bile Duct Cancer, Bladder Cancer, Bone Cancer, Brain/CNS Tumors In Adults, Brain/CNS Tumors In Children, Breast Cancer, Breast Cancer In Men, Cancer of Unknown Primary, Castleman Disease, Cervical Cancer, Colon/Rectum Cancer, Endometrial Cancer, Esophagus Cancer, Ewing Family Of Tumors, Eye Cancer, Gallbladder Cancer, Gastrointestinal Carcinoid Tumors, Gastrointestinal Stromal Tumor (GIST), Gestational Trophoblastic Disease, Hodgkin Disease, Kaposi Sarcoma, Kidney Cancer, Laryngeal and Hypopharyngeal Cancer, Leukemia, Acute Lymphocytic (ALL), Acute Myeloid (AML, including myeloid sarcoma and leukemia cutis), Chronic Lymphocytic (CLL), Chronic Myeloid (CML) Leukemia, Chronic Myelomonocytic (CMML), Leukemia in Children, Liver Cancer, Lung Cancer, Lung Cancer with Non-Small Cell, Lung Cancer with Small Cell, Lung Carcinoid Tumor, Lymphoma, Lymphoma of the Skin, Malignant Mesothelioma, Multiple Myeloma, Myelodysplastic Syndrome, Nasal Cavity and Paranasal Sinus Cancer, Nasopharyngeal Cancer, Neuroblastoma, Non-Hodgkin Lymphoma, Non-Hodgkin Lymphoma In Children, Oral Cavity and Oropharyngeal Cancer, Osteosarcoma, Ovarian Cancer, Pancreatic Cancer, Penile Cancer, Pituitary Tumors, Prostate Cancer, Retinoblastoma, Rhabdomyosarcoma, Salivary Gland Cancer, Sarcoma - Adult Soft Tissue Cancer, Skin Cancer, Skin Cancer - Basal and Squamous Cell, Skin Cancer - Melanoma, Skin Cancer - Merkel Cell, Small Intestine Cancer, Stomach Cancer, Testicular Cancer, Thymus Cancer, Thyroid Cancer, Uterine Sarcoma, Vaginal Cancer, Vulvar Cancer, Waldenstrom Macroglobulinemia, and Wilms Tumor.

In some embodiments, the cancer is a myeloid cancer. In some embodiments, the cancer is AML or CML.

In some embodiments, the patient has an inflammatory or autoimmune disease. In some embodiments, the inflammatory or autoimmune disease is selected from the group consisting of: type-1 diabetes, autoimmune uveitis, rheumatoid arthritis, psoriasis, psoriatic arthritis, multiple sclerosis, systemic lupus, inflammatory bowel disease, Addison's disease, Graves' disease, Sjögren's syndrome, Hashimoto's thyroiditis, myasthenia gravis, autoimmune vasculitis, pernicious anemia, ulcerative colitis, bullous diseases, scleroderma, and celiac disease.

In some embodiments, the inflammatory or autoimmune disease is Crohn's disease, ulcerative colitis, celiac disease, type-1 diabetes, lupus, psoriasis, psoriatic arthritis, or rheumatoid arthritis.

In some embodiments, the patient has a disease or disorder involving hyperactivity of NLPR3 inflammasome. In some embodiments, the patient has type 2 diabetes, neurodegenerative diseases, or inflammatory bowel disease.

In some embodiments, the patient has a disease or disorder involving increased IL-1β production by activated monocytes, macrophages or dendritic cells.

In some embodiments, the patient has a disease or disorder involving increased IL-18 production by activated monocytes, macrophages or dendritic cells.

In some embodiments, the patient has a disease or disorder involving increased mature caspase 1 production by activated monocytes, macrophages or dendritic cells.

In some embodiments, the patient has an allergic or atopic disease. In some embodiments, the allergic or atopic disease is selected from the group consisting of : asthma, atopic dermatitis, and rhinitis. In some embodiments, the patient has a food allergy.

In some embodiments, the method further comprises the step of cell and organ transplantation to the patient, either prior to or subsequent to administration of the population of CD4⁺ T cells or the pharmaceutical composition. In some embodiments, the poly-donor CD4^{IL-10} cells or the pharmaceutical composition prevents or reduces severity of host rejection of the cell and organ transplantation.

In some embodiments, the method further comprises the step of transplanting iPS cellderived cells or tissues to the patient, either prior to or subsequent to administration of the population of CD4⁺ T cells or the pharmaceutical composition.

In some embodiments, poly-donor CD4^{IL-10} cells or the pharmaceutical composition prevents or reduces severity of host rejection of the transplantation.

In some embodiments, the method further comprises the step of administering a recombinant AAV to the patient, either prior to or subsequent to administration of the poly-donor CD4^{IL-10} cells or the pharmaceutical composition. In some embodiments, the poly-donor CD4^{IL-10} cells or the pharmaceutical composition reduces immune responses against the recombinant AAV.

In some embodiments, the patient has an excessive immune response against viral or bacterial infection. In some embodiments, the patient has a coronavirus infection.

In some embodiments, the method further comprises the step of detecting the selection marker in a biological sample obtained from the patient, thereby detecting presence or absence of poly-donor CD4^{IL-10} T cells. In some embodiments, the biological sample is a biopsy or blood from the patient.

In one aspect, the present disclosure provides a method of treating a patient with a malignancy, comprising: administering an allo-HSCT graft to the patient, and administering a therapeutically effective amount of poly-donor CD4^{IL-10} cells.

In some embodiments, none of the donors of the CD4^{IL-10} cells in the poly-donor CD4^{IL-10} cells is the donor of the HSCT graft.

In another aspect, the present disclosure provides a method of treating a hematological cancer, comprising: administering to a hematological cancer patient an amount of poly-donor CD4^{IL-10} cells sufficient to induce anti-cancer effects, wherein the poly-donor CD4^{IL-10} cells comprise CD4⁺ T cells that have been obtained from at least three different T cell donors and then genetically modified by vector-mediated gene transfer of the coding sequence of human IL-10 under control of a constitutive promoter.

In some embodiments, the method further comprises the step of administering allo HSCT graft to the patient prior to or subsequence to administration of the poly-donor CD4^{IL-10} cells. In some embodiments, the amount of poly-donor CD4^{IL-10} cells is further sufficient to suppress graft-versus-host disease (GvHD) without suppressing graft-versus-leukemia (GvL) or graft-versus-tumor (GvT) efficacy of the allo HSCT.

In some embodiments, the hematological cancer is a myeloid leukemia.

In some embodiments, the poly-donor CD4^{IL-10} cells target and kill cancer cells that express CD13. In some embodiments, the poly-donor CD4^{1L-10} cells target and kill cancer cells that express HLA-class I. In some embodiments, the myeloid leukemia is acute myeloid leukemia (AML).

In some embodiments, the allo-HSCT graft is obtained from a related or unrelated donor with respect to the recipient. In some embodiments, the poly-donor CD4^{IL-10} cells are non-autologous to the recipient. In some embodiments, the poly-donor CD4^{IL-10} cells are allogeneic to the recipient. In some embodiments, the poly-donor CD4^{IL-10} cells are not anergized to host allo-antigens prior to administration to the host.

In some embodiments, the poly-donor CD4^{IL-10} cells are Tr1-like cells.

In some embodiments, the poly-donor CD4^{IL-10} cells are polyclonal. In some embodiments, the poly-donor CD4^{IL-10} cells are polyclonal and non-autologous to the recipient.

In some embodiments, the poly-donor CD4^{IL-10} cells are isolated from at least three donors prior to being genetically modified. In some embodiments, none of the at least three donors is the same donor as the allo-HSCT donor. In some embodiments, the allo-HSCT graft is obtained from a matched or mismatched donor with respect to the recipient.

In some embodiments, the poly-donor CD4^{IL-10} cells target and kill cells that express CD54. In some embodiments, the poly-donor CD4^{IL-10} cells target and kill cancer cells that express HLA-class I and CD54. In some embodiments, the poly-donor CD4^{IL-10} cells target and kill cancer cells that express CD112. In some embodiments, the poly-donor CD4^{IL-10} cells target and kill cancer cells that express CD58. In some embodiments, the poly-donor CD4^{IL-10} cells target and kill cancer cells in the host.

One aspect of the present disclosure provides a method of treating a hematological cancer by allogeneic hematopoietic stem cell transplant (allo-HSCT), comprising:
administering allo-HSCT graft to a subject (host);
administering to the allo-HSCT recipient (host) an amount of poly-donor CD4^{IL-10} cells sufficient to suppress graft-versus-host disease (GvHD) without suppressing graft-versus-leukemia (GvL) or graft-versus-tumor (GvT) efficacy of the allo-HSCT graft;
wherein the poly-donor CD4^{IL-10} cells comprise CD4⁺ T cells obtained from at least three different T cell donors and genetically modified by vector-mediated gene transfer of the coding sequence of human IL-10 under control of a constitutive promoter;
wherein the poly-donor CD4^{IL-10} cells are non-autologous to the recipient and non-autologous to the allo-HSCT donor;
wherein the poly-donor CD4^{IL-10} cells are not anergized to host allo-antigens prior to administration to the host; and
wherein the poly-donor CD4^{IL-10} cells are polyclonal and Tr1-like.

Another aspect of the present disclosure provides a method of treating a hematological cancer by allogeneic hematopoietic stem cell transplant (allo-HSCT), comprising:
administering allo-HSCT graft to a subject (host);
administering to the allo-HSCT recipient (host) an amount of poly-donor CD4^{1L-10} cells sufficient to suppress graft-versus-host disease (GvHD) without suppressing graft-versus-leukemia (GvL) or graft-versus-tumor (GvT) efficacy of the allo-HSCT graft;
wherein the poly-donor CD4^{IL-10} cells comprise CD4⁺ T cells obtained from at least three different T cell donors and genetically modified by vector-mediated gene transfer of the coding sequence of human IL-10 under control of a constitutive promoter;
wherein the poly-donor CD4^{IL-10} cells target and kill cancer cells in the host;
wherein the poly-donor CD4^{IL-10} cells are not anergized to host allo-antigens prior to administration to the host; and
wherein the poly-donor CD4^{IL-10} cells are non-autologous to the recipient, and polyclonal, and are Tr1-like.

### 5. BRIEF DESCRIPTION OF THE DRAWINGS

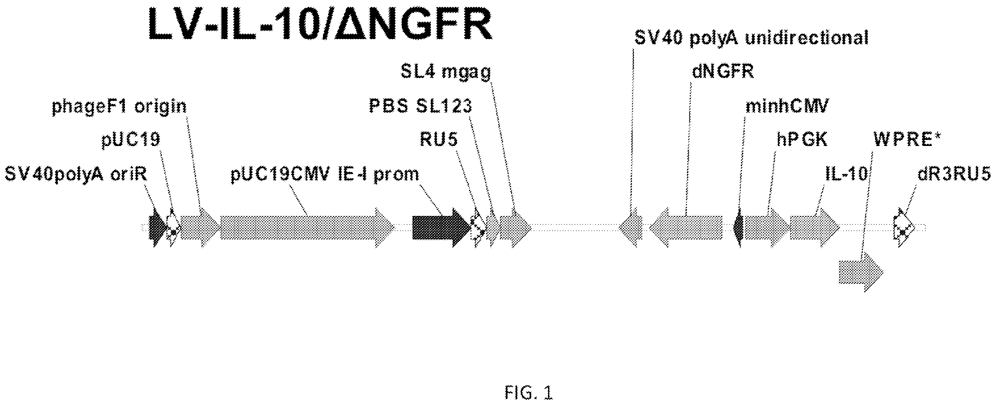

**FIG. 1** illustrates the partial structure of a bidirectional lentiviral vector for delivering human IL-10 and ΔNGFR coding sequences into CD4⁺ T cells from multiple donors to produce poly-donor CD4^{IL-10} cells.

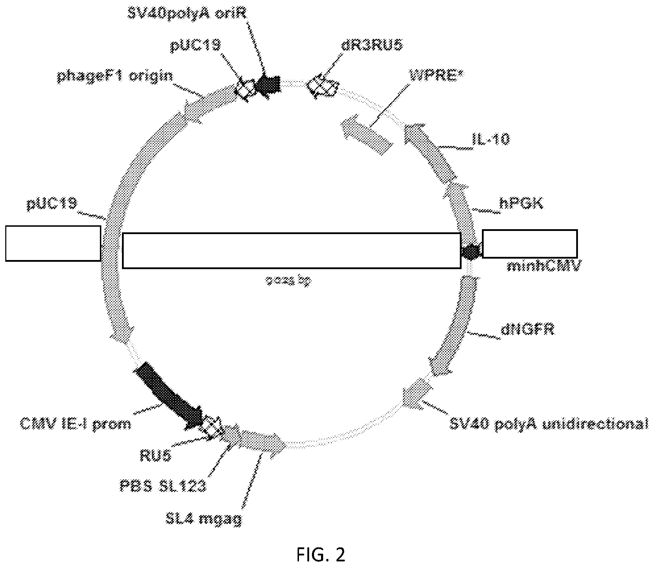

**FIG. 2** illustrates the complete and circular structure of a bidirectional lentiviral vector (hPGK.IL10.WPRE.mhCMV. ΔNGFR.SV40PA) for delivering human IL-10 and ΔNGFR coding sequences into CD4⁺ T cells from multiple donors to produce poly-donor CD4^{IL-10} cells.

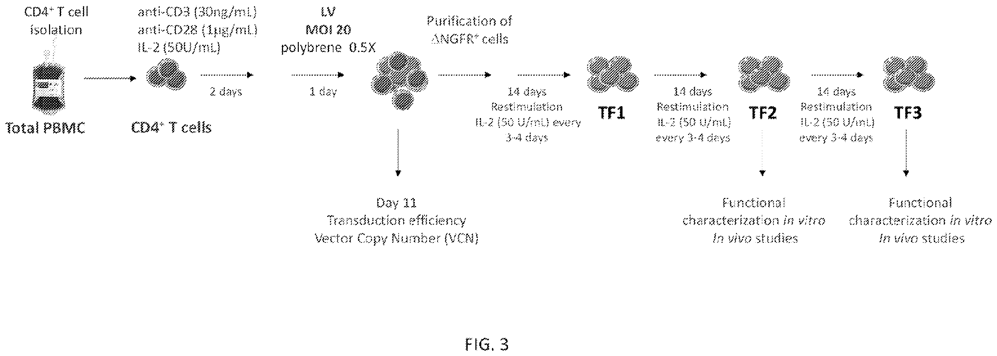

**FIG. 3** illustrates an exemplary protocol for generating CD4^{IL-10} cells.

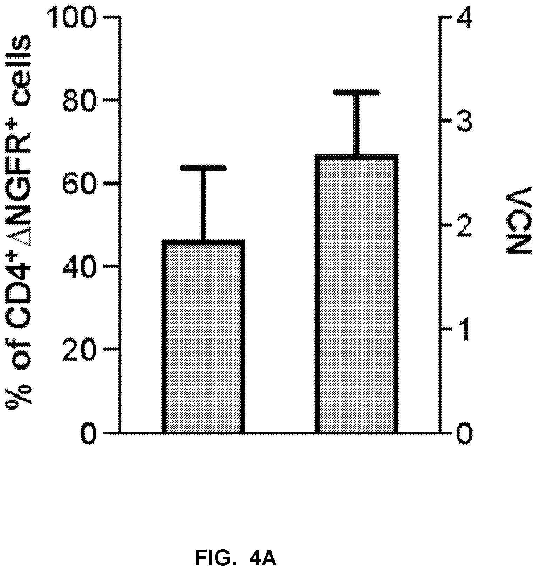

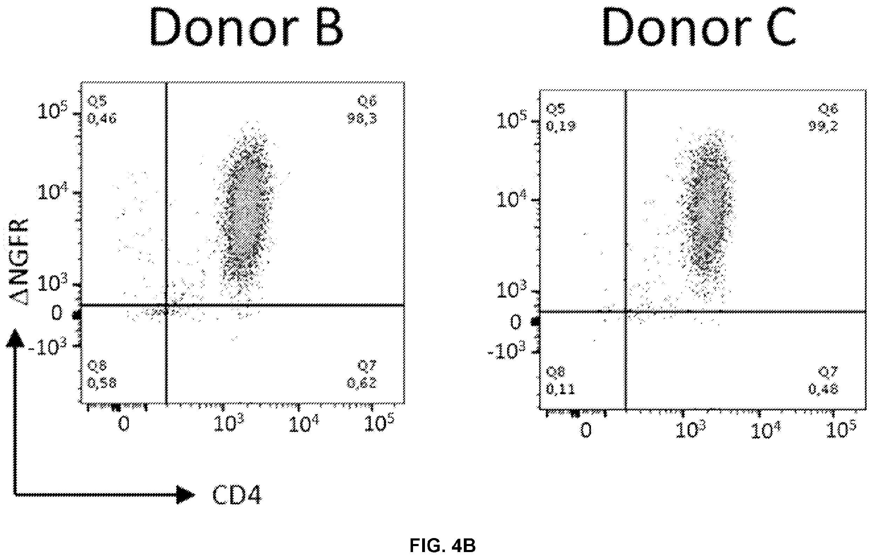

**FIG. 4A** shows percentages of CD4⁺ΔNGFR⁺ cells (mean± SD, n=10 grey bar) and vector copy numbers (VCN, mean ± SD, n=10 orange bar) in human CD4⁺ T cells transduced with LV-IL-10/ΔNGFR (a bidirectional lentiviral vector encoding for human IL-10 and a truncated form the human NGF receptor). **FIG. 4B** shows FACS analysis of expression of CD4 and ΔNGFR in human CD4⁺ T cells from two representative donors (Donor B and Donor C) transduced with LV-IL-10/ΔNGFR and purified using anti-CD271 Microbeads.

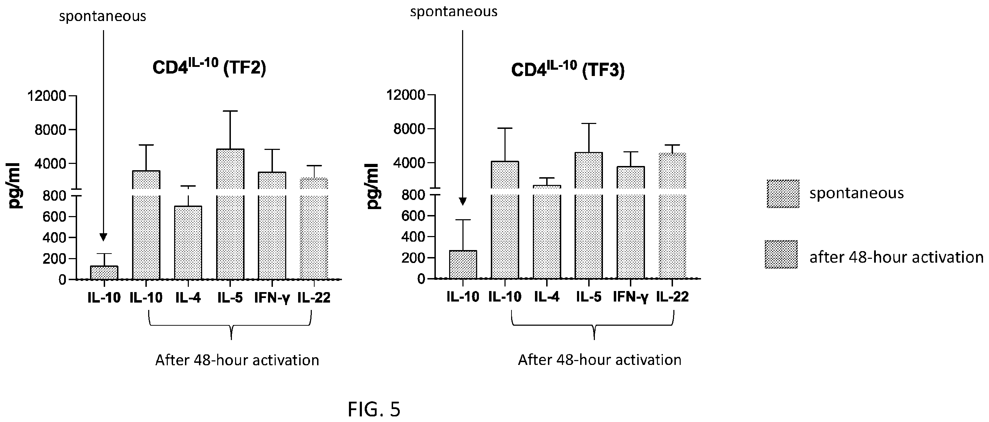

**FIG. 5** shows cytokine production profile of single donor CD4^{IL-10} cells after the second (TF2) and third (TF3) restimulation. The TF2 and TF3 CD4^{IL-10} cells were left unstimulated (orange bar) or stimulated with immobilized anti-CD3 and soluble anti-CD28 mAbs (grey bars) for 48hrs. Culture supernatants were collected and levels of IL-10, IL-4, IL-5, IFN-γ and IL-22 were determined by ELISA. All samples were tested in triplicate. Mean± SD, n=8 donors tested are presented.

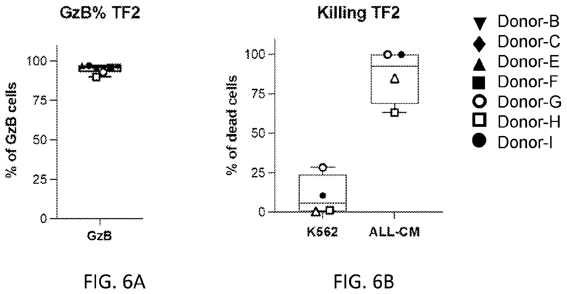

**FIG. 6A** shows the percentage of CD4^{IL-10} cells expressing granzyme B (GzB) after 2^{nd} round of stimulation (TF2) analyzed by FACS. Box and whiskers of n=7 donors and single donors are presented. **FIG. 6B** shows % dead cells when CD4^{IL-10} cells (10⁵/well) were co-cultured with K562 and ALL-CM cells (10⁵/well) at 1:1 ratio for 3 days. Box and whiskers represent data from n=4 donors and dots represent data from single donors.

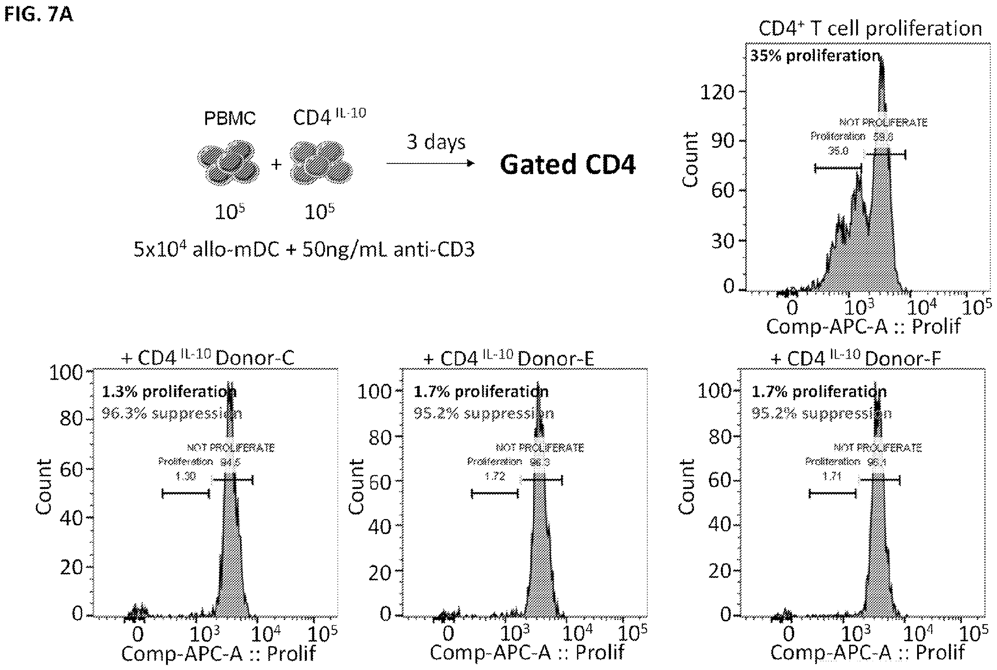

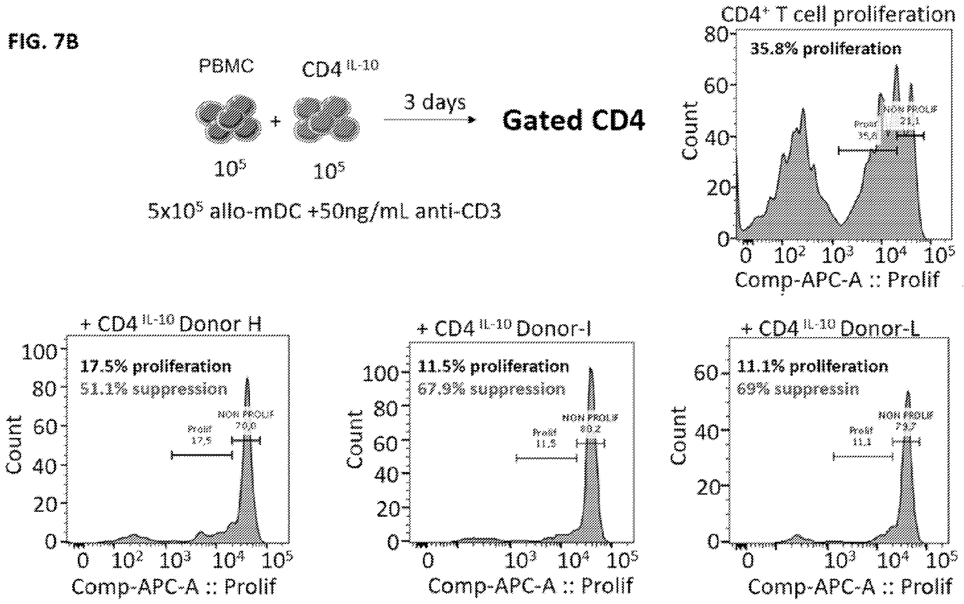

**FIGs. 7A** **and** **7B** show that single donor CD4^{IL-10} cells can suppress the proliferation of allogeneic CD4⁺ T cells. Allogeneic PBMC cells were labeled with eFluor^{®} 670 (10⁵ cells/well) and stimulated with allogenic mature dendritic (DC) cells (5x10⁴ cells/well) and soluble anti-CD3 mAbs in the absence or presence of CD4^{IL-10} cells (10⁵ cells/well) at a 1:1 Responder: Suppressor ratio. After 4 days of culture, the percentages of proliferating responder cells were determined by eFluor^{®} 670 dilution with flow cytometry after gating on CD4⁺ΔNGFR⁻ T cells. FIG. 7A show results from Donor-C, Donor-E, and Donor-F and FIG. 7B show results from Donor-H, Donor-I and Donor-L. Percentages of proliferation and suppression are indicated. The suppression mediated by CD4^{IL-10} cells was calculated as follows: 100-([proliferation of responders in the presence of CD4^{IL-10} cells/proliferation of responders alone]x 100).

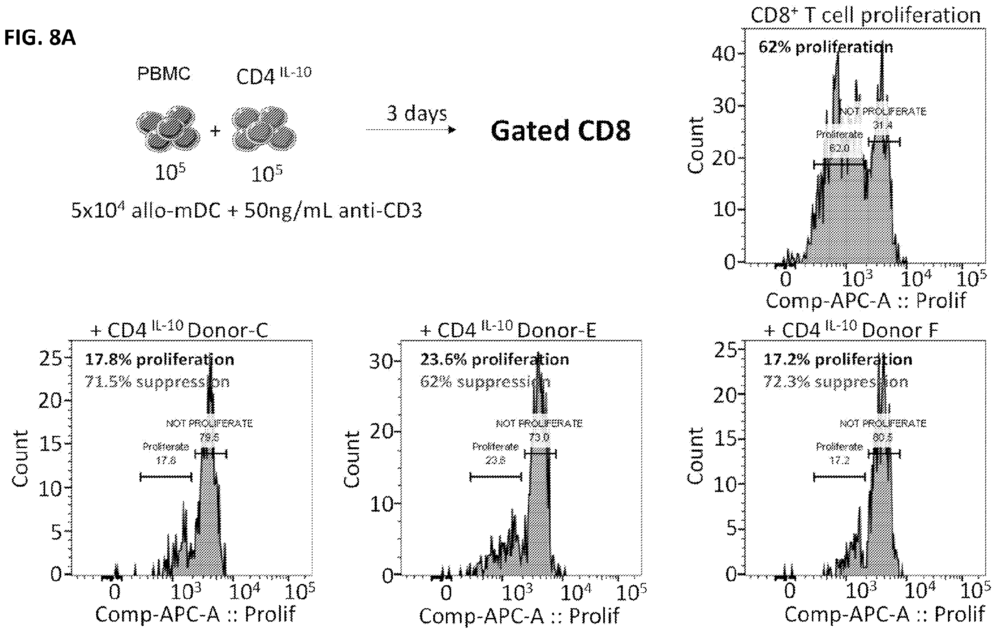

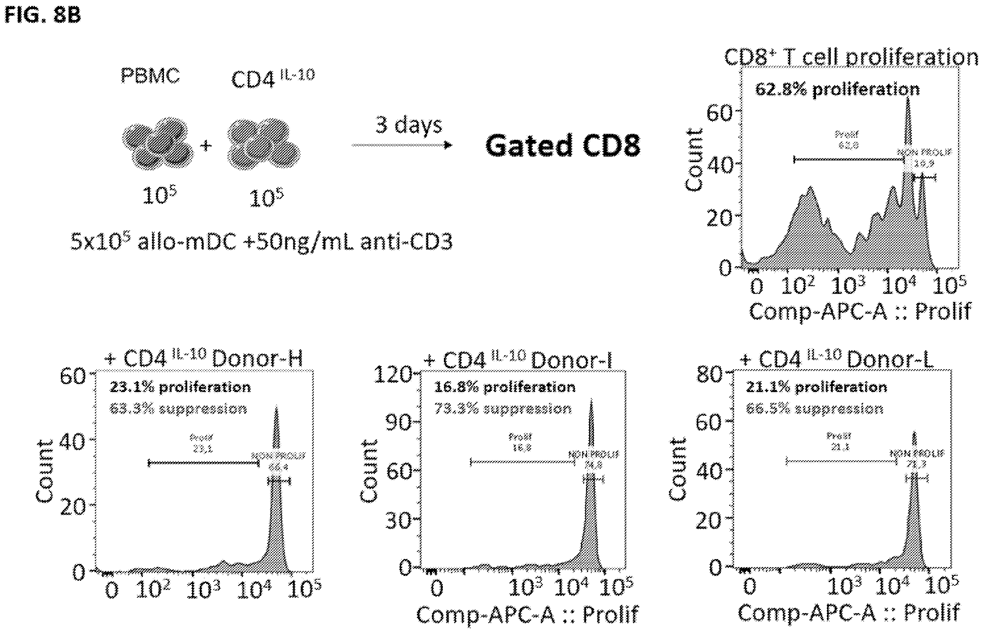

**FIGs. 8A** **and** **8B** show that single donor CD4^{IL-10} cells can suppress the proliferation of allogeneic CD8⁺ T cells. Allogeneic PBMC cells were labeled with eFluor^{®} 670 (10⁵ cells/well) and stimulated with allogeneic mature dendritic (DC) cells (5x10⁴ cells/well) and soluble anti-CD3 mAbs in the absence or presence of CD4^{IL-10} cells (10⁵ cells/well) at a 1:1 Responder: Suppressor ratio. After 4 days of culture, the percentages of proliferating responder cells were determined by eFluor^{®} 670 dilution with flow cytometry after gating on CD4⁺ΔNGFR⁻ T cells. FIG. 8A show results from Donor-C, Donor-E, and Donor-F and FIG. 8B show results from Donor-H, Donor-I and Donor-L. Percentages of proliferation and suppression are indicated. The suppression mediated by CD4^{IL-10} cells was calculated as follows: 100-([proliferation of responders in the presence of CD4^{IL-10} cells/proliferation of responders alone]x 100).

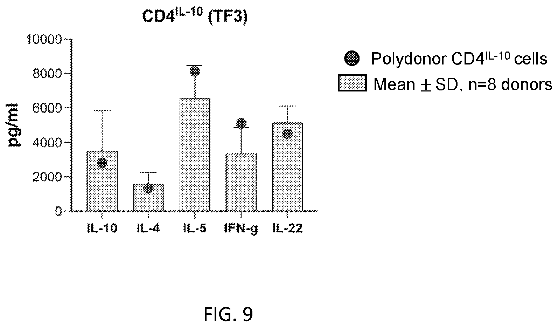

**FIG. 9** shows cytokine production profile of poly-donor CD4^{IL-10} cells after third (TF3) restimulation, compared to mean levels (+/- SD) produced by CD4^{IL-10} cells from 8 individual donors. The TF3 CD4^{IL-10} cells from three donors were pooled at a 1:1:1 ratio and stimulated with immobilized anti-CD3 and soluble anti-CD28 mAbs for 48hrs. Culture supernatants were collected and levels of IL-10, IL-4, IL-5, IFN-γ and IL-22 were determined by ELISA. Dots are results of polydonor CD4^{IL-10} cells; gray bars represent mean± SD, n=8 single donors.

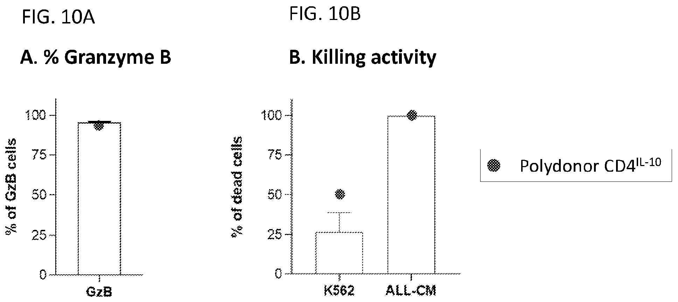

**FIG. 10A** shows the percentage of poly-donor CD4^{IL-10} cells expressing granzyme B (GzB) compared to mean % levels (+/- SD) of granzyme B expression by CD4^{IL-10} cells of n=3 single donors used to generate the pool. Cells were analyzed by FACS after the 3^{rd} round of stimulation (TF3). FIG. 10B shows % dead cells when poly-donor CD4^{IL-10} cells (10⁵/well) were co-cultured with K562 and ALL-CM cells (10⁵/well) at 1:1 ratio for 3 days. Residual leukemic cells (CD45⁺ CD33⁺ ) were counted by FACS for each target cell. Dots are results of poly-donor CD4^{IL-10} and gray bars represent mean± SD of n=3 single donors used to generate the pool.

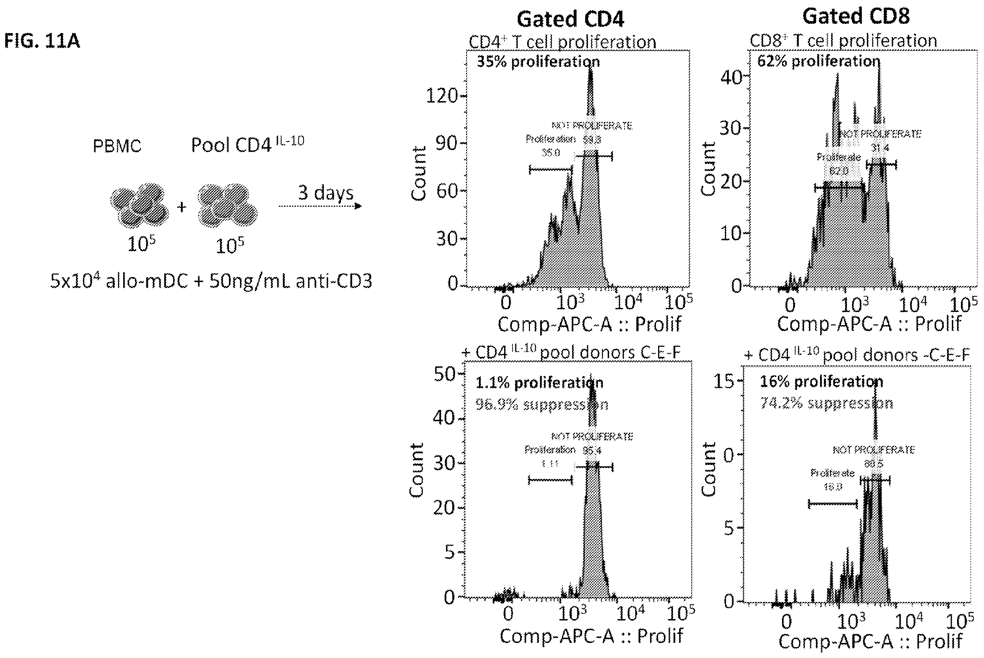

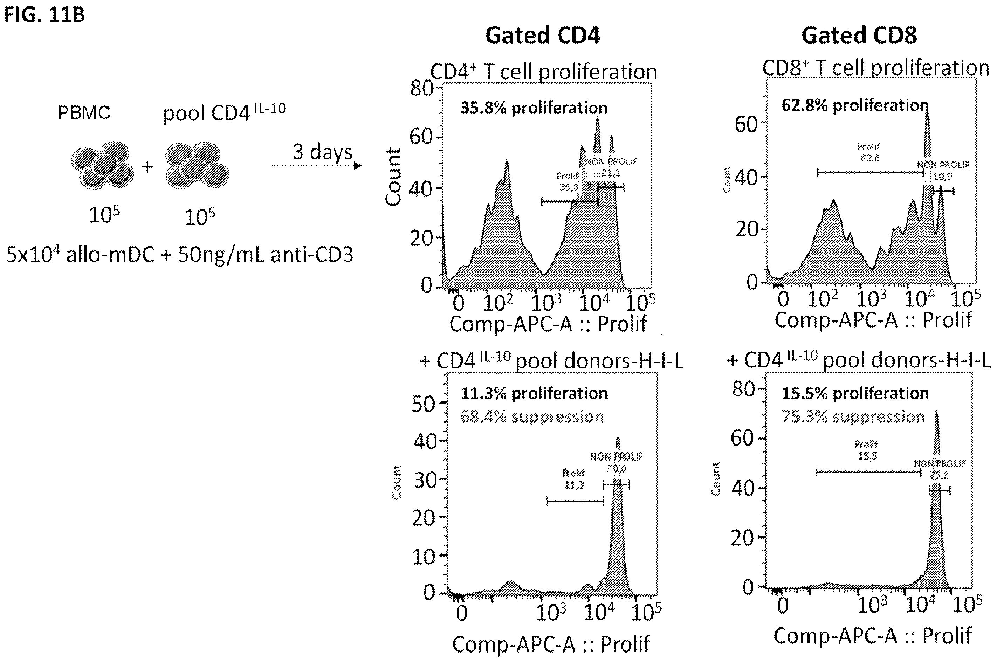

**FIGs. 11A** **and** **11B** show that poly-donor CD4^{IL-10} cells can suppress the proliferation of allogeneic CD4⁺ T cells and CD8⁺ T cells. Allogeneic PBMC cells were labeled with eFluor^{®} 670 (10⁵ cells/well) and stimulated with allogeneic mature dendritic (DC) cells (5x10⁴ cells/well) and soluble anti-CD3 mAbs in the absence or presence of poly-donor CD4^{IL-10} cells (10⁵ cells/well) at a 1:1 Responder:Suppressor ratio. After 4 days of culture, the percentages of proliferating responder cells were determined by eFluor^{®} 670 dilution with flow cytometry after gating on CD4⁺ΔNGFR⁻ T cells and CD8⁺ΔNGFR⁻ T cells. FIG. 11A shows results from polydonor CD4^{IL-10} cells containing CD4⁺ cells pooled from Donor-C, Donor-E, and Donor-F. FIG. 11B shows results from poly-donor CD4^{IL-10} cells containing CD4⁺ cells pooled from Donor-H, Donor-1, and Donor-L. The suppression mediated by CD4^{IL-10} cells was calculated as follows: 100-([proliferation of responders in the presence of CD4^{IL-10} cells/proliferation of responders alone]x 100).

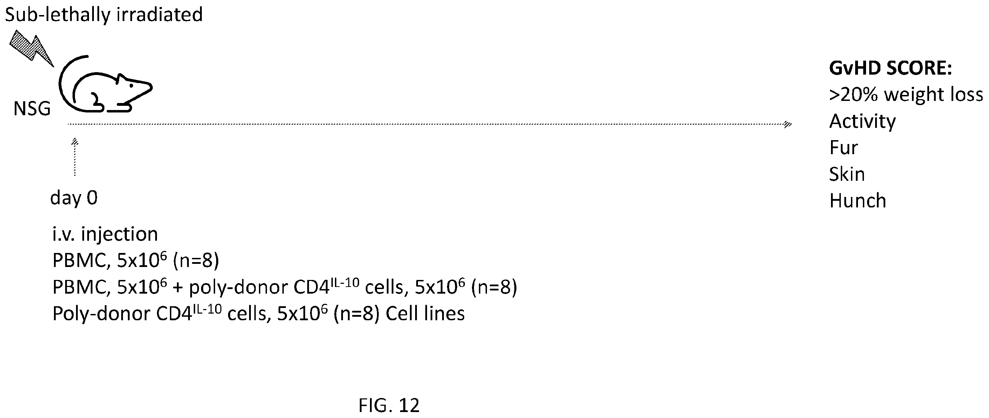

**FIG. 12** illustrates a protocol for testing induction of GvHD by human PBMC and/or poly-donor CD4^{IL-10} cells injected on day 0 post-irradiation.

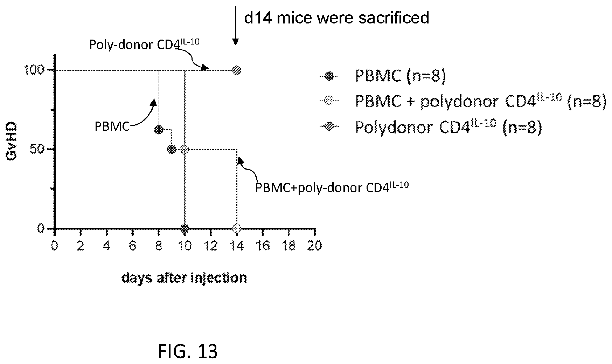

**FIG. 13** shows % of NSG mice demonstrating GvHD in each day after injection of PBMC (5x10⁶ cells/mouse), poly-donor (three donors) CD4^{IL-10} cells (5x10⁶ cells/mouse), or PBMC (5x10⁶ cells/mouse) in combination with poly-donor CD4^{IL-10} cells (three donors) (5x10⁶ cells/mouse).

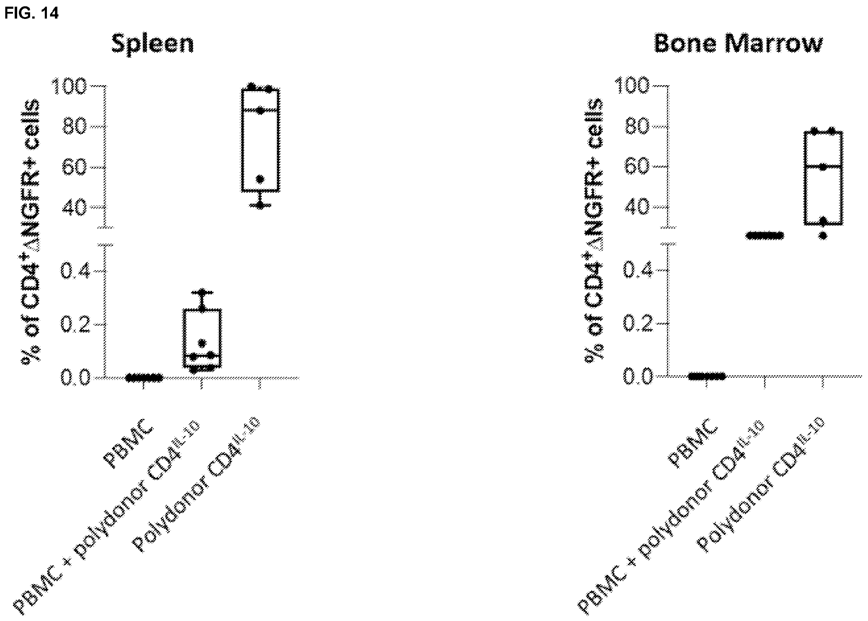

**FIG. 14** shows migration of CD4^{IL-10} cells to spleen and bone marrow in NSG mice injected with PBMC (5x10⁶ cells/mouse), poly-donor (three donors) CD4^{IL-10} cells (5x10⁶ cells/mouse), or PBMC (5x10⁶ cells/mouse) in combination with poly-donor CD4^{IL-10} cells (three donors) (5x10⁶ cells/mouse). Box and whiskers on n=8 donors and single donors are presented.

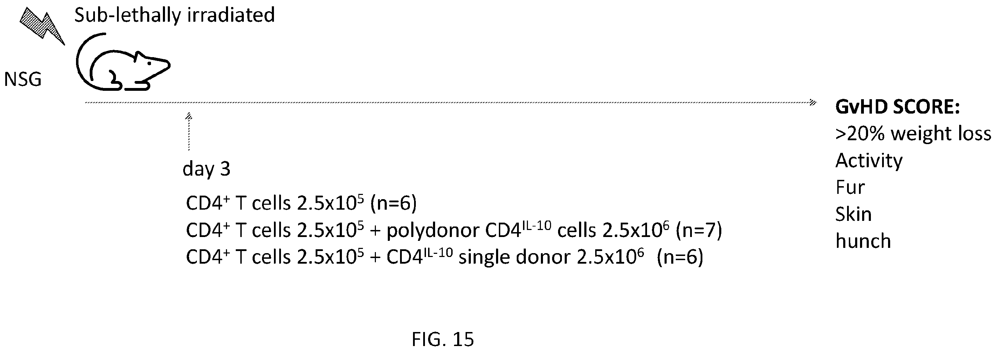

**FIG. 15** illustrates a protocol for testing induction of GvHD by CD4⁺ T cells and polydonor or single-donor CD4^{IL-10} cells injected on day 0 post-irradiation.

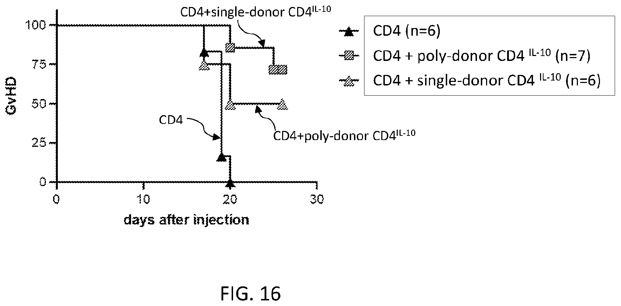

**FIG. 16** shows % of NSG mice demonstrating GvHD on each day after injection.

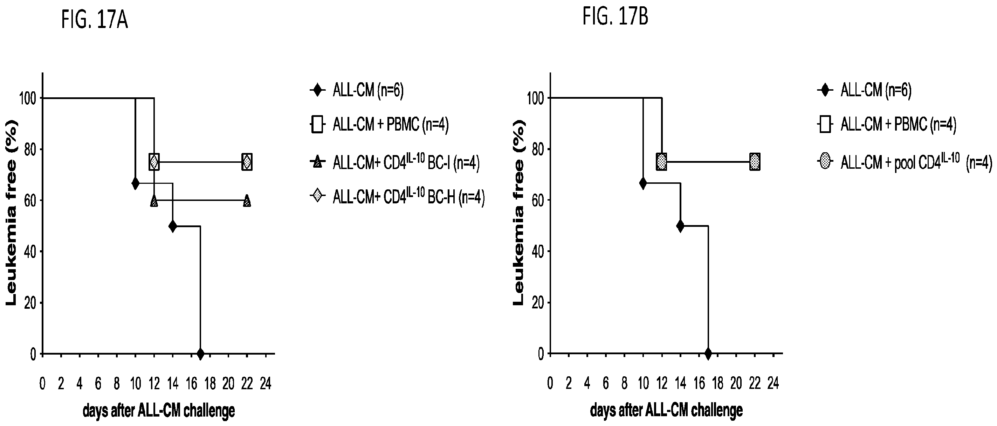

**FIG. 17A and 17B** shows graft-versus-leukemia (GvL) effect tested based on reduction of circulating leukemia cells and long-term leukemia free survival. Leukemia was measured as previously described (Locafaro G. et al Molecular Therapy 2017). NSG mice were sub-lethally irradiated and intravenously injected with myeloid leukemia cells (ALL-CM) (5x10⁶) at day 0. **FIG. 17A** shows leukemia free survival rate in the animals injected with PBMC (5x10⁶) or single donor (from donor BC-I and donor BC-H) CD4^{IL-10} cells (2.5x10⁶) at day 3. **FIG. 17B****.**shows leukemia free survival rate in the animals injected with PBMC (5x10⁶) or poly-donor CD4^{IL-10} cells (2.5x10⁶) at day 3.

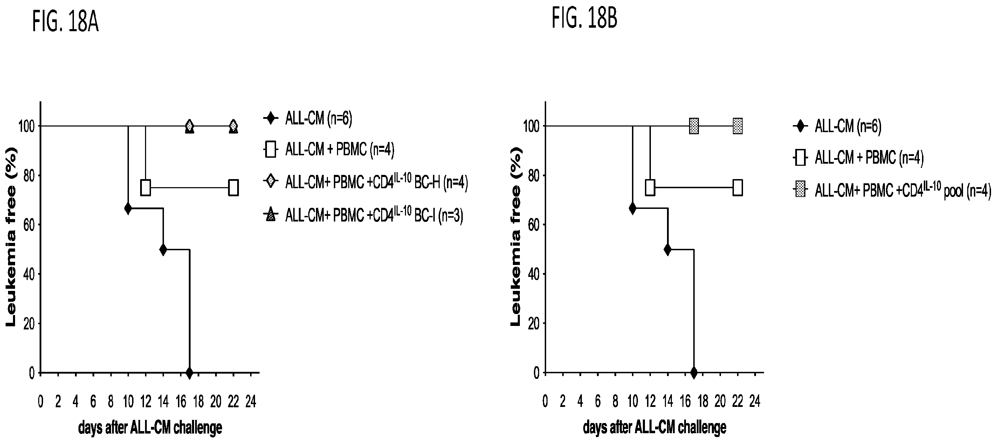

**FIG. 18A** and **FIG. 18B** show long-term leukemia free survival rate measured in NSG mice sub-lethally irradiated and intravenously injected with ALL-CM cells (5x10⁶) at day 0. **FIG. 18A** shows data from animals injected with mononuclear cells (PBMC) (5x10⁶) alone or mononuclear cells (PBMC) (5x10⁶) + single donor (from donor BC-H and donor BC-I) CD4^{IL-10} cells (2.5x10⁶) at day 3. **FIG. 18B** shows data from animals injected with mononuclear cells (PBMC) (5x10⁶) alone or mononuclear cells (PBMC) (5x10⁶) + poly-donor CD4^{IL-10} cells (2.5x10⁶) at day 3.

The figures depict various embodiments of the present invention for purposes of illustration only. One skilled in the art will readily recognize from the following discussion that alternative embodiments of the structures and methods illustrated herein may be employed without departing from the principles of the invention described herein.

### 6. DETAILED DESCRIPTION

### 6.1. Definitions

Unless defined otherwise, all technical and scientific terms used herein have the meaning commonly understood by a person skilled in the art to which this invention belongs. As used herein, the following terms have the meanings ascribed to them below.

"**Graft-versus-leukemia effect**" or "**GvL**" refers to an effect that appears after allogeneic hematopoietic stem cell transplantation (HSCT) or bone marrow transplantation (BMT). T lymphocytes in the allogeneic graft eliminate malignant residual host leukemia cells.

"**Graft versus tumor effect**" or "**GvT** refers to an effect that appears after allogeneic hematopoietic stem cell transplantation (HSCT) or bone marrow transplantation (BMT). T lymphocytes in the allogeneic graft eliminate malignant residual host cancer cells, e.g., cells of myeloma and lymphoid and myeloid leukemias, lymphoma, multiple myeloma and possibly breast cancer. The term GvT is generic to GvL.

The terms "**treatment**", "**treating**", and the like are used herein in the broadest sense understood in the medical arts. In particular, the terms generally mean obtaining a desired pharmacologic and/or physiologic effect. The effect may be prophylactic, in terms of completely or partially preventing a disease, condition, or symptoms thereof, and/or may be therapeutic in terms of a partial or complete cure for a disease or condition and/or adverse effect, such as a symptom, attributable to the disease or condition. "Treatment" as used herein covers any treatment of a disease or condition of a mammal, particularly a human, and includes: (a) preventing the disease or condition from occurring in a subject which may be predisposed to the disease or condition but has not yet been diagnosed as having it; (b) inhibiting the disease or condition (e.g., arresting its development); or (c) relieving the disease or condition (e.g., causing regression of the disease or condition, providing improvement in one or more symptoms). Improvements in any conditions can be readily assessed according to standard methods and techniques known in the art. The population of subjects treated by the method of the disease includes subjects suffering from the undesirable condition or disease, as well as subjects at risk for development of the condition or disease.

"**HLA-matched**" as used herein refers to a pair of individuals having a matching HLA allele in the HLA class I (HLA-A, HLA-B, and HLA-C) and class II (HLA-DRB1 and HLA-DQB1) loci that allow the individuals to be immunologically compatible with each other. HLA compatibility can be determined using any of the methods available in the art, for example, as described in Tiervy, Haematologica 2016 Volume 101(6):680-687, which is incorporated by reference herein.

For a given locus, a pair of individuals have 2/2 match when each of two alleles of one individual match with the two alleles of the other individual. A pair of individuals have ½ match when only one of two alleles of one individual match with one of two alleles of the other individual. A pair of individuals have 10/10 match at the HLA-A, HLA-B, HLA-C, HLA-DRB1, and HLA-DQB1 loci when all of the ten alleles (two for each of the HLA-A, HLA-B, HLA-C, HLA-DRB1, and HLA-DQB1 loci) of one individual match with all ten alleles of the other individual.

In preferred embodiments, allele level typing is used for determination of HLA compatibility. Allele level typing corresponds to a unique nucleotide sequence for an HLA gene, as defined by using all digits in the first, second, third and fourth fields, e.g. A*02:01:01:01. Functionally, the third and fourth fields which characterize alleles that differ, respectively, by silent substitutions in the coding sequence and by substitutions in the non-coding sequence, are irrelevant, except when substitutions prevent the expression of HLA alleles (*e.g.* the null allele B*15:01:01:02N). Missing a null allele will lead to a mismatch that is very likely to be recognized by alloreactive T cells and have a deleterious clinical impact. Substitutions in non-coding sequences may influence the level of expression (*e.g*. the A24low allele A*24:02:01:02L). Such variability may also have an impact on anti-HLA allorecognition.

The term "**HLA-mismatched**" as used herein refers to a pair of individuals having a mis-matching HLA allele in the HLA class I (HLA-A, HLA-B, and HLA-C) and class II (HLA-DRB1 and HLA-DQB1) loci that make the individuals to be immunologically incompatible with each other.

The term "**partially HLA-mismatched**" as used herein refers to a pair of individuals having a mis-matching HLA allele in the HLA class I (HLA-A, HLA-B, and HLA-C) and class II (HLA-DRB1 and HLA-DQB1) loci that make the individuals to be immunologically incompatible with each other in a permissible degree. Some studies have identified permissive mismatches. Some HLA class I incompatibilities are considered to be more permissive.

"**HLA haplotype**" refers to a series of HLA loci-alleles by chromosome, one passed from the mother and one from the father. Genotypes for HLA class I (HLA-A, HLA-B, and HLA-C) and class II (HLA-DRB 1 and HLA-DQB1) loci can be used to determine the HLA haplotype.

The term "**therapeutically effective amount**" is an amount that is effective to treat, and thus ameliorate a symptom of a disease. A therapeutically effective amount can be a "**prophylactically effective amount**" as prophylaxis can be considered therapy.

The term "**ameliorating**" refers to any therapeutically beneficial result in the treatment of a disease state, e.g., a neurodegenerative disease state, including prophylaxis, lessening in the severity or progression, remission, or cure thereof.

### 6.2. Other interpretational conventions

Ranges recited herein are understood to be shorthand for all of the values within the range, inclusive of the recited endpoints. For example, a range of 1 to 50 is understood to include any number, combination of numbers, or sub-range from the group consisting of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, and 50.

### 6.3. Summary of experimental observations

The present disclosure provides the methods for production and use of highly purified, allogeneic CD4⁺ T cells that have been transduced with a bidirectional lentiviral vector containing the human IL-10 gene and a truncated, non-signaling form of the human NGFR. The successfully transduced CD4⁺ T cells were purified utilizing a NGFR specific monoclonal antibody resulting in > 95% pure IL-10 producing and NGFR expressing CD4⁺ T cells (designated CD4^{IL-10} cells). CD4^{IL-10} cells from 3 different allogeneic HLA mismatched donors were pooled at 1:1:1 ratios.

These pooled populations, also referred to herein as poly-donor CD4^{IL-10} cells, had cytokine production profiles comparable to those of single-donor CD4^{IL-10} cells and naturally derived type 1 regulatory T (Tr1) cells. They produce high levels of IL-10, IL-22, IFN-y, IL-5 and low levels of IL-4. The poly-donor CD4^{IL-10} cells were polyclonal (has multiple antigen specificities) and suppressed proliferation of both allogeneic CD4⁺ and CD8⁺ T cells *in vitro.* In addition, they specifically killed myeloid leukemia cells *in vitro.* Adoptive transfer of polydonor CD4^{IL-10} cells in a humanized mouse model for Graft versus Host Disease (GvHD) indicated that these cells efficiently home to the spleen. Adoptive transfer of poly-donor CD4^{IL-10} cells in a humanized mouse model of GvHD inhibited severe xeno-GvHD induced by human CD4⁺ T cells. Importantly, even at high concentrations, poly-donor CD4IL-10 cells did not induce GvHD by themselves. These results demonstrate that poly-donor CD4^{IL-10} cells can be used for the treatment and/or prevention of GvHD; can be used as an adjunct to allogeneic hematopoietic stem cell transplant (HSCT) for treatment of leukemias and other malignancies to reduce GvHD while preserving GvL or GvT therapeutic effects of the HSCT; and for treating cell and organ rejection and autoimmune and inflammatory diseases.

### 6.4. Poly-donor CD4^{IL-10} cells

In a first aspect, a population of CD4⁺ T cells that have been genetically modified to comprise an exogenous polynucleotide encoding IL-10 is provided (CD4^{IL-10} cells). The population comprises CD4⁺ T cells obtained from at least three different T cell donors (polydonor CD4^{IL-10} cells).

### 6.4.1. CD4⁺ T cells and T cell donors

CD4⁺ T cells used in poly-donor CD4^{IL-10} populations can be isolated from peripheral blood, cord blood, or other blood samples from a donor, using methods available in the art. In typical embodiments, CD4⁺ T cells are isolated from peripheral blood. In certain embodiments, CD4⁺ T cells are isolated from peripheral blood obtained from third party-blood banks.

In some embodiments, CD4⁺ T cells are isolated from a prior-frozen stock of blood or a prior-frozen stock of peripheral blood mononuclear cells (PBMCs). In some embodiments, CD4⁺ T cells are isolated from peripheral blood or from PBMCs that have not previously been frozen. In some embodiments, the CD4⁺ T cells are separately isolated from blood or PBMCs obtained from a plurality of donors, and then pooled. In some embodiments, the CD4⁺ T cells are isolated from blood or PBMCs that have first been pooled from a plurality of donors.

In some embodiments, the CD4⁺ T cells are obtained from three, four, five, six, seven, eight, nine, or ten different T cell donors.

In some embodiments, the at least three different T cell donors are selected without regard to genotype. In some embodiments, the at least three different T cell donors are selected based on genotype.

In certain embodiments, the at least three different T cell donors are selected based on their HLA haplotypes.

In some embodiments, some or all of the at least three different T cell donors have matching HLA haplotypes. In some embodiments, some or all of the at least three different T cell donors have a mis-matched HLA haplotype.

In some embodiments, all of the CD4⁺ T cells in the population have at least 5/10, 6/10, 7/10, 8/10, or 9/10 match at the HLA-A, HLA-B, HLA-C, HLA-DRB1, and HLA-DQB1 loci to each other. In some embodiments, all of the CD4⁺ T cells in the population have at least 4/8, 5/8, 6/8, 7/8, or 8/8 match at the HLA-A, HLA-B, HLA-C, and HLA-DRB1 loci to each other. In some embodiments, all the CD4⁺ T cells in the population have 2/2 match at the HLA-A locus to each other. In some embodiments, all the CD4⁺ T cells in the population have 2/2 match at the HLA-B locus to each other. In some embodiments, all the CD4⁺ T cells in the population have 2/2 match at the HLA-C locus to each other. In some embodiments, all the CD4⁺ T cells in the population have at least 3/4 or 4/4 match at the HLA-DRB1 and HLA DQB1 loci with each other. In some embodiments, all the CD4⁺ T cells in the population have an A*02 allele.

In preferred embodiments, none of the at least three different T cell donors is a host to be treated with the CD4^{IL-10} cells. In preferred embodiments, none of the at least three different T cell donors is a donor of stem cells (e.g., HSC), tissue or organ that will be used together with the CD4^{IL-10} cells in the methods of treatment described herein.

In some embodiments, one or more of the T cell donors are HLA-mismatched or partially HLA-mismatched to the patient to be treated (host). In some embodiments, one or more of the T cell donors have less than 5/10, 6/10, 7/10, 8/10, 9/10 or 10/10 match at the HLA-A, HLA-B, HLA-C, HLA-DRB1, and HLA-DQB1 loci to the patient. In some embodiments, one or more of the T cell donors have less than 4/8, 5/8, 6/8, 7/8, or 8/8 match at the HLA-A, HLA-B, HLA-C, and HLA-DRB1 loci to the patient. In some embodiments, one or more of the T cell donors have less than 2/2 match at the HLA-A, HLA-B, or HLA-C locus to the patient. In some embodiments, one or more of the T cell donors have less than 2/4, 3/4 or 4/4 match at the HLA-DRB1 and HLA-DQB1 loci to the patient.

In some embodiments, one or more of the T cell donors are HLA-mismatched or partially HLA-mismatched with the HSC donor. In some embodiments, one or more of the T cell donors have less than 5/10, 6/10, 7/10, 8/10, 9/10 or 10/10 match at the HLA-A, HLA-B, HLA-C, HLA-DRB1, and HLA-DQB1 loci to the HSC donor. In some embodiments, one or more of the T cell donors have less than 4/8, 5/8, 6/8, 7/8, or 8/8 match at the HLA-A, HLA-B, HLA-C, and HLA-DRB1 loci to the HSC donor. In some embodiments, one or more of the T cell donors have less than 2/2 match at the HLA-A, HLA-B, or HLA-C locus to the HSC donor. In some embodiments, one or more of the T cell donors have less than 3/4 or 4/4 match at the HLA-DRB1 and HLA-DQB1 loci to the HSC donor.

In the preferred embodiments, none of the CD4⁺ T cells is immortalized.

### 6.4.2. Exogenous polynucleotide encoding IL-10

Poly-donor CD4^{IL-10} cells of the present disclosure are CD4⁺ T cells that have been genetically modified to comprise an exogenous polynucleotide encoding IL-10. The exogenous polynucleotide comprises an IL-10-encoding polynucleotide segment operably linked to expression control elements.

The IL-10-encoding polynucleotide segment can encode IL-10 of a human, bonobo or rhesus. In some embodiments, the IL-10-encoding polynucleotide segment encodes human IL-10 having the sequence of SEQ ID NO: 1. In some embodiments, the IL-10-encoding polynucleotide segment encodes a variant of human IL-10 having at least 90%, 95%, 98%, or 99% sequence identity to SEQ ID NO: 1. In some embodiments, the IL-10-encoding polynucleotide segment has the nucleotide sequence of SEQ ID NO:2. In some embodiments, the IL-10-encoding polynucleotide segment has at least 90%, 95%, 98%, or 99% sequence identity to SEQ ID NO: 2.

In some embodiments, the exogenous polynucleotide encodes viral-IL-10. In various embodiments, the exogenous polypeptide encodes IL-10 from HCMV, GMCMV, RhCMV, BaCMV, MOCMV, SMCMV, EBV, Bonobo-HV, BaLCV, OvHV-2, EHV-2, CyHV-3, AngHV-1, ORFV, BPSV, PCPV, LSDV, SPV, GPV, or CNPV. In some embodiments, the exogenous polypeptide encodes viral IL-10 from EBV or ORFV.

The exogenous polynucleotide further comprises expression control elements that direct expression of the encoded IL-10 in transduced CD4⁺ T cells.

In some embodiments, the expression control elements comprise a promoter capable of directing expression of IL-10 in CD4⁺ T cells. In some embodiments, the promoter drives constitutive expression of IL-10 in CD4⁺ T cells. In some embodiments, the promoter drives expression of IL-10 in activated CD4⁺ T cells.

In some embodiments, an inducible promoter is used to induce expression of IL-10 when therapeutically appropriate. In some embodiments, the IL-10 promoter is used. In some embodiments a tissue-specific promoter is used. In some embodiments, a lineage-specific promoter is used. In some embodiments, a ubiquitously expressed promoter is used.

In some embodiments, a native human promoter is used. In some embodiments, a human elongation factor (EF)1α promoter is used. In some embodiments, a human phosphoglycerate kinase promoter (PGK) is used. In some embodiments, a human ubiquitin C promoter (UBI-C) is used.

In some embodiments, a synthetic promoter is used. In certain embodiments, a minimal CMV core promoter is used. In particular embodiments, an inducible or constitutive bidirectional promoter is used. In specific embodiments, the synthetic bidirectional promoter disclosed in Amendola et al., Nature Biotechnology, 23(1):108-116 (2005) is used. This promoter can mediate coordinated transcription of two mRNAs in a ubiquitous or a tissue-specific manner. In certain embodiments, the bidirectional promoter induces expression of IL-10 and a selection marker.

In some embodiments, the exogenous polynucleotide further comprises a segment encoding a selection marker that permits selection of successfully transduced CD4⁺ T cells. In some embodiments, the selection marker is ΔNGFR. In certain embodiments, the selection marker is a polypeptide having the sequence of SEQ ID NO:3. In certain embodiments, the selection marker is a polypeptide having at least 90%, 95%, 98%, or 99% sequence identity to SEQ ID NO: 3. In particular embodiments, the nucleotide sequence encoding the ΔNGFR selection marker has the sequence of SEQ ID NO: 4. In some embodiments, the nucleotide sequence encoding the ΔNGFR selection marker has at least 90%, 95%, 98%, or 99% sequence identity to SEQ ID NO: 4.

In some embodiments, the selection marker is a truncated form of EGFR polypeptide. In some embodiments, the selection marker is a truncated form of the human EGFR polypeptide, optionally huEGFRt disclosed in Wang et al. "A transgene-encoded cell surface polypeptide for selection, in vivo tracking, and ablation of engineered cells", Blood, v. 118, n. 5 (2011), incorporated by reference in its entirety herein.

In some embodiments, the exogenous polynucleotide further comprises a sequence encoding an antibiotic resistance gene. In some embodiments, the exogenous polynucleotide comprises a sequence encoding an ampicillin resistance gene. In some embodiments, the exogenous polynucleotide comprises a sequence encoding a kanamycin resistance gene.

In typical embodiments, the exogenous polynucleotide is delivered into CD4⁺ T cells using a vector. In some embodiments, the vector is a plasmid vector. In some embodiments, the vector is a viral vector.

In certain embodiments, the exogenous polynucleotide is delivered into CD4⁺ T cells using a lentiviral vector and the exogenous polynucleotide comprises lentiviral vector sequences. In certain embodiments, a lentiviral vector disclosed in Mátrai et al., Molecular Therapy 18(3):477-490 (2010) ("Mdtrai"), incorporated by reference herein, is used.

In some embodiments, the lentiviral vector is capable of integrating into the T cell nuclear genome. In some embodiments, the lentiviral vector is not capable of integrating into T cell nuclear genome. In some embodiments, an integration-deficient lentiviral vector is used. For example, in some embodiments, an integration-deficient or other lentiviral vector disclosed in Mátrai is used. In some embodiments, an integrase-defective lentivirus is used. For example, an integrase-defective lentivirus containing an inactivating mutation in the integrase (D64V) can be used as described in Mátrai et al., Hepatology 53:1696-1707 (2011), which is incorporated by reference herein, is used.

In some embodiments, the exogenous polynucleotide is integrated in the T cell nuclear genome. In some embodiments, the exogenous polynucleotide is not integrated in the nuclear genome. In some embodiments, the exogenous polynucleotide exists in the T cell cytoplasm.

In particular embodiments, the exogenous polynucleotide has the sequence of SEQ ID NO:5. In some embodiments, the exogenous polynucleotide has at least 90%, 95%, 98%, or 99% sequence identity to SEQ ID NO: 5.

### 6.4.3. Gene expression of poly-donor CD4^{1L-10} T cells

Poly-donor CD4^{IL-10} T cells express IL-10. In some embodiments, poly-donor CD4^{IL-10} T cells constitutively express IL-10. In some embodiments, poly-donor CD4^{IL-10} T cells express IL-10 when activated.

In some embodiments, poly-donor CD4^{IL-10} T cells constitutively express at least 100 pg of IL-10 per 10⁶ of the CD4⁺ T cells/ml of culture. In some embodiments, poly-donor CD4^{IL-10} T cells constitutively express at least 200pg, 500pg, 1ng, 5ng, 10ng, or 50ng of IL-10 per 10⁶ of the CD4⁺ T cells/ml of culture.

In some embodiments, poly-donor CD4^{IL-10} T cells express at least 1ng or 2ng IL-10 per 10⁶ of the CD4⁺ T cells/ml of culture after activation with a combination of anti-CD3 and anti-CD28 antibodies, or anti-CD3 antibody and anti-CD28 antibody coated beads. In some embodiments, poly-donor CD4^{IL-10} T cells express at least 5ng, 10ng, 100ng, 200ng, or 500ng IL-10 per 10⁶ of the CD4⁺ T cells/ml of culture after activation with anti-CD3 and anti-CD28 antibodies or CD3 antibody and CD28 antibody coated beads.

In various embodiments, the amount of IL-10 production is determined 12 hours, 24 hours, or 48 hours after activation using various methods for protein detection and measurement, such as ELISA, spectroscopic procedures, colorimetry, amino acid analysis, radiolabeling, Edman degradation, HPLC, western blotting, etc. In preferred embodiments, the amount of IL-10 production is determined by ELISA 48 hours after activation with anti-CD3 and anti-CD28 antibodies.

In some embodiments, poly-donor CD4^{IL-10} T cells express IL-10 at a level at least 5-fold higher than unmodified CD4⁺ T cells. In some embodiments, poly-donor CD4^{IL-10} T cells express IL-10 at a level at least 6, 7, 8, 9, 10, 11, 12, 13, 14, 15, 20, 30, 40, or 50-fold higher than unmodified CD4⁺ T cells.

In some embodiments, poly-donor CD4^{IL-10} T cells further express a selection marker. In some embodiments, poly-donor CD4^{IL-10} T cells express a protein typically expressed in Tr1 cells. In some embodiments, poly-donor CD4^{IL-10} T cells express a marker protein characteristic of Tr1 cells.

In some embodiments, poly-donor CD4^{IL-10} T cells express CD49b. In some embodiments, poly-donor CD4^{IL-10} T cells express LAG-3. In some embodiments, poly-donor CD4^{IL-10} T cells express TGF-β. In some embodiments, poly-donor CD4^{IL-10} T cells express IFNy. In some embodiments, poly-donor CD4^{IL-10} T cells express GzB. In some embodiments, poly-donor CD4^{IL-10} T cells release GzB when activated with myeloid antigen-presenting cells. In some embodiments, poly-donor CD4^{IL-10} T cells express perforin. In some embodiments, poly-donor CD4^{IL-10} T cells release perforin when activated with myeloid antigen-presenting cells. In some embodiments, poly-donor CD4^{IL-10} T cells express CD18. In some embodiments, poly-donor CD4^{IL-10} T cells express CD2. In some embodiments, poly-donor CD4^{IL-10} T cells express CD226. In some embodiments, poly-donor CD4^{IL-10} T cells express IL-22. In some embodiments, poly-donor CD4^{IL-10} T cells express IL-10.

In some embodiments, poly-donor CD4^{IL-10} T cells exhibit at least one phenotypic function of Tr1 cells. In various embodiments, the function is secretion of IL-10, secretion of TGF-β, and by the specific killing of myeloid antigen-presenting cells through the release of Granzyme B (GzB) and perforin.

### 6.4.4. Product by process

In typical embodiments, poly-donor CD4^{IL-10} T cells are obtained by modifying CD4⁺ T cells with an exogenous polynucleotide encoding IL-10.

In some embodiments, the exogenous polynucleotide is introduced to CD4⁺ T cells by a viral vector or a plasmid vector. In particular embodiments, CD4⁺ T cells are transduced with a lentivirus containing a coding sequence of IL-10.

In some embodiments, poly-donor CD4^{IL-10} T cells are generated by (i) pooling primary CD4⁺ T cells obtained from at least three different T cell donors; and (ii) modifying the pooled CD4⁺ T cells by introducing an exogenous polynucleotide encoding IL-10. In some embodiments, poly-donor CD4^{IL-10} T cells are generated by (i) obtaining primary CD4⁺ T cells from at least three different T cell donors; (ii) separately modifying each donor's CD4⁺ T cells by introducing an exogenous polynucleotide encoding IL-10, and then (iii) pooling the genetically modified CD4⁺ T cells.

In some embodiments, poly-donor CD4^{IL-10} T cells have been cultured in the presence of proteins capable of activating CD4⁺ T cells. In some embodiments, poly-donor CD4^{IL-10} T cells have been cultured in the presence of anti-CD3 antibody and anti-CD28 antibody, or anti-CD3 antibody and anti-CD28 antibody coated beads. In some embodiments, poly-donor CD4^{IL-10} T cells have been cultured in the presence of anti-CD3 antibodies, anti-CD28 antibodies, and IL-2, or anti-CD3 antibody and anti-CD28 antibody coated beads and IL-2. In some embodiments, poly-donor CD4^{IL-10} T cells have been cultured in the presence of T Cell TransAct^{™} from Miltenyi Biotec. In some embodiments, poly-donor CD4^{IL-10} T cells have been cultured in the presence of ImmunoCult Human T Cell Activator^{™} from STEMCELL Technologies.

In some embodiments, poly-donor CD4^{IL-10} T cells are in a frozen stock.

### 6.5. Pharmaceutical compositions

In another aspect, pharmaceutical compositions are provided. The pharmaceutical comprises the poly-donor CD4^{IL-10} T cells disclosed herein and a pharmaceutically acceptable carrier or diluent.

The pharmaceutical composition can be formulated for administration by any route of administration appropriate for human or veterinary medicine. In typical embodiments, the composition is formulated for intravenous (IV) administration. In some embodiments, the composition is formulated for intravenous (IV) infusion. In embodiments formulated for IV administration, the pharmaceutical composition will be in the form of a parenterally acceptable aqueous solution which is pyrogen-free and has suitable pH, isotonicity and stability.

In some embodiments, the pharmaceutically acceptable carrier or diluent is saline, lactated Ringer's solution, or other physiologically compatible solution. In various embodiments, the pharmaceutical composition solution comprises 2-20%, preferably 5 %, human serum albumin.

In some embodiments, unit dosage forms of the pharmaceutical composition are provided that are adapted for administration of the pharmaceutical composition by systemic administration, in particular, for intravenous administration.

In some embodiments, the unit dosage form contains 10⁴ to 10¹¹ poly-donor CD4^{IL-10} T cells, 10⁴ to 10¹⁰ poly-donor CD4^{IL-10} T cells, 10⁴ to 10⁹ poly-donor CD4^{IL-10} T cells, 10⁵ to 10¹⁰ poly-donor CD4^{IL-10} T cells, 10⁵ to 10⁹ poly-donor CD4^{IL-10} T cells, 10⁵ to 10⁸ polydonor CD4^{IL-10} T cells, or 10⁵ to 10⁷ poly-donor CD4^{IL-10} T cells.

In typical embodiments, the pharmaceutical composition in the unit dosage form is in liquid form.

### 6.6. Methods of making poly-donor CD4^{1L-10} cells

In another aspect, the present disclosure provides a method of making poly-donor CD4^{IL-10} cells.

In some embodiments, the method comprises the steps of: (i) pooling primary CD4⁺ T cells obtained from at least three different T cell donors; and (ii) modifying the pooled CD4⁺ T cells by introducing an exogenous polynucleotide encoding IL-10. In other embodiments, the method comprises the steps of: (i) obtaining primary CD4⁺ T cells from at least three different T cell donors; (ii) separately modifying each donor's CD4⁺ T cells by introducing an exogenous polynucleotide encoding IL-10; and then (iii) pooling the genetically modified CD4⁺ T cells, thereby obtaining the poly-donor CD4^{IL-10} cells. Various methods known in the art can be used to introduce an exogenous polynucleotide encoding IL-10 to primary CD4⁺ T cells.

In some embodiments, the method further comprises the step of incubating the primary CD4⁺ T cells or genetically-modified CD4⁺ T cells in the presence of an anti-CD3 antibody and anti-CD28 antibody, or anti-CD3 antibody and anti-CD28 antibody coated beads. In some embodiments, the method further comprises the step of incubating the primary CD4⁺ T cells or genetically-modified CD4⁺ T cells in the presence of anti-CD3 antibody, anti-CD28 antibody and IL-2 or anti-CD3 antibody and anti-CD28 antibody coated beads and IL-2. In some embodiments, the method further comprises the step of incubating the primary CD4⁺ T cells or genetically-modified CD4⁺ T cells in the presence of a mixture of feeder cells. In some embodiments, the method further comprises the step of incubating the primary CD4⁺ T cells or genetically-modified CD4⁺ T cells in the presence of nanopreparations of anti-CD3 antibody and anti-CD28 antibody. In some embodiments, the incubation is done in the presence of T Cell TransAct^{™} from Miltenyi Biotec. In some embodiments, the incubation is done in the presence of ImmunoCult Human T Cell Activator^{™} from STEMCELL Technologies.

In some embodiments, the incubation step is performed before introducing an exogenous polynucleotide encoding IL-10. In some embodiments, the incubation step is performed after (i) pooling primary CD4⁺ T cells obtained from at least three different T cell donors; but before (ii) modifying the pooled CD4⁺ T cells by introducing an exogenous polynucleotide encoding IL-10. In some embodiments, the incubation step is performed after (i) obtaining primary CD4⁺ T cells from at least three different T cell donors; but before (ii) separately modifying each donor's CD4⁺ T cells by introducing an exogenous polynucleotide encoding IL-10.

In some embodiments, the incubation step is performed after step (ii). In other words, in some embodiments, the incubation step is performed after (ii) modifying the pooled CD4⁺ T cells by introducing an exogenous polynucleotide encoding IL-10. In some embodiments, the incubation step is performed after (ii) separately modifying each donor's CD4⁺ T cells by introducing an exogenous polynucleotide encoding IL-10, but before (iii) pooling the genetically modified CD4⁺ T cells, thereby obtaining the genetically-modified CD4⁺ T cells. In some embodiments, the incubation step is performed after (iii) pooling the genetically modified CD4⁺ T cells, thereby obtaining the poly-donor CD4^{IL-10} cells.

In some embodiments, the incubation step is performed more than once. In some embodiments, the incubation step is performed both before and after genetic modification of CD4⁺ T cells.

In some embodiments, the exogenous polynucleotide is introduced into the primary CD4⁺ T cells using a viral vector. In some embodiments, the viral vector is a lentiviral vector. In some embodiments, the exogenous polynucleotide comprises a segment encoding IL-10 having the sequence of SEQ ID NO: 1. In some embodiments, the exogenous polynucleotide comprises a segment encoding IL-10 having at least 90%, 95%, 98%, or 99% sequence identity to SEQ ID NO: 1. In some embodiments, the IL-10-encoding polynucleotide segment has the sequence of SEQ ID NO: 2. In some embodiments, the IL-10-encoding polynucleotide segment has at least 90%, 95%, 98%, or 99% sequence identity to SEQ ID NO: 2. In some embodiments, the exogenous polynucleotide further comprises a segment encoding a marker permitting selection of successfully transduced CD4⁺ T cells. In some embodiments, the encoded selection marker is ΔNGFR. In certain embodiments, the encoded selection marker has the sequence of SEQ ID NO:3. In particular embodiments, the exogenous polynucleotide comprises a sequence of SEQ ID NO:4. In some embodiments, the encoded selection marker is a truncated form of human EGFR polypeptide.

In some embodiments, the method further comprises the step of isolating the genetically-modified CD4⁺ T cells expressing the selection marker, thereby generating an enriched population of genetically-modified CD4^{IL-10} cells.

In some embodiments, at least 90% of the genetically-modified CD4⁺ T cells in the enriched population express a selection marker. In some embodiments, at least 95% of the genetically-modified CD4⁺ T cells in the enriched population express a selection marker. In some embodiments, at least 96, 97, 98, or 99% of the genetically-modified CD4⁺ T cells in the enriched population express a selection marker.

In some embodiments, at least 90% of the genetically-modified CD4⁺ T cells in the enriched population express IL-10. In some embodiments, at least 95% of the genetically-modified CD4⁺ T cells in the enriched population express IL-10. In some embodiments, at least 96, 97, 98, or 99% of the genetically-modified CD4⁺ T cells in the enriched population express IL-10.

In some embodiments, the method further comprises the step of incubating the enriched population of the genetically-modified CD4⁺ T cells. In some embodiments, the incubation is performed in the presence of anti-CD3 antibody and anti-CD28 antibody, or anti-CD3 antibody and anti-CD28 antibody coated beads. In some embodiments, the incubation is performed further in presence of IL-2. In some embodiments, the incubation is performed in the presence of feeder cells. In some embodiments, the incubation is performed in the presence of nanopreparations of anti-CD3 antibody and anti-CD28 antibody. In some embodiments, the incubation is performed in the presence of T Cell TransAct^{™} from Miltenyi Biotec. In some embodiments, the incubation is performed in the presence of ImmunoCult Human T Cell Activator^{™} from STEMCELL Technologies.

In some embodiments, the method further comprises the step of freezing the genetically-modified CD4⁺ T cells.

In some embodiments, the primary CD4⁺ T cells are from donors selected based on their HLA haplotypes. In some embodiments, the method further comprises the step of selecting T cell donors by analyzing their genetic information. In some embodiments, the method comprises the step of analyzing genetic information or HLA haplotype of potential T cell donors.

In some embodiments, the primary CD4⁺ T cells are from donors having at least a partial HLA match with a host to be treated with the primary CD4⁺ T cells or a modification thereof. In some embodiments, the primary CD4⁺ T cells are from donors having at least a partial HLA match with a stem cell (HSC), tissue or organ donor. In some embodiments, the primary CD4⁺ T cells are obtained from third party donors who are not biologically related with a host. In some embodiments, the primary CD4⁺ T cells are obtained from third party donors who are not biologically related with a stem cell, tissue or organ donor.

In some embodiments, in step (i), the primary CD4⁺ T cells are obtained from three, four, five, six, seven, eight, nine, or ten different T cell donors. In some embodiments, the at least three T cell donors have at least 5/10, 6/10, 7/10, 8/10, or 9/10 match at the HLA-A, HLA-B, HLA-C, HLA-DRB1, and HLA-DQB1 loci to each other. In some embodiments, the at least three T cell donors have at least 4/8, 5/8, 6/8, 7/8, or 8/8 match at the HLA-A, HLA-B, HLA-C, and HLA-DRB1 loci to each other. In some embodiments, the at least three T cell donors have 2/2 match at the HLA-A locus to each other. In some embodiments, the at least three T cell donors have 2/2 match at the HLA-B locus to each other. In some embodiments, the at least three T cell donors have 2/2 match at the HLA-C locus to each other. In some embodiments, the at least three T cell donors have at least 3/4 or 4/4 match at the HLA-DRB1 and HLA-DQB 1 loci to each other. In some embodiments, each of the at least three T cell donors has an A*02 allele.

In some embodiments, in step (i), the primary CD4⁺T cells are obtained from one or more frozen stocks. In some embodiments, in step (i), the primary CD4⁺T cells are obtained from unfrozen peripheral blood mononuclear cells of the at least three different T cell donors. In some embodiments, the method further comprises the step of isolating CD4⁺ T cells from the peripheral blood mononuclear cells. In some embodiments, in step (i), the primary CD4⁺T cells are obtained from a liquid suspension. In some embodiments, the liquid suspension is obtained from a previously frozen stock.

In some embodiments, CD4⁺ T cells from donors are contacted with patient antigen-presenting cells (monocytes, dendritic cells, or DC-10 cells), generating allo-specific CD4⁺ T cells that are then modified to produce high levels of IL-10 (allo-CD4^{IL-10} cell).

In some embodiments, the method does not comprise the step of anergizing the CD4⁺ T cells in the presence of peripheral blood mononuclear cells (PBMCs) from a host. In some embodiments, the method does not comprise the step of anergizing the CD4⁺ T cells in the presence of recombinant IL-10 protein, wherein the recombinant IL-10 protein is not expressed from the CD4⁺ T cells. In some embodiments, the method does not comprise the step of anergizing the CD4⁺ T cells in the presence of DC10 cells from a host.

### 6.7. Methods of using poly-donor CD4^{IL-10} cells

In yet another aspect, the present disclosure provides a method of treating a patient, comprising the step of administering the poly-donor CD4^{IL-10} cells or the pharmaceutical composition provided herein to a patient in need of immune tolerization.

In some embodiments, the method further comprises the preceding step of thawing a frozen suspension of poly-donor CD4^{IL-10} cells.

In some embodiments, the poly-donor CD4^{IL-10} cells or the pharmaceutical composition prevents or reduces severity of pathogenic T cell response in the patient.

In some embodiments, the treatment method further comprises monitoring polydonor CD4^{IL-10} cells in a patient after administration. In some embodiments, the method comprises the step of detecting a selection marker in a biological sample obtained from the patient, thereby detecting presence or absence of poly-donor CD4^{IL-10} T cells. In some embodiments, the selection marker is detected at multiple time points to trace changes in presence of poly-donor CD4^{IL-10} cells in a patient. In some embodiments, the biological sample is a biopsy or blood sample from the patient.

The poly-donor CD4^{IL-10} T cells are administered in a therapeutically effective amount. The amount can be determined based on the body weight and other clinical factors. In some embodiments, 10³ to 10⁹ cells/kg are administered. In some embodiments, 10³ to 10⁸ cells/kg are administered. In some embodiments, 10³ to 10⁷ cells/kg are administered. In some embodiments, 10³ to 10⁶ cells/kg are administered. In some embodiments, 10³ to 10⁵ cells/kg are administered. In some embodiments, 10³ to 10⁴ cells/kg are administered.

In various embodiments, poly-donor CD4^{IL-10} T cells are administered on a therapeutically effective schedule. In some embodiments, poly-donor CD4^{IL-10} T cells are administered once. In some embodiments, poly-donor CD4^{IL-10} cells are administered every day, every 3 days, every 7 days, every 14 days, every 21 days, or every month.

The poly-donor CD4^{IL-10} T cells can be administered according to different administration routes, such as systemically, subcutaneously, or intraperitoneally. In some embodiments, the cells are administered within a saline or physiological solution which may contain 2-20%, preferably 5 % human serum albumin.

### 6.7.1. Methods of reducing or preventing GvHD

In some embodiments, the poly-donor CD4^{IL-10} cells or the pharmaceutical composition comprising poly-donor CD4^{IL-10} cells is used to treat a patient before a hematopoietic stem cell (HSC) transplant (HSCT), concurrently with an HSCT, or following an HSCT.

In various embodiments, the HSCT is a matched related HSCT. In various embodiments, the HSCT is a haploidentical HSCT, a mismatched related HSCT, or a mismatched unrelated HSCT.

In some embodiments, the patient has a hematological malignancy which requires treatment with allo-HSCT. In some embodiments, the hematological malignancy is mediated by aberrant myeloid cells.

In some embodiments, T cell donors are selected based on genetic information of a patient to be treated with poly-donor CD4^{IL-10} cells and HSC, and/or genetic information of the HSC donor. In some embodiments, T cell donors are selected based on HLA haplotype of a patient to be treated with poly-donor CD4^{IL-10} cells and HSC, and/or HLA haplotype of the HSC donor. In some embodiments, the method further comprises the step, prior to administering CD4^{IL-10} cells, of analyzing genetic information or HLA haplotype of T cell donors. In some embodiments, the method further comprises the step of analyzing genetic information or HLA haplotype of a host. In some embodiments, the method further comprises the step of analyzing genetic information or HLA haplotype of an HSC donor.

In some embodiments, T cell donors, a host and an HSC donor are not biologically related. In some embodiments, T cell donors, a host and an HSC donor have different HLA haplotypes. In some embodiments, T cell donors, a host and an HSC donor have at least partial mismatch in HLA haplotype. In some embodiments, T cell donors are selected when they have HLA haplotype with an HLA match over a threshold value.

In some embodiments, the HSC donor is partially HLA mismatched to the patient. In some embodiments, the HSC donor has less than 5/10, 6/10, 7/10, 8/10, 9/10 or 10/10 match at the HLA-A, HLA-B, HLA-C, HLA-DRB1, and HLA-DQB1 loci to the patient. In some embodiments, the HSC donor has less than 4/8, 5/8, 6/8, 7/8, or 8/8 match at the HLA-A, HLA-B, HLA-C, and HLA-DRB1 loci to the patient. In some embodiments, the HSC donor has less than 2/2 match at the HLA-A, HLA-B, or HLA-C locus to the patient. In some embodiments, the HSC donor has less than 3/4 or 4/4 match at the HLA-DRB1 and HLA-DQB1 loci to the patient.

In some embodiments, one or more of the T cell donors are HLA-mismatched or partially HLA-mismatched to the patient. In some embodiments, one or more of the T cell donors have less than 5/10, 6/10, 7/10, 8/10, 9/10 or 10/10 match at the HLA-A, HLA-B, HLA-C, HLA-DRB1, and HLA-DQB1 loci to the patient. In some embodiments, one or more of the T cell donors have less than 4/8, 5/8, 6/8, 7/8, or 8/8 match at the HLA-A, HLA-B, HLA-C, and HLA-DRB1 loci to the patient. In some embodiments, one or more of the T cell donors have less than 2/2 match at the HLA-A, HLA-B, or HLA-C locus to the patient. In some embodiments, one or more of the T cell donors have less than 2/4, 3/4 or 4/4 match at the HLA-DRB1 and HLA-DQB1 loci to the patient.

In some embodiments, one or more of the T cell donors are HLA-mismatched or partially HLA-mismatched with the HSC donor. In some embodiments, one or more of the T cell donors have less than 5/10, 6/10, 7/10, 8/10, 9/10 or 10/10 match at the HLA-A, HLA-B, HLA-C, HLA-DRB1, and HLA-DQB1 loci to the HSC donor. In some embodiments, one or more of the T cell donors have less than 4/8, 5/8, 6/8, 7/8, or 8/8 match at the HLA-A, HLA-B, HLA-C, and HLA-DRB1 loci to the HSC donor. In some embodiments, one or more of the T cell donors have less than 2/2 match at the HLA-A, HLA-B, or HLA-C locus to the HSC donor. In some embodiments, one or more of the T cell donors have less than 3/4 or 4/4 match at the HLA-DRB1 and HLA-DQB1 loci to the HSC donor. In some embodiments, the poly-donor CD4^{IL-10} cells or the pharmaceutical composition prevents or reduces severity of GvHD by the transplanted hematopoietic stem cells.

In some embodiments, the poly-donor CD4^{IL-10} cells or the pharmaceutical composition prevents or reduces severity of pathological T cell response by the transplanted hematopoietic cells. In specific embodiments, the poly-donor CD4^{IL-10} cells prevents or reduces GvHD.

### 6.7.2. Methods of treating cancer

In some embodiments, poly-donor CD4^{IL-10} cells are used for treatment of cancer. In preferred embodiments, the poly-donor CD4^{IL-10} cells directly mediate anti-tumor effects (Graft versus Tumor, GvT), and in particular embodiments, an anti-leukemic effect (Graft versus Leukemia, GvL).

In some embodiments, poly-donor CD4^{IL-10} cells are administered in combination with allogeneic mononuclear cells or PBMC for treatment of cancer. In some embodiments, poly-donor CD4^{IL-10} cells are administered prior to or subsequence to administration of PBMC. In some embodiments, poly-donor CD4^{IL-10} cells and allogeneic mononuclear cells or PBMC are administered concurrently.

In some embodiments, poly-donor CD4^{IL-10} cells and allogeneic mononuclear cells or PBMC are administered at 1:3, 1:2, 1:1, 2:1 or 3:1 ratio.

In some embodiments, the neoplastic cells express CD13. In some embodiments, the neoplastic cells express HLA-class 1. In some embodiments, the neoplastic cells express CD54. In some embodiments, the neoplastic cells express CD13, HLA-class I and CD54. In some embodiments, the neoplastic cells express CD112. In some embodiments, the neoplastic cells express CD58. In some embodiments, the neoplastic cells express CD155. In some embodiments, the tumor expresses CD112, CD58, or CD155. In various embodiments, the tumor is a solid or hematological tumor.

In some embodiments, the patient has a cancer selected from the group consisting of: Adrenal Cancer, Anal Cancer, Bile Duct Cancer, Bladder Cancer, Bone Cancer, Brain/CNS Tumors In Adults, Brain/CNS Tumors In Children, Breast Cancer, Breast Cancer In Men, Cancer of Unknown Primary, Castleman Disease, Cervical Cancer, Colon/Rectum Cancer, Endometrial Cancer, Esophagus Cancer, Ewing Family Of Tumors, Eye Cancer, Gallbladder Cancer, Gastrointestinal Carcinoid Tumors, Gastrointestinal Stromal Tumor (GIST), Gestational Trophoblastic Disease, Hodgkin Disease, Kaposi Sarcoma, Kidney Cancer, Laryngeal and Hypopharyngeal Cancer, Leukemia, Acute Lymphocytic (ALL), Acute Myeloid (AML, including myeloid sarcoma and leukemia cutis), Chronic Lymphocytic (CLL), Chronic Myeloid (CML) Leukemia, Chronic Myelomonocytic (CMML), Leukemia in Children, Liver Cancer, Lung Cancer, Lung Cancer with Non-Small Cell, Lung Cancer with Small Cell, Lung Carcinoid Tumor, Lymphoma, Lymphoma of the Skin, Malignant Mesothelioma, Multiple Myeloma, Myelodysplastic Syndrome, Nasal Cavity and Paranasal Sinus Cancer, Nasopharyngeal Cancer, Neuroblastoma, Non-Hodgkin Lymphoma, Non-Hodgkin Lymphoma In Children, Oral Cavity and Oropharyngeal Cancer, Osteosarcoma, Ovarian Cancer, Pancreatic Cancer, Penile Cancer, Pituitary Tumors, Prostate Cancer, Retinoblastoma, Rhabdomyosarcoma, Salivary Gland Cancer, Sarcoma - Adult Soft Tissue Cancer, Skin Cancer, Skin Cancer - Basal and Squamous Cell, Skin Cancer - Melanoma, Skin Cancer - Merkel Cell, Small Intestine Cancer, Stomach Cancer, Testicular Cancer, Thymus Cancer, Thyroid Cancer, Uterine Sarcoma, Vaginal Cancer, Vulvar Cancer, Waldenstrom Macroglobulinemia, and Wilms Tumor.

In some embodiments, the cancer is a myeloid tumor. In particular embodiments, the cancer is AML or CML. In some embodiments, the cancer is a myeloid tumor.

In some embodiments, the method is used to treat a hematological cancer affecting blood, bone marrow, and lymph nodes. In various embodiments, the hematological cancer is a lymphoma (e.g. Hodgkin's Lymphoma), lymphocytic leukemias, myeloma. In various embodiments, the hematological cancer is acute or chronic myelogenous (myeloid) leukemia (AML, CML), or a myelodysplastic syndrome.

In some embodiments, the cancer is refractory or resistant to a therapeutic intervention.

In some embodiments, the poly-donor CD4^{1L-10} cells are used in combination with a therapeutic intervention. The combination may be simultaneous or performed at different times. Preferably the therapeutic intervention is selected from the group consisting of : chemotherapy, radiotherapy, allo-HSCT, immune suppression, blood transfusion, bone marrow transplant, growth factors, biologicals.

In some embodiments, the poly-donor CD4^{IL-10} cells induce cell death of tumor infiltrating myeloid lineage cells (e.g., monocytes, macrophages, neutrophils).

### 6.7.3. Methods of treating other disorders

In some embodiments, poly-donor CD4^{IL-10} cells are administered to treat autoimmune disease.

In some embodiments, the autoimmune disease is selected from the group consisting of: type-1 diabetes, autoimmune uveitis, rheumatoid arthritis, psoriasis, psoriatic arthritis, multiple sclerosis, systemic lupus, inflammatory bowel disease, Addison's disease, Graves' disease, Sjögren's syndrome, Hashimoto's thyroiditis, myasthenia gravis, autoimmune vasculitis, pernicious anemia, ulcerative colitis, bullous diseases, scleroderma, and celiac disease. In some embodiments, the autoimmune disease is Crohn's disease, ulcerative colitis, celiac disease, type-1 diabetes, lupus, psoriasis, psoriatic arthritis, or rheumatoid arthritis. In some embodiments, the patient has an allergic or atopic disease. The allergic or atopic disease can be selected from the group consisting of: asthma, atopic dermatitis, and rhinitis. In some embodiments, the patient has a food allergy.

In some embodiments, poly-donor CD4^{IL-10} cells are administered to prevent or reduce severity of pathogenic T cell response to cell and organ transplantation other than HSCT. In some embodiments, the method comprises the step of organ transplantation to the patient, either prior to or subsequent to administration of poly-donor CD4^{IL-10} T cells or the pharmaceutical composition. In certain embodiments, the organ is a kidney, a heart, or pancreatic islet cells. In preferred embodiments, the poly-donor CD4^{IL-10} cells or the pharmaceutical composition prevents or reduces severity of host rejection of the organ transplantation.

In some embodiments, poly-donor CD4^{IL-10} cells are administered to prevent or reduce immune response associated with gene therapy, e.g., administration of recombinant AAV (rAAV). In these embodiments, the method further comprises the step of administering a recombinant AAV to the patient, either prior to or subsequent to administration of the poly-donor CD4^{IL-10} cells or the pharmaceutical composition.

In some embodiments, poly-donor CD4^{IL-10} cells are administered to prevent or reduce immune response associated with transplantation of iPS-derived tissues or cells. The iPS-derived tissues and cells include, but are not limited to cardiomyocytes, hepatocytes, epithelial cells, cartilage, bone and muscle cells, neurons.

In some embodiments, poly-donor CD^{4IL-10} cells are administered to treat inflammation. The inflammation can be related to coronary artery disease (CAD), Type 2 diabetes, neurodegenerative diseases, or inflammatory bowel disease, but is not limited thereto.

In some embodiments, poly-donor CD^{4IL-10} cells are administered to treat a disease or disorder involving hyperactivity of NLPR3 inflammasome. In some embodiments, poly-donor CD^{4IL-10} cells are administered to treat a disease or disorder involving increased IL-1β production by activated monocytes, macrophages or dendritic cells. In some embodiments, poly-donor CD4^{IL-10} cells are administered to treat a disease or disorder involving increased IL-18 production by activated monocytes, macrophages or dendritic cells. In some embodiments, poly-donor CD^{4IL-10} cells are administered to treat a disease or disorder involving increased mature caspase 1 production by activated monocytes, macrophages or dendritic cells.

In some embodiments, poly-donor CD^{4IL-10} cells are administered to reduce IL-1β production by activated monocytes, macrophages or dendritic cells. In some embodiments, polydonor CD^{4IL-10} cells are administered to reduce IL-18 production by activated monocytes, macrophages or dendritic cells. In some embodiments, poly-donor CD^{4IL-10} cells are administered to reduce mature caspase 1 production by activated monocytes, macrophages or dendritic cells.

In some embodiments, poly-donor CD4^{IL-10} cells are administered to reduce patient hyperactive immune response to viral infection. In some embodiments, the virus is SARS-coV-2. In some embodiments, poly-donor CD4^{IL-10} cells are administered to reduce hyperactive immune responses to bacterial infections, such as toxic shock and cytokine storm.

### 6.8. Examples

The following examples are provided by way of illustration not limitation.

### 6.8.1. Example 1: Generation of poly-donor CD4^{IL-10} cells

### Vector production

Poly-donor CD4^{IL-10} cells were produced by transduction with a lentiviral vector containing coding sequences of both the human IL-10 and a truncated form of the NGFR (ΔNGFR) (FIGs. 1 and 2), as described in WO2016/146542, incorporated by reference in its entirety herein. The sequence of the vector is provided as SEQ ID NO:5. In short, the lentiviral vector was generating by ligating the coding sequence of human IL-10 from 549 bp fragment of pH15C (ATCC 68192)) into plasmid #1074.1071.hPGK.GFP.WPRE.mhCMV.dNGFR.SV40PA. The presence of the bidirectional promoter (human PGK promoter plus minimal core element of the CMV promoter in the opposite direction) allows co-expression of the two transgenes. The plasmid further contains a coding sequence of an antibiotic resistance gene (e.g., ampicillin or kanamycin).

The lentiviral vectors were produced by Ca₃PO₄ transient four-plasmid cotransfection into 293T cells and concentrated by ultracentrifugation: 1 µM sodium butyrate was added to the cultures for vector collection. Titer was estimated on 293T cells by limiting dilution, and vector particles were measured by HIV-1 Gag p24 antigen immune capture (NEN Life Science Products; Waltham, MA). Vector infectivity was calculated as the ratio between titer and particle. For concentrated vectors, titers ranged from 5x10⁸ to 6x10⁹ transducing units/ml, and infectivity from 5x10⁴ to 5x10⁵ transducing units/ng.

### Production of CD4^{IL-10} cells

FIG. 3 is a schematic representation of the production process of CD4^{IL-10} cells. CD4⁺ T cells from healthy donors were purified. Human CD4⁺ T cells were activated with soluble anti-CD3, soluble anti-CD28 mAbs, and rhIL-2 (50 U/mL) for 48 hours before transduction with a bidirectional lentiviral vector encoding for human IL-10 and a truncated form the human NGF receptor (LV-IL-10/ΔNGFR) at multiplicity of infection (MOI) of 20.

After 11 days, transduced cells were analyzed by FACS for the expression of ΔNGFR, and the vector copy number (VCN) was quantified by digital droplet PCR (ddPCR).

The mean transduction efficiency of CD4⁺ T cells from 10 different donors was 45 ± 17% with VCN of 2.7 ± 0.6%. FIG. 4A shows percentages of CD4⁺ΔNGFR⁺ cells (mean± SD, n=10 left bar) and vector copy numbers (VCN, mean ± SD, n=10 right bar) in human CD4⁺ T cells transduced with LV-IL-10/ΔNGFR (a bidirectional lentiviral vector encoding for human IL-10 and a truncated form the human NGF receptor). The frequency of CD4⁺ΔNGFR⁺cells and the vector copy numbers were quantified by digital droplet PCR (ddPCR) in CD4^{IL-10} cells.

ΔNGFR⁺ T cells were purified using anti-CD271 mAb-coated microbeads and resulted in > 95% pure CD4^{IL-10} cells populations. After purification, cells were stained with markers for CD4 and ΔNGFR and analyzed by FACS. The data showed purity resulting from the purification step was over 98%. FIG. 4B shows FACS data from two representative donors (Donor B and Donor C) out of 10 donors tested. The purified CD4^{IL-10} cells were restimulated 3 times at 14 day intervals and their *in vitro* and *in vivo* functions were tested after the second (TF2) and or third restimulation (TF3) functions.

Resting CD4^{IL-10} cells produced IL-10 constitutively. Upon activation, the level of IL-10 produced was strongly enhanced.

### CD4^{IL-10} cells have a cytokine production profile which is comparable to that of naturally derived Tr1 cells.

Cytokine production profiles of single donor CD4^{IL-10} cells were analyzed after the second (TF2) and third (TF3) restimulation and the results are provided in FIG. 5. Specifically, CD4^{IL-10} cells (2x10⁵ cells in 200 µl) were restimulated as previously described (Andolfi et al. Mol Ther. 2012;20(9):1778-1790 and Locafaro et al.Mol Ther. 2017;25(10):2254-2269). At day 14, after the 2^{nd} round (TF2) and 3^{rd} round (TF3) of restimulation, CD4^{IL-10} cells were left unstimulated (orange bar) or stimulated with immobilized anti-CD3 and soluble anti-CD28 mAbs (grey bars) for 48hrs. Culture supernatants were collected and levels of IL-10, IL-4, IL-5, IFN-γ and IL-22 were determined by ELISA. All samples were tested in triplicate. Mean± SD, n=8 donors tested are presented. The results provided in FIG. 5 show that CD4^{IL-10} cells stimulated with immobilized anti-CD3 and soluble anti-CD28 mAbs show a Tr1 cell cytokine production profile.

Although considerable variations between the different donors were observed, the overall cytokine production profiles after the second (TF2) or the third (TF3) restimulation were comparable and reflected those of Tr1 cells (Roncarolo et al., Immunity, 2018). Like Tr1 cells, the CD4^{IL-10} cells produced high levels of IL-10, IL-5, IFN-γ and IL-22, but low levels of IL-4 and undetectable levels of IL-2.

### CD4^{IL-10} cells express high levels of Granzyme B and selectively kill myeloid leukemia cells

The CD4^{IL-10} cells were further analyzed after the 2^{nd} round (TF2) of restimulation for expression of granzyme B (GzB). The data in FIG. 6A show that most of the CD4^{IL-10} cells expressed GzB. More than 95 % of all CD4^{IL-10} cells derived from 7 different donors expressed high levels of Granzyme B.

The CD4^{IL-10} cells from the 2^{nd} round (TF2) of restimulation were further analyzed for their cytotoxic effects against myeloid leukemia cells (ALL-CM) and an erythroid leukemia cell line (K562). CD4^{IL-10} cells (10⁵/well) were co-cultured with K562 and ALL-CM cells (10⁵/well) at 1:1 ratio for 3 days. Residual leukemic cell lines (CD45^{low}CD33⁺) were counted by FACS for each target cell.

The CD4^{IL-10} cells selectively killed the myeloid leukemia cells (ALL-CM) as shown in FIG. 6B. The % of killed ALL-CM cells varied between 62% and 100%, whereas the killing of the erythroid leukemia cell line K562 (which are highly sensitive for nonspecific cytotoxic activities) varied between 0 and 27% (4 different donors tested). Taken together, these data confirm that CD4^{IL-10} cells express Granzyme B and efficiently kill myeloid leukemia cells. As expected, some variations in the killing capacity of the CD4^{IL-10} cells from individual donors was observed.

### CD4^{IL-10} cells suppress the proliferative responses of both allogeneic CD4⁺ and CD8⁺ T cells

The CD4^{IL-10} cells were also analyzed for their effects on allogeneic CD4⁺ T cells or CD8⁺ T cells. Specifically, allogeneic PBMC cells were labeled with eFluor^{®} 670 (10⁵ cells/well) and stimulated with allogeneic mature dendritic (DC) cells (5x10⁴ cells/well) and soluble anti-CD3 mAbs in the absence or presence of CD4^{IL-10} cells (10⁵ cells/well) at a 1:1 Responder: Suppressor ratio. After 4 days of culture, the percentages of proliferating responder cells were determined by eFluor^{®} 670 dilution with flow cytometry after gating on CD4⁺ΔNGFR⁻ T cells or CD8⁺ΔNGFR⁻ T cells. FIGs. 7A and 7B show effects of CD4^{IL-10} cells from six different, unpooled, donors (Donor-C, Donor-E, and Donor-F in FIG. 7A and Donor-H, Donor-I, and Donor-L in FIG. 7B) on CD4⁺ T cells with percentages of proliferation and suppression. FIGs. 8A and 8B show effects of CD4^{IL-10} cells from six different, unpooled, donors (Donor -C, Donor-E, and Donor-F in FIG. 8A and Donor-H, Donor-I, and Donor-L in FIG. 8B) on CD8⁺ T cells.

The results demonstrated that CD4^{IL-10} cells from 6 different donors, unpooled and tested separately, downregulated the proliferative responses of both allogeneic CD4⁺ and CD8⁺ T cells. The suppressive effects on the CD4⁺ T-cells varied between 51% and 96%, while the suppressive effects on the CD8⁺ T-cells varied between 62% and 73 %.

### Production and characterization of poly-donor CD4^{IL-10} cells

CD4^{IL-10} cells were generated as described above and FIG. 3 using CD4⁺ cells from multiple donors. CD4^{IL-10} cells from each donor were stimulated by the second (TF2) and third (TF3) restimulation. After the third stimulation, CD4^{IL-10} cells from the three donors were pooled at a 1:1:1 ratio and stimulated with immobilized anti-CD3 and soluble anti-CD28 mAbs for 48hrs.

### Poly-donor CD4^{IL-10} cells have a cytokine production profile which is comparable to that of CD4 ⁻¹⁰ cells of individual donors and Tr1 cells.

Culture supernatants were collected and levels of IL-10, IL-4, IL-5, IFN-γ and IL-22 were determined by ELISA. The results provided in FIG. 9 show that the cytokine production of polydonor CD4^{IL-10} cells pooled from 3 different allogeneic donors (pooled 1:1:1) (red dot) was comparable to that of CD4^{IL-10} cells from individual donor (n=8) derived CD4^{IL-10} cells (gray bars). The poly-donor CD4^{IL-10} cells produced high levels of IL-10, IL-5, IFN-γ and IL-22 and low levels of IL-4 and undetectable levels of IL-2 (not shown). These data indicate that it is feasible to pool CD4^{IL-10} cells and that these poly-donor CD4^{IL-10} cells maintain the cytokine production signature of single donor derived CD4^{IL-10} cells and Tr1 cells. Importantly, the pooled allogeneic cell populations contained > 95% viable cells indicating that they did not kill each other.

### Poly-donor CD4^{IL-10} cells express high levels of Granzyme B and kill myeloid leukemia cell lines.

The poly-donor CD4^{IL-10} cells were further analyzed after 3^{rd} round (TF3) of restimulation for expression of granzyme B (GzB). The data in FIG. 10A show that most of the poly-donor CD4^{IL-10} cells express GzB. Over 95 % of the polydonor CD4^{IL-10} cells expressed Granzyme B, comparable to the GzB expression of single donor derived CD4^{IL-10} cells.

The CD4^{IL-10} cells from 3^{rd} round (TF3) of restimulation were further analyzed for their cytotoxic effects on myeloid leukemia cells (ALL-CM cell line) or K562. The poly-donor CD4^{IL-10} cells (10⁵/well) were co-cultured with K562 and ALL-CM cells (10⁵/well) at 1:1 ratio for 3 days. Residual leukemic cell lines (CD45^{low}CD33⁺) were counted by FACS for each target cell. The results provided in FIG. 10B show that some level of cytotoxicity against K562 cells, which are highly sensitive for nonspecific cytotoxicity. Nevertheless, a level of selectivity towards myeloid leukemia cells (ALL-CM) was obtained which is comparable to that of single donor derived CD4^{IL-10} cells.

### Poly-donor CD4^{IL-10} cells suppress the proliferative responses of both allogeneic CD4+ and CD8+ T cells.

The poly-donor CD4^{IL-10} cells were also analyzed for their effects on allogeneic CD4⁺ T cells or CD8⁺ T cells. Specifically, allogeneic PBMC cells were labeled with eFluor^{®} 670 (10⁵ cells/well) and stimulated with allogenic mature dendritic (DC) cells (5x10⁴ cells/well) and soluble anti-CD3 mAbs in the absence or presence of poly-donor CD4^{IL-10} cells (10⁵ cells/well) at a 1:1 Responder: Suppressor ratio. After 4 days of culture, the percentages of proliferating responder cells were determined by eFluor^{®} 670 dilution with flow cytometry after gating on CD4⁺ΔNGFR⁻ T cells and CD8⁺ΔNGFR⁻ T cells. FIG. 11A shows results from polydonor CD4^{IL-10} cells containing CD4^{IL-10} cells from Donor-C, Donor-E, and Donor-F. FIG. 11B shows results from poly-donor CD4^{IL-10} cells containing CD4^{IL-10} cells from Donor-H, Donor-I, and Donor-L, which had been frozen, stored and thawed prior to testing.

FIG. 11A shows that the poly-donor CD4^{IL-10} cells (from 3 different donors) suppress CD4⁺ and CD8⁺ T-cell responses by 96% and 74%, respectively. Comparable results were obtained with a second, different batch of poly-donor CD4^{IL-10} cells which was tested after the cells had been frozen, stored and thawed prior to testing (FIG. 11B). Suppression of CD4⁺ and CD8⁺ T cell proliferation was 68% and 75 %, respectively. These data indicate that polydonor CD4^{IL-10} cells can be frozen and stored without loss of function.

Collectively the data obtained with poly-donor CD4^{IL10} cells indicate that these cell preparations can be pooled without any problems. They contain > 95 % viable cells and maintain all the relevant functions (cytokine production, cytotoxic capacity, and suppression of allogeneic T cell responses) of single donor CD4^{IL-10} cells. The use of larger pools of poly-donor CD4^{TL-10} cells should reduce the natural variations observed between CD4^{IL-10} cell lots originating from different individual donors, and should provide a large quantity of off-the-shelf CD4^{IL-10} cells for human therapy.

A poly-donor CD4^{IL-10} cell product will have significant advantages in terms of a more homogeneous product which will allow the determination of well defined, less lot-to-lot variation, potency, and release criteria. In addition, it will enable the development of a continuous large-scale cell production process.

### Other methods for production of poly-donor CD4^{IL-10} cells

Before the lentiviral transduction, buffy coats from minimally 3-5 different donors are pooled. CD4⁺ cells are isolated from buffy coats by positive selection using anti-CD4 antibody. Purity of the pooled CD4⁺ cells is checked by FACS. Alternatively, frozen human CD4⁺ cells are obtained from minimally 3-5 normal healthy donors. The frozen human CD4⁺ cells are thawed before use. CD4⁺ cells from buffy coats or frozen stocks are activated for 24-48 hrs by a combination of CD3 and CD28 antibodies or CD3- and CD28 antibody coated beads in the presence of IL-2. In some cases, CD4⁺ cells from buffy coats or frozen stocks are activated with soluble anti-CD3, soluble anti-CD28 mAbs, and rhIL-2 (50 U/mL) for 48 hours and transduced with a bidirectional lentiviral vector encoding for human IL-10 as described above for production of CD4^{IL-10} cells.

In some cases, the HLA haplotype of the T cell donors (or CD4⁺ cells isolated from the donors) are first determined and CD4⁺ cells having desired HLA haplotypes are selectively pooled and used.

Poly-donor CD4^{IL-10} cells are generated by transducing the activated CD4⁺ cells described above with the lentiviral vector containing human IL-10 and ΔNGFR coding sequences described above.

On Day 7-11, which is 5-9 days after the transduction, the cells are harvested and successfully transduced T cells purified utilizing an anti-NGFR antibody. This process generally results in 95% pure populations of poly-donor CD4^{IL-10} cells.

The purified poly-donor CD4^{IL-10} cells are counted and re-stimulated by a mixture of CD3- and CD28 antibodies, CD3- and CD28 antibody coated beads, optionally in the presence of feeder cells for another 8-10 days in the presence of IL-2. In some cases, the purified poly-donor CD4^{IL-10} cells are re-stimulated in the presence of feeder cells.

After a total culture period of 14-18 days, CD4^{IL-10} cells are harvested, counted and tested for their capacity to produce IL-10 spontaneously or following activation with CD3 and CD28 antibodies or CD3 and CD28 antibody coated beads. Additionally, the levels of GrzB and perforin are measured. Their capacity to suppress human T cell (PBMC) and purified CD4⁺ and CD8⁺ T cell proliferation are also tested.

In addition, the production of IL-22 is measured both constitutively and following activation of 200,000 CD4^{IL-10} cells in a volume of 200 microliter using a combination of CD3 and CD28 antibodies as described previously for the production of other cytokines such as IFNγ, IL-10, IL-4 and IL-5. IL-22 production levels are measured in IL-22 specific ELISA as described for the other cytokines in WO2016/146542. The pooled CD4^{IL-10} cells are frozen before storage.

### 6.8.2. Example 2:Treatment or prevention of GvHD using poly-donor CD4^{IL-10} cells

### Effects of poly-donor CD4^{IL-10} cells in vivo.

A population of poly-donor CD4^{IL-10} cells were tested in a humanized xeno GvHD disease model, an NSG mouse model, for their effect on GvHD induced by human PBMC as illustrated in FIG. 12. NSG mice were sub-lethally irradiated and intravenously injected with human PBMC (5x10⁶ cells/mouse), with poly-donor (three donors) CD4^{IL-10} cells (5x10⁶ cells/mouse), or with human PBMC (5x10⁶ cells/mouse) in combination with poly-donor CD4^{IL-10} cells (three donors) (5x10⁶ cells/mouse). GvHD was evaluated as previously described (Bondanza et al. Blood 2006) based on weight loss (>20% weight loss), skin lesions, fur condition, activity, and hunch.

FIG. 13 shows % of NSG mice demonstrating GvHD on each day after injection. Administration of 5x 10⁶ human PBMC to irradiated NSG mice resulted unexpectedly in an unusually fulminant GvHD. All mice died at day 10 which reflects very lethal GvHD. Coadminstration of 5x10⁶ poly-donor CD4^{IL-10} cells delayed this fulminant GvHD, but the mice were sacrificed at day 14 because they reached the prespecified humane 20 % body weight loss criterion for sacrifice (Fig 13). Nevertheless, these results indicate that poly-donor CD4^{IL-10} can delay very severe GvHD. Importantly, poly-donor CD4^{IL-10} cells administered alone at the same dose as the PBMC (5x10⁶ cells ) failed to induce any sign of GvHD.

The presence of human CD4^{IL-10} cells were also tested in the spleen (FIG. 14, left panels) and bone marrow (FIG. 14, right panels) of the NSG mice injected with human PBMC (5x10⁶ cells/mouse), poly-donor (three donors) CD4^{IL-10} cells (5x10⁶ cells/mouse), or human PBMC (5x10⁶ cells/mouse) in combination with poly-donor CD4^{IL-10} cells (three donors) (5x10⁶ cells/mouse) at 14 days post injection. The results provided in FIG. 14 show that poly-donor CD4^{IL-10} cells migrated to spleen and bone marrow. Low percentages of these cells were found to be present 14 days after infusion of the cells. These results indicate that poly-donor CD4^{IL-10} cells delayed fulminant GvHD induced by human PBMC and that they do not themselves induce any xeno GvHD.

### Poly-donor CD4^{IL-10} cells inhibit severe xeno GvHD by purified CD4⁺ cells.

Poly-donor CD4^{IL-10} cells were tested in a humanized xeno GvHD model in which GvHD disease was induced by administration of 2.5 X 10⁶ purified human CD4⁺ T cells as illustrated in FIG. 15. NSG mice were sub-lethally irradiated at day 0 and on day 3 were intravenously injected with human CD4⁺ T cells (2.5x10⁶ cells/mouse) alone or in combination with poly-donor CD4^{IL-10} cells (three different donors) (2.5x10⁶ cells/mouse) or with CD4^{IL-10} cells from a single donor from the pool (2.5x10⁶ cells/mouse). GvHD was evaluated as previously described (Bondanza et al. Blood 2006) based on weight loss (>20% weight loss), skin lesions, fur condition, activity, and hunch.

FIG. 16 shows % of NSG mice demonstrating GvHD on each day after injection. The results show that poly-donor CD4^{IL-10} cells can inhibit GvHD mediated by human allogeneic CD4⁺ T cells. In this particular experiment, xeno GvHD was very severe, because all mice in the control group which received CD4⁺ T cells were dead at day 20. In contrast, co-administration of 2.5 x 10 ⁶ poly-donor CD4^{IL-10} inhibited GvHD by 75 %. Single-donor CD4^{IL-10} cells were also protective but the effects were less potent.

### Other experiments

Therapeutic effects of the poly-donor CD4^{IL-10} cells are tested in four different groups of mice: (i) mice receiving human PBMC from a donor unrelated to the CD4^{IL-10} cells (GvHD positive control); (ii) mice receiving the poly-donor CD4^{IL-10} cells (negative control); (iii) mice receiving a combination of PBMC and the poly-donor CD4^{IL-10} cells at 1:1 ratio; and (iv) mice receiving a combination of PBMC and the poly-donor CD4^{IL-10} cells at 2:1 ratio or at different ratios. Among animals receiving combination of PBMC and the poly-donor CD4^{IL-10} cells, some animals receive PBMC and the poly-donor CD4^{IL-10} cells concurrently, some animals receive poly-donor CD4^{IL-10} cells several days (e.g., 5 days) after receiving PBMC, and some animals receive poly-donor CD4^{IL-10} cells several days (e.g., 5 days) before receiving PBMC.

The mice are monitored for development of GvHD by measuring weight at weeks 1, 2, 3, 4, and if necessary week 5, after administration of PBMC and/or the poly-donor CD4^{IL-10} cells. In addition to weight loss, the mice will be inspected for skin lesions, fur condition and activity. The mice in the treatment groups are monitored for additional periods to determine effects of the poly-donor CD4^{IL-10} cells on long term survival.

The amount and localization of the poly-donor CD4^{IL-10} cells are also monitored in peripheral blood and tissues after administration. Specifically, presence of poly-donor CD4^{IL-10} cells are monitored in peripheral blood and at sites of inflammation: lymph nodes, spleen, gut, and bone marrow. The mice in the treatment group(s) are monitored for an additional 3 weeks to determine long-term survival.

The results demonstrate that poly-donor CD4^{IL-10} cells are effective in reducing and preventing xeno-GvHD.

### 6.8.3. Example 3: Inhibition of GvHD and treatment of cancer

A population of poly-donor CD4^{IL-10} cells are tested in an NSG mouse model transplanted with human PBMC and AML tumor cells for their effect on xeno-GvHD induced by human PBMC and anti-tumor effects. AML cells (ALL-CM) are administered i.v. as described previously in WO 2016/146542. PBMC or poly-donor CD4^{IL-10} cells or combinations thereof are administered 3 days later.

Poly-donor CD4^{IL-10} cells are obtained as described in Example 1. Therapeutic effects of the poly-donor CD4^{IL-10} cells are tested in four different groups of mice, each having received irradiation and 5x10⁶ ALL-CM cells (AML mice) at day 0: (i) AML mice without additional treatment; (ii) AML mice receiving 5x10⁶ human PBMC from a donor unrelated to the poly-donor CD4^{IL-10} cells - the PBMCs cause severe xeno-GvHD; (iii) AML mice receiving 2.5x10⁶ poly-donor CD4^{IL-10} cells; and (iv) AML mice receiving combinations of PBMC and the poly-donor CD4^{IL-10} cells at 1:1 or 2:1 ratio or at different ratios. One additional group of mice do not receive ALL-CML cells but receive 5x10⁶ human PBMC at day 3 after irradiation.

Effects of the poly-donor CD4^{IL-10} cells on xeno-GvHD induced by human PBMC are tested based on weight loss, skin lesions, fur condition, activity, death rate and long-term survival. Anti-tumor or graft versus leukemia (GvL) effects of the poly-donor CD4^{IL-10} cells are tested based on reduction of tumor cells in the circulation and long-term tumor free survival.

Some mice are monitored for up to 7 weeks in order to monitor long-term survival and complete tumor remissions.

Results demonstrate that poly-donor CD4^{IL-10} cells are effective in both inhibition of xeno-GvHD and treatment of cancer.

### 6.8.4. Example 4: Treatment of cancer using poly-donor CD4^{IL-10} cells

A population of poly-donor CD4^{IL-10} cells are tested in an ALL-CM leukemia model of T cell therapy in NSG mice.

NSG mice were sub-lethally irradiated and intravenously injected with myeloid leukemia cells (ALL-CM) (5x10⁶) at day 0. In the first group of animals, PBMC (5x10⁶) or single donor (from donor BC-I and donor BC-H) CD4^{IL-10} cells (2.5x10⁶) were injected at day 3. In the second group of animals, PBMC (5x10⁶) or poly-donor CD4^{IL-10} cells (2.5x10⁶) were injected at day 3. Graft-versus-leukemia (GvL) effect was tested in the animals based on reduction of circulating leukemia cells and long-term leukemia free survival. Leukemia was measured as previously described (Locafaro G. et al Molecular Therapy 2017).

As provided in FIG. 17A and FIG. 17B, all of the mice injected with ALL-CM myeloid leukemia cells had extensive leukemia progression at day 17. Administration of 5x10⁶ PBMC resulted in a strong inhibition of leukemia progression. Interestingly, a comparable level of inhibition of leukemia progression was obtained by lower number (2.5x10⁶) of single-donor CD4^{IL10} (Fig17A) or poly-donor CD4^{IL10} (Fig17B). These data indicate that single donor and poly-donor CD4^{IL10} have strong direct anti leukemia effects.

Graft-versus-leukemia (GvL) effects of single-donor CD4^{IL10} and poly-donor CD4^{IL10} were further tested in combination with PBMC in mice injected with ALL-CM myeloid leukemia cells. Administration of 5x10⁶ PBMC resulted in a strong inhibition of leukemia progression and administration of 5x10⁶ PBMC combined with single donors CD4^{IL10} (2.5x10⁶) had synergistic effect (Fig18A). Interestingly, administration of 5x10⁶ PBMC combined with 2.5x10⁶ poly-donor CD4^{IL10} had a comparable synergistic GvL effect (Fig18B). These data indicate that poly-donor CD4^{IL10} act in synergy with PBMC to mediate strong GvL effects.

### 6.8.5. Example 5: Treatment of chronic inflammatory and autoimmune diseases using poly-donor CD4^{IL-10} cells

Activation of the NLPR3 inflammasome has been implicated in many chronic inflammatory and autoimmune diseases. The NLPR3 inflammasome can be activated by "danger signals" which lead to caspase1-mediated production of the pro-inflammatory cytokines IL-1β and IL-18 by monocytes/macrophages. A series of in vitro experiments are performed to investigate the effects of poly-donor CD4^{IL-10} cells on the NLPR3 inflammasome and IL-1 β/IL-18 production by human monocytes.

First, human PBMC are isolated from peripheral blood by standard density centrifugation on Ficoll/Paque (Sigma-Aldrich). Monocytes are isolated from the human PBMC by negative selection using monocyte isolation kit II (Miltenyi) according to the manufacturer's instructions. Negative selection is preferred because positive selection or adherence can lead to undesired activation of the cells. Isolated monocytes are plated at 5x10⁴ cells/200 µl in the presence of 2x10⁵ or 1x10⁵ poly-donor CD4^{IL-10} cells /200 µl per well in 96-well microtiter plates in culture medium containing 3% toxin free human AB serum.

**Table 1 summarizes treatment conditions applied to 17 sets of monocytes, each set including 6 wells of cells. It is known that LPS alone can activate human monocytes without a second signal provided by ATP.**

| **Table 1** | | | | | |
|---|---|---|---|---|---|
| **Group #** | **Monocytes** | **CD4 cells/supernatant** | **YVADfmk*** | **LPS**** | **Other** |
| **Medium control** | | | | | |
| Incubation time: 1.5 hour; followed by activation by LPS for 4 hours | | | | | |
| 1 | Monocytes | No | No | No | |
| 2 | Monocytes | No | No | LPS | |
| 3 | Monocytes | No | YVADfmk | LPS | |

| **Co cultivation of monocytes and poly-donor CD4^{IL-10} cells** | | | | | |
|---|---|---|---|---|---|
| 4 | Monocytes | CD4^{IL-10} cells | No | No | |
| 5 | Monocytes | CD4^{IL-10} cells | No | LPS | |
| 6 | Monocytes | CD4^{IL-10} cells | YVADfmk | LPS | |

| **Co cultivation of monocytes and control CD4 GFP+ cells** | | | | | |
|---|---|---|---|---|---|
| 7 | Monocytes | CD4 GFP cells | No | No | |
| 8 | Monocytes | CD4 GFP cells | No | LPS | |
| 9 | Monocytes | CD4 GFP cells | YVADfmk | LPS | |

| **Cultivation of monocytes in the presence of supernatants*** of poly-donor CD4^{IL-10} cell or CD4 GFP+ cell culture** | | | | | |
|---|---|---|---|---|---|
| 10 | Monocytes | 50% supernatant of CD4^{IL-10} cells | No | LPS | |
| 11 | Monocytes | 25% supernatant of CD4^{IL-10} cells | No | LPS | |
| 12 | Monocytes | 12.5% supernatant of CD4^{IL-10} cells | No | LPS | |
| 13 | Monocytes | 50% supernatant of CD4^{IL-10} cells | No | LPS | Anti-IL-10 antibody |
| 14 | Monocytes | 50% supernatant of CD4 GFP cells | No | LPS | |
| 15 | Monocytes | 25% supernatant of CD4 GFP cells | No | LPS | |
| 16 | Monocytes | 12.5% supernatant of CD4 GFP cells | No | LPS | |
| 17 | Monocytes | 50% supernatant of CD4 GFP cells | No | LPS | Anti-IL-10 antibody |

| | | | | | |
|---|---|---|---|---|---|
| * YVADfmk is an inhibitor specific to caspase 1. 20 mM Z-YV ADfmk (Biovision, Enzo Life Sciences, or Axxora Life Sciences) dissolved in DMSO is used. ** LPS (Signa-Aldrich) 100ng/ml *** Supernatants of CD4^{IL-10} and CD4 GFP or NGFR cultures are obtained by incubating CD4^{IL-} ¹⁰ or CD4 GFP cells at 1X10⁶/ml for 3days and collecting the supernatants. IL-10 production levels are measured by IL-10 specific ELISA. | | | | | |

After treatments outlined in Table 1, supernatants are collected from 6 wells for each group and IL-1 β/IL-18 production is measured by ELISA specific for mature IL-1β or IL-18 (Biolegend). Cells collected from 6 wells for Group #3, 10, 13, 14, and 17 are analyzed by Western Blot to determine levels of activated caspase 1.

Data from the experiments show that poly-donor CD4^{IL-10} cells down-regulate IL-1β and IL-18 production by activated monocytes. They further show that poly-donor CD4^{IL-10} cells down-regulate mature caspase-1 production in activated monocytes. Additionally, poly-donor CD4^{IL-10} and IL-10 produced by the poly-donor CD4^{IL-10} down-regulate inflammasome.

Similar experiments are performed with human macrophages or dendritic cells instead of monocytes. Results from the experiments demonstrate that poly-donor CD4^{IL-10} cells further down-regulate IL-1β, IL-18, and mature caspase-1 production from activated macrophages and dendritic cells.

These suggest that poly-donor CD4^{IL-10} cells can be used to treat diseases or disorders involving hyperactivation of NLPR3 inflammasome. In particular, poly-donor CD4^{IL-10} cells can be used to treat chronic inflammatory and autoimmune diseases. The NLPR3 inflammasome can be activated by exogenous or endogenous "danger signals", such as Pathogen Associated Molecular Patterns (PAMPs), silica, asbestos, Danger Associated Molecular Patterns (DAMPs) like products from damaged mitochondria, necrotic and stressed cells, and uremic acid crystals.

### 6.8.6. Experimental methods and materials

**Cell preparation and cell lines.** Peripheral blood mononuclear cells (PBMC) were prepared by centrifugation over Ficoll-Hypaque gradients. CD4⁺ T cells were purified with a CD4 T cell isolation kit (Miltenyi Biotec, Bergisch Gladbach, Germany) with a resulting purity of >95%. Mature dendritic cells (DC) were generated from peripheral blood CD14⁺ monocytes positively selected using CD14⁺ MicroBeads (Miltenyi Biotech, Germany) according to the manufacturer's instructions and cultured in RPMI 1640 (Lonza, Italy) supplemented with 10% fetal bovine serum (FBS; Lonza, Italy), 100 U/mL penicillin/streptomycin (Lonza, Italy), 2 mM L-glutamine (Lonza, Italy), at 37°C in the presence of 10 ng/mL recombinant human (rh) IL-4 (R&D Systems, Minneapolis MN, USA) and 100 ng/mL rhGM-CSF (Genzyme, Seattle, WA, USA) for 5 days and matured with 1 mg/mL of lipopolysaccharide (LPS, Sigma, CA, USA) for an additional two days.

**Plasmid construction.** The coding sequence of human IL-10 was excised from pH15C (ATCC n° 68192), and the 549bp fragment was cloned into the multiple cloning site of pBluKSM (Invitrogen) to obtain pBluKSM-hIL-10. A fragment of 555bp was obtained by excision of hIL-10 from pBluKSM-hIL-10 and ligation to 1074.1071.hPGK.GFP.WPRE.mhCMV.dNGFR.SV40PA (here named LV-ΔNGFR), to obtain LV-IL-10/ΔNGFR. The presence of the bidirectional promoter (human PGK promoter plus minimal core element of the CMV promoter in opposite direction) allows co-expression of the two transgenes (Locafaro et al. Mol Ther. 2017;25(10):2254-2269). The sequence of LV-IL-10/ΔNGFR was verified by pyrosequencing (Primm).

**Vector production and titration.** VSV-G-pseudotyped third generation bidirectional lentiviral vectors were produced by Ca₃PO₄ transient four-plasmid co-transfection into 293T cells and concentrated by ultracentrifugation as described (Locafaro et al. Mol Ther. 2017;25(10):2254-2269). Titer was estimated by limiting dilution, vector particles were measured by HIV-1 Gag p24 antigen immune capture (NEN Life Science Products; Waltham, MA), and vector infectivity was calculated as the ratio between titer and particle. Titers ranged from 5x10⁸ to 6x10⁹ transducing units/mL, and infectivity from 5x10⁴ to 10⁵ transducing units/ng of p24.

**Generation of CD4^{IL-10} cell lines.** Polyclonal CD4-transduced cells were obtained as previously described (Andolfi et al. Mol Ther. 2012;20(9):1778-1790). Briefly, CD4 purified T cells were activated for 48 hours with soluble anti-CD3 monoclonal antibody (mAb, 30 ng/mL, OKT3, Janssen-Cilag, Raritan, NJ, USA), anti-CD28 mAb (1 µg/mL, BD) and rhIL-2 (50 U/mL, PROLEUKIN, Novartis, Italy). T cells were transduced with LV-IL-10/ΔNGFR (CD4^{IL-10}) with multiplicity of infection (MOI) of 20. At day 11, CD4⁺ΔNGFR⁺ cells were beads-sorted using CD271⁺ Microbeads (Miltenyi Biotec, Bergisch Gladbach, Germany) and expanded in X-VIVO15 medium with 5% human serum (BioWhittaker-Lonza, Washington), 100 U/mL penicillin-streptomycin (BioWhittaker), and 50 U/mL rhIL-2 (PROLEUKIN, Novartis, Italy). At day 7 and 10, medium was replaced by fresh medium supplemented with 50U/mL of rhIL-2. At day 14, cells were collected, washed, and restimulated with allogeneic feeder mixture as previously described (Andolfi et al. Mol Ther. 2012;20(9):1778-1790). After 14 days, cells were collected and frozen. Thawed CD4^{IL-10} cells were restimulated and after the 2^{nd} and 3^{rd} re-stimulation and expansion were functionally characterized *in vitro* and used for *in vivo* experiments.

**Vector Copy Number Analysis.** Cells were cultured for at 11 days after transduction in order to get rid of non-integrated vector forms. Genomic DNA was isolated with QIAamp DNA Blood Mini Kit (QIAGEN, 51106), according to the manufacturer's instructions. Vector integrations were quantified by QX200 Droplet Digital PCR System (Bio-Rad), according to the manufacturer's instructions.

**Cytokine determination.** To measure cytokine production, after 2^{nd} and 3^{rd} re-stimulation single donor and poly-donor CD4^{IL-10} cells were left unstimulated or stimulated with immobilized anti-CD3 (10 µg/mL) and soluble anti-CD28 (1µg/mL) mAbs in a final volume of 200 µl of medium (96 well round-bottom plates, 2x10⁵/well). Supernatants were harvested after 48 hours of culture and levels of IL-10, IL-4, IL-5, IFN-γ and IL-22 were determined by ELISA according to the manufacturer's instructions (BD Biosciences).

**Flow cytometry analysis.** For the expression of Granzyme B (clone MHGB04, Invitrogen, USA) after surface staining with CD4, CD4^{IL-10} cells were fixed, permeabilized, and stained using the BD Cytofix/Cytoperm^{™} Kit according to the manufacturer's instructions (Cat. No. 554714, Biolegend, USA). Stained cells were washed two times with PBS supplemented with 1% FBS and analysed with a BD LSRFortessa analysed utilizing FlowJo 10 software.

**Killing assays.** After 2^{nd} and 3^{rd} re-stimulation, cytotoxicity of single-donor and poly-donor CD4^{IL-10} cells was analysed in co-culture experiments. Briefly, non-myeloid leukemia and a myeloid leukemia cell lines, K562 and ALL-CM respectively, were used as target cells and plated with CD4^{IL-10} cells at 1:1 ratio (10⁵ target cells and 10⁵ CD4^{IL-10} cells) for 3 days. At the end of co-culture, cells were harvested and K562 and ALL-CM cells were analysed and counted by FACS.

**Suppression assays.** To measure the suppressive capacity of single donor and poly-donor CD4^{IL-10} cells, allogeneic PBMC were labeled with Cell Proliferation Dye eFluor^{®} 670 (Invitrogen, CA, USA), according to manufacturer's instructions prior to stimulation with allogeneic mature DC (5x10⁴ cells/well) and soluble anti-CD3 (50 ng/mL) mAb. PBMC and suppressor cells were added at a 1:1 ratio (10⁵ PBMC and 10⁵ CD4^{IL-10} cells). After 3 days of culture, proliferation of responder cells was determined by analyzing the eFluor670 dilution of CD4⁺ΔNGFR⁻ or CD8⁺ΔNGFR⁻ T cells by FACS.

**Graft-versus Host Disease models:** In all experiments 6/8 week-old female NSG mice were used. On day 0 mice received total body irradiation with a single dose of 175-200 cGy from a linear accelerator according to the weight of the mice, and were intravenously with PBMC cells (5x10⁶), or CD4^{IL-10} cells (single-donors or poly-donor - pool of three donors - 5x10⁶ or 2.5x10⁶), or with PBMC (5x10⁶) in combination with CD4^{IL-10} cells (5x10⁶ or 2.5x10⁶). Survival, weight loss, activity, fur, skin, and hunch were monitored at least 3 times per week as previously described (Bondanza et al. Blood. 2006;107(5):1828-1836). Mice were euthanized for ethical reasons when their loss of bodyweight was 20%.

Alternatively, on day 0 mice received total body irradiation as above. On day 3 mice were injected with CD4⁺ T cells (2.5x10⁶), single and poly-donor (pool of three donors) CD4^{IL-10} cells (2.5x10⁶), or CD4⁺ T cells (2.5x10⁶) in combination with single and poly-donor (pool of three donors) CD4^{IL-10} cells (2.5x10⁶). GvHD induction was monitored as indicated above.

### 7. INCORPORATION BY REFERENCE

All publications, patents, patent applications and other documents cited in this application are hereby incorporated by reference in their entireties for all purposes to the same extent as if each individual publication, patent, patent application or other document were individually indicated to be incorporated by reference for all purposes.

### 8. EQUIVALENTS

While various specific embodiments have been illustrated and described, the above specification is not restrictive. It will be appreciated that various changes can be made without departing from the spirit and scope of the invention(s). Many variations will become apparent to those skilled in the art upon review of this specification.

### 9. SEQUENCES

SEQ ID NO: 1 (Human IL-10 amino acid sequence) 
M H S S A L L C C L V L L T G V R A S P G Q G T Q S E N S C T H F P
G N L P N M L R D L R D A F S R V K T F F Q M K D Q L D N L L L K
E S L L E D F K G Y L G C Q A L S E M I Q F Y L E E V M P Q A E N Q
D P D I K A H V N S L G E N L K T L R L R L R R C H R F L P C E N
K S K A V E Q V K N A F N K L Q E K G I Y K A M S E F D I F I N Y I
E A Y M T M K I R N
SEQ ID NO: 2 (Human IL-10 exemplary nt sequence) 
ggggtgagggccagcccaggccagggcacccagtctgagaacagctgcacccacttccca
ggcaacctgcctaacatgcttcgagatctccgagatgccttcagcagagtgaagactttc
tttcaaatgaaggatcagctggacaacttgttgttaaaggagtccttgctggaggacttt
aagggttacctgggttgccaagccttgtctgagatgatccagttttacctggaggaggtg
atgccccaagctgagaaccaagacccagacatcaaggcgcatgtgaactccctgggggag
aacctgaagaccctcaggctgaggctacggcgctgtcatcgatttcttccctgtgaaaac
aagagcaaggccgtggagcaggtgaagaatgcctttaataagctccaagagaaaggcatc
tacaaagccatgagtgagtttgacatcttcatcaactacatagaagcctacatgacaatgaagatacgaaactgagtc
SEQ ID NO: 3 (ΔNGFR amino acid sequence) 
LEDPPVPPLEGYVGHKAHNHSSQDGAIDRDEVV
GGASGHHGLGAAHHCGHHTCHRAGYEV LFWRC
LRLLGAGGCAVRALW GCGPCNPTARDLLALGV
GPA CALAELALGVLAHGAGQARVHVVGLVGIRA
VGALLAHGVL LVLAGEHEARARLAHAARLA AP
SRLILVVAVGAAAHGVVG LHARRRHALEPHALG
ARLAR LGRAHHVGERHAVQAGLTHG LVGSTRL
GHTLAQVAGFAAL TAVCVQACGACLLGTSKGHP
QKQQQQQARPVHGAAGGTCP H
SEQ ID NO: 4 (ΔNGFR exemplary nt sequence) 
ctagaggatccccctgttccacctcttgaaggctatgtaggccacaaggcccacaaccac
agcagccaggatggagcaatagacagggatgaggttgtcggtggtgcctcgggtcaccac
gggctgggagctgcccatcactgtggtcaccacacctgccaccgtgctggctatgaggtc
ttgttctggaggtgcctcaggctcctgggtgctgggggctgtgctgtccgagccctctgg
gggtgtggaccgtgtaatccaacggccagggatctcctcgcactcggcgtcggcccagcg
tgtgcactcgcggagctggcgctcggtgtcctcgcacacggtgcagggcaggcacgggtc
cacgtggttggcctcgtcggaatacgtgccgtcggggcactcctcgcacacggtgttctg
cttgtcctggcaggagaacacgaggcccgagcccgcctcgcacacgcggcacgcctcgca
gcgcccagtcgtctcatcctggtagtagccgtaggcgcagcggcacacggcgtcgtcggc
ctccacgcacggcgccgacatgctctggagccccacgcactcggtgcacggcttgcacgg
ctcggtcgcgctcaccacgtcggagaacgtcacgctgtccaggcagggctcacacacggt
ctggttggctccacaaggctgggccacaccctcgcccaggttgcaggctttgcagcactc
accgctgtgtgtgtacaggcctgtggggcatgcctccttggcacctccaagggacacccc
cagaagcagcaacagcagcaggcgcggcccgtccatggcgcggccggtggcacctgcccc cat
SEQ ID NO: 5 (nucleotide sequence of bd.ΔNGFR.PGK.IL-10)

aaatttcacaaataaagcatttttttcactgcattctagttgtggtttgtccaaactcatcaatgtatcttatcatgtctggctctagctatcccgcccc
taactccgcccagttccgcccattctccgccccatggctgactaattttttttatttatgcagaggccgaggccgcctcggcctctgagctattc
cagaagtagtgaggaggcttttttggaggcctaggcttttgcgtcgagacgtacccaattcgccctatagtgagtcgtattacgcgcgctcact
ggccgtcgttttacaacgtcgtgactgggaaaaccctggcgttacccaacttaatcgccttgcagcacatccccctttcgccagctggcgtaa
tagcgaagaggcccgcaccgatcgcccttcccaacagttgcgcagcctgaatggcgaatggcgcgacgcgccctgtagcggcgcattaa
gcgcggcgggtgtggtggttacgcgcagcgtgaccgctacacttgccagcgccctagcgcccgctcctttcgctttcttcccttcctttctcg
ccacgttcgccggctttccccgtcaagctctaaatcgggggctccctttagggttccgatttagtgctttacggcacctcgaccccaaaaaact
tgattagggtgatggttcacgtagtgggccatcgccctgatagacggtttttcgccctttgacgttggagtccacgttctttaatagtggactctt
gttccaaactggaacaacactcaaccctatctcggtctattcttttgatttataagggattttgccgatttcggcctattggttaaaaaatgagctg
atttaacaaaaatttaacgcgaattttaacaaaatattaacgtttacaatttcccaggtggcacttttcggggaaatgtgcgcggaacccctattt
gtttatttttctaaatacattcaaatatgtatccgctcatgagacaataaccctgataaatgcttcaataatattgaaaaaggaagagtatgagtatt
caacatttccgtgtcgcccttattcccttttttgcggcattttgccttcctgtttttgctcacccagaaacgctggtgaaagtaaaagatgctgaag
atcagttgggtgcacgagtgggttacatcgaactggatctcaacagcggtaagatccttgagagttttcgccccgaagaacgttttccaatga
tgagcacttttaaagttctgctatgtggcgcggtattatcccgtattgacgccgggcaagagcaactcggtcgccgcatacactattctcagaa
tgacttggttgagtactcaccagtcacagaaaagcatcttacggatggcatgacagtaagagaattatgcagtgctgccataaccatgagtga
taacactgcggccaacttacttctgacaacgatcggaggaccgaaggagctaaccgcttttttgcacaacatgggggatcatgtaactcgcc
ttgatcgttgggaaccggagctgaatgaagccataccaaacgacgagcgtgacaccacgatgcctgtagcaatggcaacaacgttgcgca
aactattaactggcgaactacttactctagcttcccggcaacaattaatagactggatggaggcggataaagttgcaggaccacttctgcgct
cggcccttccggctggctggtttattgctgataaatctggagccggtgagcgtgggtctcgcggtatcattgcagcactggggccagatggt
aagccctcccgtatcgtagttatctacacgacggggagtcaggcaactatggatgaacgaaatagacagatcgctgagataggtgcctcac
tgattaagcattggtaactgtcagaccaagtttactcatatatactttagattgatttaaaacttcatttttaatttaaaaggatctaggtgaagatcct
ttttgataatctcatgaccaaaatcccttaacgtgagttttcgttccactgagcgtcagaccccgtagaaaagatcaaaggatcttcttgagatcc
tttttttctgcgcgtaatctgctgcttgcaaacaaaaaaaccaccgctaccagcggtggtttgtttgccggatcaagagctaccaactctttttcc
gaaggtaactggcttcagcagagcgcagataccaaatactgtccttctagtgtagccgtagttaggccaccacttcaagaactctgtagcacc
gcctacatacctcgctctgctaatcctgttaccagtggctgctgccagtggcgataagtcgtgtcttaccgggttggactcaagacgatagtta

ccggataaggcgcagcggtcgggctgaacggggggttcgtgcacacagcccagcttggagcgaacgacctacaccgaactgagatacc
tacagcgtgagctatgagaaagcgccacgcttcccgaagggagaaaggcggacaggtatccggtaagcggcagggtcggaacaggag
agcgcacgagggagcttccagggggaaacgcctggtatctttatagtcctgtcgggtttcgccacctctgacttgagcgtcgatttttgtgatg
ctcgtcaggggggcggagcctatggaaaaacgccagcaacgcggcctttttacggttcctggccttttgctggccttttgctcacatgttcttt
cctgcgttatcccctgattctgtggataaccgtattaccgcctttgagtgagctgataccgctcgccgcagccgaacgaccgagcgcagcga
gtcagtgagcgaggaagcggaagagcgcccaatacgcaaaccgcctctccccgcgcgttggccgattcattaatgcagctggcacgaca
ggtttcccgactggaaagcgggcagtgagcgcaacgcaattaatgtgagttagctcactcattaggcaccccaggctttacactttatgcttc
cggctcgtatgttgtgtggaattgtgagcggataacaatttcacacaggaaacagctatgaccatgattacgccaagcgcgcaattaaccctc
actaaagggaacaaaagctggagctgcaagcttggccattgcatacgttgtatccatatcataatatgtacatttatattggctcatgtccaacat
taccgccatgttgacattgattattgactagttattaatagtaatcaattacggggtcattagttcatagcccatatatggagttccgcgttacataa
cttacggtaaatggcccgcctggctgaccgcccaacgacccccgcccattgacgtcaataatgacgtatgttcccatagtaacgccaatagg
gactttccattgacgtcaatgggtggagtatttacggtaaactgcccacttggcagtacatcaagtgtatcatatgccaagtacgccccctattg
acgtcaatgacggtaaatggcccgcctggcattatgcccagtacatgaccttatgggactttcctacttggcagtacatctacgtattagtcatc
gctattaccatggtgatgcggttttggcagtacatcaatgggcgtggatagcggtttgactcacggggatttccaagtctccaccccattgacg
tcaatgggagtttgttttggcaccaaaatcaacgggactttccaaaatgtcgtaacaactccgccccattgacgcaaatgggcggtaggcgtg
tacggtgggaggtctatataagcagagctcgtttagtgaaccggggtctctctggttagaccagatctgagcctgggagctctctggctaact
agggaacccactgcttaagcctcaataaagcttgccttgagtgcttcaagtagtgtgtgcccgtctgttgtgtgactctggtaactagagatcc
ctcagacccttttagtcagtgtggaaaatctctagcagtggcgcccgaacagggacctgaaagcgaaagggaaaccagagctctctcgac
gcaggactcggcttgctgaagcgcgcacggcaagaggcgaggggcggcgactggtgagtacgccaaaaattttgactagcggaggcta
gaaggagagagatgggtgcgagagcgtcagtattaagcgggggagaattagatcgcgatgggaaaaaattcggttaaggccaggggga
aagaaaaaatataaattaaaacatatagtatgggcaagcagggagctagaacgattcgcagttaatcctggcctgttagaaacatcagaagg
ctgtagacaaatactgggacagctacaaccatcccttcagacaggatcagaagaacttagatcattatataatacagtagcaaccctctattgt
gtgcatcaaaggatagagataaaagacaccaaggaagctttagacaagatagaggaagagcaaaacaaaagtaagaccaccgcacagc
aagcggccgctgatcttcagacctggaggaggagatatgagggacaattggagaagtgaattatataaatataaagtagtaaaaattgaacc
attaggagtagcacccaccaaggcaaagagaagagtggtgcagagagaaaaaagagcagtgggaataggagctttgttccttgggttctt
gggagcagcaggaagcactatgggcgcagcctcaatgacgctgacggtacaggccagacaattattgtctggtatagtgcagcagcaga
acaatttgctgagggctattgaggcgcaacagcatctgttgcaactcacagtctggggcatcaagcagctccaggcaagaatcctggctgtg
gaaagatacctaaaggatcaacagctcctggggatttggggttgctctggaaaactcatttgcaccactgctgtgccttggaatgctagttgg
agtaataaatctctggaacagattggaatcacacgacctggatggagtgggacagagaaattaacaattacacaagcttaatacactccttaa
ttgaagaatcgcaaaaccagcaagaaaagaatgaacaagaattattggaattagataaatgggcaagtttgtggaattggtttaacataacaa
attggctgtggtatataaaattattcataatgatagtaggaggcttggtaggtttaagaatagtttttgctgtactttctatagtgaatagagttagg
cagggatattcaccattatcgtttcagacccacctcccaaccccgaggggacccgacaggcccgaaggaatagaagaagaaggtggaga
gagagacagagacagatccattcgattagtgaacggatctcgacggtatcggttaacttttaaaagaaaaggggggattggggggtacagt
gcaggggaaagaatagtagacataatagcaacagacatacaaactaaagaattacaaaaacaaattacaaaaattcaaaattttatcgatcac
gagactagcctcgagagatctgatcataatcagccataccacatttgtagaggttttacttgctttaaaaaacctcccacacctccccctgaacc
tgaaacataaaatgaatgcaattgttgttgttaacttgtttattgcagcttataatggttacaaataaggcaatagcatcacaaatttcacaaataa
ggcatttttttcactgcattctagttttggtttgtccaaactcatcaatgtatcttatcatgtctggatctcaaatccctcggaagctgcgcctgtcat
cgaattcctgcagcccggtgcatgactaagctagctcagttagcctcccccatctcccctagaggatccccctgttccacctcttgaaggctat
gtaggccacaaggcccacaaccacagcagccaggatggagcaatagacagggatgaggttgtcggtggtgcctcgggtcaccacgggc
tgggagctgcccatcactgtggtcaccacacctgccaccgtgctggctatgaggtcttgttctggaggtgcctcaggctcctgggtgctggg
ggctgtgctgtccgagccctctgggggtgtggaccgtgtaatccaacggccagggatctcctcgcactcggcgtcggcccagcgtgtgca
ctcgcggagctggcgctcggtgtcctcgcacacggtgcagggcaggcacgggtccacgtggttggcctcgtcggaatacgtgccgtcgg
ggcactcctcgcacacggtgttctgcttgtcctggcaggagaacacgaggcccgagcccgcctcgcacacgcggcacgcctcgcagcg
cccagtcgtctcatcctggtagtagccgtaggcgcagcggcacacggcgtcgtcggcctccacgcacggcgccgacatgctctggagcc
ccacgcactcggtgcacggcttgcacggctcggtcgcgctcaccacgtcggagaacgtcacgctgtccaggcagggctcacacacggtc
tggttggctccacaaggctgggccacaccctcgcccaggttgcaggctttgcagcactcaccgctgtgtgtgtacaggcctgtggggcatg
cctccttggcacctccaagggacacccccagaagcagcaacagcagcaggcgcggcccgtccatggcgcggccggtggcacctgccc
ccatcgcccgcctcccgcggcagcgctcgacttccagctcggtccgctttgcggactgatggggctgcgctgcgctgcgctccagcgccc
cccctgcccgccggagctggccgcggcccgaattccgcggaggctggatcggtcccggtgtcttctatggaggtcaaaacagcgtggat
ggcgtctccaggcgatctgacggttcactaaacgagctctgcttatataggcctcccaccgtacacgcctaccctcgagaagcttgatatcga

attcccacggggttggggttgcgccttttccaaggcagccctgggtttgcgcagggacgcggctgctctgggcgtggttccgggaaacgc
agcggcgccgaccctgggtctcgcacattcttcacgtccgttcgcagcgtcacccggatcttcgccgctacccttgtgggccccccggcga
cgcttcctgctccgcccctaagtcgggaaggttccttgcggttcgcggcgtgccggacgtgacaaacggaagccgcacgtctcactagtac
cctcgcagacggacagcgccagggagcaatggcagcgcgccgaccgcgatgggctgtggccaatagcggctgctcagcggggcgcg
ccgagagcagcggccgggaaggggcggtgcgggaggcggggtgtggggcggtagtgtgggccctgttcctgcccgcgcggtgttcc
gcattctgcaagcctccggagcgcacgtcggcagtcggctccctcgttgaccgaatcaccgacctctctccccagggggatccccggtctg
caggaattcatgcacagctcagcactgctctgttgcctggtcctcctgactggggtgagggccagcccaggccagggcacccagtctgag
aacagctgcacccacttcccaggcaacctgcctaacatgcttcgagatctccgagatgccttcagcagagtgaagactttctttcaaatgaag
gatcagctggacaacttgttgttaaaggagtccttgctggaggactttaagggttacctgggttgccaagccttgtctgagatgatccagtttta
cctggaggaggtgatgccccaagctgagaaccaagacccagacatcaaggcgcatgtgaactccctgggggagaacctgaagaccctc
aggctgaggctacggcgctgtcatcgatttcttccctgtgaaaacaagagcaaggccgtggagcaggtgaagaatgcctttaataagctcca
agagaaaggcatctacaaagccatgagtgagtttgacatcttcatcaactacatagaagcctacatgacaatgaagatacgaaactgagtcg
agaatcaacctctggattacaaaatttgtgaaagattgactggtattcttaactatgttgctccttttacgctatgtggatacgctgctttaatgcctt
tgtatcatgctattgcttcccgtatggctttcattttctcctccttgtataaatcctggttgctgtctctttatgaggagttgtggcccgttgtcaggca
acgtggcgtggtgtgcactgtgtttgctgacgcaacccccactggttggggcattgccaccacctgtcagctcctttccgggactttcgctttc
cccctccctattgccacggcggaactcatcgccgcctgccttgcccgctgctggacaggggctcggctgttgggcactgacaattccgtgg
tgttgtcggggaagctgacgtcctttccatggctgctcgcctgtgttgccacctggattctgcgcgggacgtccttctgctacgtcccttcggc
cctcaatccagcggaccttccttcccgcggcctgctgccggctctagagcctcttccgcgtcttcgccttcccgggtcgagctcggtaccttta
agaccaatgacttacaaggcagctgtagatcttagccactttttaaaagaaaaggggggactggaagggctaattcactcccaacgaagac
aagatctgctttttgcttgtactgggtctctctggttagaccagatctgagcctgggagctctctggctaactagggaacccactgcttaagcct
caataaagcttgccttgagtgcttcaagtagtgtgtgcccgtctgttgtgtgactctggtaactagagatccctcagacccttttagtcagtgtgg
aaaatctctagcagtagtagttcatgtcatcttattattcagtatttataacttgcaaagaaatgaatatcagagagtgagaggaacttgtttattgc
agcttataatggttacaaataaagcaatagcatcac

### CLAUSES:

1. A population of CD4⁺ T cells that have been genetically modified to comprise an exogenous polynucleotide encoding IL-10, wherein the CD4⁺ T cells were obtained from at least three different T cell donors (poly-donor CD4^{IL-10} cells).
2. The population of CD4⁺ T cells of Clause 1, wherein the CD4⁺ T cells were obtained from three, four, five, six, seven, eight, nine, or ten different T cell donors.
3. The population of CD4⁺ T cells of Clause 1 or Clause 2, wherein the CD4⁺ T cells in the population collectively have six, seven, eight, nine, ten, eleven, twelve, or more different HLA haplotypes.
4. The population of CD4⁺ T cells of any one of the above Clauses, wherein all the CD4⁺ T cells in the population have at least 5/10, 6/10, 7/10, 8/10, or 9/10 match at the HLA-A, HLA-B, HLA-C, HLA-DRB1, and HLA-DQB1 loci to each other.
5. The population of CD4⁺ T cells of any one of the above Clauses, wherein all the CD4⁺ T cells in the population have at least 4/8, 5/8, 6/8, 7/8, or 8/8 match at the HLA-A, HLA-B, HLA-C, and HLA-DRB1 loci to each other.
6. The population of CD4⁺ T cells of any one of the above Clauses, wherein all the CD4⁺ T cells in the population have 2/2 match at the HLA-A locus to each other.
7. The population of CD4⁺ T cells of any one of the above Clauses, wherein all the CD4⁺ T cell in the population have 2/2 match at the HLA-B locus to each other.
8. The population of CD4⁺ T cells of any one of the above Clauses, wherein all the CD4⁺ T cell in the population have 2/2 match at the HLA-C locus to each other.
9. The population of CD4⁺ T cells of any one of the above Clauses, wherein all the CD4⁺ T cells in the population have at least 3/4 or 4/4 match at the HLA-DRB1 and HLA-DQB1 loci with each other.
10. The population of CD4⁺ T cells of any one of the above Clauses, wherein all the CD4⁺ T cells in the population have an A*02 allele.
11. The population of CD4⁺ T cells of any one of the above Clauses, wherein none of the CD4⁺ T cells is immortalized.
12. The population of CD4⁺ T cells of any one of the above Clauses, wherein the exogenous polynucleotide comprises an IL-10-encoding polynucleotide segment operably linked to expression control elements.
13. The population of CD4⁺ T cells of any one of the above Clauses, wherein the IL-10 is a human IL-10.
14. The population of CD4⁺ T cells of any one of Clauses 1-13, wherein the IL-10 is a viral IL-10.
15. The population of CD4⁺ T cells of any one of the above Clauses, wherein the IL-10-encoding polynucleotide segment encodes a protein having the sequence of SEQ ID NO:1.
16. The population of CD4⁺ T cells of Clause 15, wherein the IL-10-encoding polynucleotide segment has the sequence of SEQ ID NO:2.
17. The population of CD4⁺ T cells of any one of Clauses 12-16, wherein the expression control elements drive constitutive expression of the encoded IL-10.
18. The population of CD4⁺ T cells of any one of the above Clauses, wherein the exogenous polynucleotide further comprises a sequence encoding a selection marker.
19. The population of CD4⁺ T cells of Clause 18, wherein the selection marker is ΔNGFR.
20. The population of CD4⁺ T cells of Clause 19, wherein the ΔNGFR has the sequence of SEQ ID NO: 3.
21. The population of CD4⁺ T cells of Clause 19, wherein the exogenous polynucleotide comprises a sequence of SEQ ID NO:4.
22. The population of CD4⁺ T cells of Clause 18, wherein the selection marker is a truncated form of EGFR polypeptide.
23. The population of CD4⁺ T cells of any one of the above Clauses, wherein the exogenous polynucleotide having a sequence of SEQ ID NO: 5.
24. The population of CD4⁺ T cells of any one of Clauses 1-23, wherein the exogenous polynucleotide is integrated into the T cell nuclear genome.
25. The population of CD4⁺ T cells of any one of Clauses 1-23, wherein the exogenous polynucleotide is not integrated into the T cell nuclear genome.
26. The population of CD4⁺ T cells of Clause 24 or 25, wherein the exogenous polynucleotide further comprises lentiviral vector sequences.
27. The population of CD4⁺ T cells of any one of Clauses 1-26, wherein the exogenous polynucleotide is not integrated into the T cell nuclear genome.
28. The population of CD4⁺ T cells of any one of the above Clauses, wherein at least 90% of the CD4⁺ T cells within the population express IL-10.
29. The population of CD4⁺ T cells of Clause 28, wherein at least 95% of the CD4⁺ T cells within the population express IL-10.
30. The population of CD4⁺ T cells of Clause 29, wherein at least 98% of the CD4⁺ T cells within the population express IL-10.
31. The population of CD4⁺ T cells of any one of the above Clauses, wherein the genetically modified CD4⁺ T cells constitutively express at least 100pg IL-10 per 10⁶ of the CD4⁺ T cells/ml of culture medium.
32. The population of CD4⁺ T cells of Clause 31, wherein the genetically modified CD4⁺ T cells constitutively express at least 100 pg, 200 pg, 500pg, 1ng, 5ng, 10ng, or 50ng IL-10 per 10⁶ of the CD4⁺ T cells/ml.
33. The population of CD4⁺ T cells of any one of the above Clauses, wherein the genetically modified CD4⁺ T cells express at least 1ng IL-10 per 10⁶ of the CD4⁺ T cells/ml after activation with anti-CD3 and anti-CD28 antibodies.
34. The population of CD4⁺ T cells of Clause 33, wherein the genetically modified CD4⁺ T cells express at least 2ng, 5ng, 10ng, 100ng, 200ng, or 500ng IL-10 per 10⁶ of the CD4⁺ T cells/ml after activation with anti-CD3 and anti-CD28 antibodies.
35. The population of CD4⁺ T cells of any one of the above Clauses, wherein the genetically modified CD4⁺ T cells express IL-10 at a level at least 5-fold higher than unmodified CD4⁺ T cells.
36. The population of CD4⁺ T cells of Clause 35, wherein the genetically modified CD4⁺ T cells express IL-10 at a level at least 10-fold higher than unmodified CD4⁺ T cells.
37. The population of CD4⁺ T cells of any one of the above Clauses, wherein at least 90% of the CD4⁺ T cells within the population express the selection marker from the exogenous polynucleotide.
38. The population of CD4⁺ T cells of Clause 37, wherein at least 95% of the CD4⁺ T cells within the population express the selection marker from the exogenous polynucleotide.
39. The population of CD4⁺ T cells of Clause 38, wherein at least 98% of the CD4⁺ T cells within the population express the selection marker from the exogenous polynucleotide.
40. The population of CD4⁺ T cells of any one of the above Clauses, wherein the genetically modified CD4⁺ T cells express CD49b.
41. The population of CD4⁺ T cells of any one of the above Clauses, wherein the genetically modified CD4⁺ T cells express LAG-3.
42. The population of CD4⁺ T cells of any one of the above Clauses, wherein the genetically modified CD4⁺ T cells express TGF-β.
43. The population of CD4⁺ T cells of any one of the above Clauses, wherein the genetically modified CD4⁺ T cells express IFNγ.
44. The population of CD4⁺ T cells of any one of the above Clauses, wherein the genetically modified CD4⁺ T cells express GzB.
45. The population of CD4⁺ T cells of any one of the above Clauses, wherein the genetically modified CD4⁺ T cells express perforin.
46. The population of CD4⁺ T cells of any one of the above Clauses, wherein the genetically modified CD4⁺ T cells express CD18.
47. The population of CD4⁺ T cells of any one of the above Clauses, wherein the genetically modified CD4⁺ T cells express CD2.
48. The population of CD4⁺ T cells of any one of the above Clauses, wherein the genetically modified CD4⁺ T cells express CD226.
49. The population of CD4⁺ T cells of any one of the above Clauses, wherein the genetically modified CD4⁺ T cells express IL-22.
50. The population of CD4⁺ T cells of any one of the above Clauses, wherein the CD4⁺ T cells have not been anergized in the presence of peripheral blood mononuclear cells (PBMCs) from a host.
51. The population of CD4⁺ T cells of any one of the above Clauses, wherein the CD4⁺ T cells have not been anergized in the presence of recombinant IL-10 protein, wherein the recombinant IL-10 protein is not expressed from the CD4⁺ T cells.
52. The population of CD4⁺ T cells of any one of the above Clauses, wherein the CD4⁺ T cells have not been anergized in the presence of DC10 cells from a host.
53. The population of CD4⁺ T cells of any one of Clauses 1-52, wherein the CD4⁺ T cells are in a frozen suspension.
54. The population of CD4⁺ T cells of any one of Clauses 1-52, wherein the CD4⁺ T cells are in a liquid suspension.
55. The population of CD4⁺ T cells of Clause 54, wherein the liquid suspension has previously been frozen.
56. A pharmaceutical composition comprising:
   (i) the population of CD4⁺ T cells of any one of the above Clauses; suspended in
   (ii) a pharmaceutically acceptable carrier.
57. A method of making poly-donor CD4^{IL-10} cells, comprising the steps of:
   (i) pooling primary CD4⁺ T cells obtained from at least three different T cell donors; and
   (ii) modifying the pooled CD4⁺ T cells by introducing an exogenous polynucleotide encoding IL-10,
   thereby obtaining the poly-donor CD4^{IL-10} cells.
58. A method of making poly-donor CD4^{IL-10} cells, comprising the steps of:
   (i) obtaining primary CD4⁺ T cells from at least three different T cell donors; and
   (ii) separately modifying each donor's CD4⁺ T cells by introducing an exogenous polynucleotide encoding IL-10, and then
   (iii) pooling the genetically modified CD4⁺ T cells,
   thereby obtaining the poly-donor CD4^{IL-10} cells.
59. The method of Clause 57 or Clause 58, further comprising the step, after step (i) and before step (ii), after step (ii), after step (ii) and before step (iii), or after step (iii) of:
   incubating the primary CD4⁺ T cells in the presence of an anti-CD3 antibody, and anti-CD28 antibody or anti-CD3 antibody and CD28 antibody coated beads.
60. The method of Clause 59, incubating the primary CD4⁺ T cells further in the presence of IL-2.
61. The method of any one of Clauses 57-60, wherein the exogenous polynucleotide is introduced into the primary CD4⁺ T cells using a viral vector.
62. The method of Clause 61, wherein the viral vector is a lentiviral vector.
63. The method of any one of Clauses 57-62, wherein the exogenous polynucleotide comprises a segment encoding IL-10 having the sequence of SEQ ID NO:1.
64. The method of any one of Clauses 53-58, wherein the IL-10-encoding polynucleotide segment has the sequence of SEQ ID NO:2.
65. The method of any one of Clauses 57-64, wherein the exogenous polynucleotide further comprises a segment encoding a selection marker.
66. The method of Clause 65, wherein the encoded selection marker is ΔNGFR.
67. The method of Clause 66, wherein the encoded selection marker has the sequence of SEQ ID NO:3.
68. The method of any one of Clauses 65-67, further comprising the step, after step (ii), of:
   isolating the genetically-modified CD4⁺ T cells expressing the selection marker, thereby generating an enriched population of genetically-modified CD4⁺ T cells.
69. The method of Clause 67, wherein at least 90% or at least 95% of the genetically-modified CD4⁺ T cells in the enriched population express IL-10.
70. The method of Clause 68, wherein at least 98% of the genetically-modified CD4⁺ T cells in the enriched population express IL-10.
71. The method of any one of Clauses 68-69, wherein at least 90% or at least 95% of the genetically-modified CD4⁺ T cells in the enriched population express the selection marker.
72. The method of Clause 70, wherein at least 98% of the genetically-modified CD4⁺ T cells in the enriched population express the selection marker.
73. The method of any one of Clauses 68-72, further comprising the step of incubating the enriched population of genetically-modified CD4⁺ T cells.
74. The method of Clause 73, wherein the step of incubating the enriched population of genetically-modified CD4⁺ T cells is performed in the presence of anti-CD3 antibody and anti-CD28 antibody or CD3 antibody and CD28 antibody coated beads in the presence of IL-2.
75. The method of any one of Clauses 57-74, further comprising the later step of freezing the genetically-modified CD4⁺ T cells.
76. The method of any one of Clauses 57-75, wherein in step (i), the primary CD4⁺ T cells are obtained from three, four, five, six, seven, eight, nine, or ten different T cell donors.
77. The method of any one of Clause 76, wherein the at least three T cell donors have at least 5/10, 6/10, 7/10, 8/10, or 9/10 match at the HLA-A, HLA-B, HLA-C, HLA-DRB1, and HLA-DQB1 loci to each other.
78. The method of any one of Clauses 57-77, wherein the at least three T cell donors have at least 4/8, 5/8, 6/8, 7/8, or 8/8 match at the HLA-A, HLA-B, HLA-C, and HLA-DRB1 loci to each other.
79. The method of any one of Clauses 57-78, wherein the at least three T cell donors have 2/2 match at the HLA-A locus to each other.
80. The method of any one of Clauses 57-79, wherein the at least three T cell donors have 2/2 match at the HLA-B locus to each other.
81. The method of any one of Clauses 57-80, wherein the at least three T cell donors have 2/2 match at the HLA-C locus to each other.
82. The method of any one of Clauses 57-81, wherein the at least three T cell donors have at least 3/4 or 4/4 match at the HLA-DRB1 and HLA-DQB1 loci to each other.
83. The method of any one of Clauses 57-82, wherein each of the at least three T cell donors has an A*02 allele.
84. The method of any one of Clauses 57-83, wherein in step (i), the primary CD4⁺T cells are obtained from one or more frozen stocks.
85. The method of any one of Clauses 57-83, wherein in step (i), the primary CD4⁺T cells are obtained from unfrozen peripheral blood mononuclear cells of the at least three different T cell donors.
86. The method of Clause 85, further comprising the step of isolating CD4⁺ T cells from the peripheral blood mononuclear cells.
87. A method of treating a patient, comprising the step of:
   administering the poly-donor CD4^{IL-10} cells of any one of Clauses 1-55, or the pharmaceutical composition of Clause 56, to a patient in need of immune tolerization.
88. The method of Clause 87, further comprising the preceding step of thawing a frozen suspension of poly-donor CD4^{IL-10} cells.
89. The method of Clause 87 or 88, wherein the poly-donor CD4^{IL-10} cells or the pharmaceutical composition prevents or reduces severity of pathogenic T cell response in the patient.
90. The method of any one of Clauses 87-89, further comprising the step of administering mononuclear cells to the patient.
91. The method of Clause 90, wherein the poly-donor CD4^{IL-10} cells or the pharmaceutical composition and the mononuclear cells are administered concurrently.
92. The method of Clause 90, wherein the mononuclear cells are administered either prior to or subsequent to administration of the poly-donor CD4^{IL-10} cells or the pharmaceutical composition.
93. The method of any one of Clauses 87-92, further comprising the step of:
   administering hematopoietic stem cells (HSC) of an HSC donor to the patient either prior to or subsequent to administration of the pooled donor CD4^{IL-10} cells or pharmaceutical composition.
94. The method of Clause 93, wherein the HSC donor is partially HLA-mismatched to the patient.
95. The method of Clause 94, wherein the HSC donor has less than 5/10, 6/10, 7/10, 8/10, 9/10 or 10/10 match at the HLA-A, HLA-B, HLA-C, HLA-DRB1, and HLA-DQB1 loci to the patient.
96. The method of Clause 94, wherein the HSC donor has less than 4/8, 5/8, 6/8, 7/8, or 8/8 match at the HLA-A, HLA-B, HLA-C, and HLA-DRB1 loci to the patient.
97. The method of Clause 94, wherein the HSC donor has less than 2/2 match at the HLA-A, HLA-B, or HLA-C locus to the patient.
98. The method of Clause 94, wherein the HSC donor has less than 3/4 or 4/4 match at the HLA-DRB1 and HLA-DQB1 loci to the patient.
99. The method of any one of Clauses 87-98, wherein one or more of the T cell donors are HLA-mismatched or partially HLA-mismatched to the patient.
100. The method of Clause 99, wherein one or more of the T cell donors have less than 5/10, 6/10, 7/10, 8/10, 9/10 or 10/10 match at the HLA-A, HLA-B, HLA-C, HLA-DRB1, and HLA-DQB1 loci to the patient.
101. The method of Clause 99, wherein one or more of the T cell donors have less than 4/8, 5/8, 6/8, 7/8, or 8/8 match at the HLA-A, HLA-B, HLA-C, and HLA-DRB1 loci to the patient.
102. The method of Clause 99, wherein one or more of the T cell donors have less than 2/2 match at the HLA-A, HLA-B, or HLA-C locus to the patient.
103. The method of Clause 99, wherein one or more of the T cell donors have less than 2/4, 3/4 or 4/4 match at the HLA-DRB1 and HLA-DQB1 loci to the patient.
104. The method of any one of Clauses 87-103, wherein one or more of the T cell donors are HLA-mismatched or partially HLA-mismatched with the HSC donor.
105. The method of Clause 104, wherein one or more of the T cell donors have less than 5/10, 6/10, 7/10, 8/10, 9/10 or 10/10 match at the HLA-A, HLA-B, HLA-C, HLA-DRB1, and HLA-DQB1 loci to the HSC donor.
106. The method of Clause 104, wherein one or more of the T cell donors have less than 4/8, 5/8, 6/8, 7/8, or 8/8 match at the HLA-A, HLA-B, HLA-C, and HLA-DRB1 loci to the HSC donor.
107. The method of Clause 104, wherein one or more of the T cell donors have less than 2/2 match at the HLA-A, HLA-B, or HLA-C locus to the HSC donor.
108. The method of Clause 104, wherein one or more of the T cell donors have less than 3/4 or 4/4 match at the HLA-DRB1 and HLA-DQB1 loci to the HSC donor.
109. The method of any one of Clauses 79-108, wherein the poly-donor CD4^{IL-10} cells or the pharmaceutical composition prevents or reduces severity of GvHD by the transplanted hematopoietic stem cells.
110. The method of any one of Clauses 93-109, wherein the poly-donor CD4^{IL-10} cells or the pharmaceutical composition prevents or reduces severity of pathogenic response of lymphoid cells from the transplanted hematopoietic cells.
111. The method of any one of Clauses 87-110, wherein the patient has neoplastic cells.
112. The method of Clause 111, wherein the neoplastic cells expresses CD13, HLA-class I and CD54.
113. The method of any one of Clauses 111-112, wherein the neoplastic cells expresses CD112, CD58, or CD155.
114. The method of any one of Clauses 111-113, wherein the patient has a cancer, optionally wherein the cancer is a solid or hematological neoplasm.
115. The method of any one of Clauses 87-114 , wherein the patient has a cancer selected from the group consisting of: Adrenal Cancer, Anal Cancer, Bile Duct Cancer, Bladder Cancer, Bone Cancer, Brain/CNS Tumors In Adults, Brain/CNS Tumors In Children, Breast Cancer, Breast Cancer In Men, Cancer of Unknown Primary, Castleman Disease, Cervical Cancer, Colon/Rectum Cancer, Endometrial Cancer, Esophagus Cancer, Ewing Family Of Tumors, Eye Cancer, Gallbladder Cancer, Gastrointestinal Carcinoid Tumors, Gastrointestinal Stromal Tumor (GIST), Gestational Trophoblastic Disease, Hodgkin Disease, Kaposi Sarcoma, Kidney Cancer, Laryngeal and Hypopharyngeal Cancer, Leukemia, Acute Lymphocytic (ALL), Acute Myeloid (AML, including myeloid sarcoma and leukemia cutis), Chronic Lymphocytic (CLL), Chronic Myeloid (CML) Leukemia, Chronic Myelomonocytic (CMML), Leukemia in Children, Liver Cancer, Lung Cancer, Lung Cancer with Non-Small Cell, Lung Cancer with Small Cell, Lung Carcinoid Tumor, Lymphoma, Lymphoma of the Skin, Malignant Mesothelioma, Multiple Myeloma, Myelodysplastic Syndrome, Nasal Cavity and Paranasal Sinus Cancer, Nasopharyngeal Cancer, Neuroblastoma, Non-Hodgkin Lymphoma, Non-Hodgkin Lymphoma In Children, Oral Cavity and Oropharyngeal Cancer, Osteosarcoma, Ovarian Cancer, Pancreatic Cancer, Penile Cancer, Pituitary Tumors, Prostate Cancer, Retinoblastoma, Rhabdomyosarcoma, Salivary Gland Cancer, Sarcoma - Adult Soft Tissue Cancer, Skin Cancer, Skin Cancer - Basal and Squamous Cell, Skin Cancer - Melanoma, Skin Cancer - Merkel Cell, Small Intestine Cancer, Stomach Cancer, Testicular Cancer, Thymus Cancer, Thyroid Cancer, Uterine Sarcoma, Vaginal Cancer, Vulvar Cancer, Waldenstrom Macroglobulinemia, and Wilms Tumor.
116. The method of Clause 115, wherein the patient has a myeloid cancer.
117. The method of Clause 115, wherein the patient has AML or CML.
118. The method of any one of Clauses 87, wherein the patient has an inflammatory or autoimmune disease.
119. The method of Clause 118, wherein the inflammatory or autoimmune disease is selected from the group consisting of: type-1 diabetes, autoimmune uveitis, rheumatoid arthritis, psoriasis, psoriatic arthritis, multiple sclerosis, systemic lupus, inflammatory bowel disease, Addison's disease, Graves' disease, Sjögren's syndrome, Hashimoto's thyroiditis, myasthenia gravis, autoimmune vasculitis, pernicious anemia, ulcerative colitis, bullous diseases, scleroderma, and celiac disease.
120. The method of Clause 119, wherein the inflammatory or autoimmune disease is Crohn's disease, ulcerative colitis, celiac disease, type-1 diabetes, lupus, psoriasis, psoriatic arthritis, or rheumatoid arthritis.
121. The method of any one of Clauses 87-113 or 118-120, wherein the patient has a disease or disorder involving hyperactivity of NLPR3 inflammasome.
122. The method of any one of Clauses 87-113 or 118-121, wherein the patient has type 2 diabetes, neurodegenerative diseases, cardiovascular diseases or inflammatory bowel disease.
123. The method of any one of Clauses 87-113 or 118-121, wherein the patient has a disease or disorder involving increased IL-1β production by activated monocytes, macrophages or dendritic cells
124. The method of any one of Clauses 87-113 or 118-121, wherein the patient has a disease or disorder involving increased IL-18 production by activated monocytes, macrophages or dendritic cells.
125. The method of any one of Clauses 87-113 or 118-121, wherein the patient has a disease or disorder involving increased mature caspase 1 production by activated monocytes, macrophages or dendritic cells.
126. The method of any one of Clauses 87-113, wherein the patient has an allergic or atopic disease.
127. The method of Clause 126, wherein the allergic or atopic disease is selected from the group consisting of : asthma, atopic dermatitis, and rhinitis.
128. The method of any one of Clauses 87-113, wherein the patient has a food allergy.
129. The method of any one of Clauses 87-113, further comprising the step of organ transplantation to the patient, either prior to or subsequent to administration of the population of CD4⁺ T cells or the pharmaceutical composition.
130. The method of Clause 129, wherein the poly-donor CD4^{IL-10} cells or the pharmaceutical composition prevents or reduces severity of host rejection of the organ transplantation.
131. The method of any one of Clauses 87-113, further comprising the step of transplanting iPS cell-derived cells or tissues to the patient, either prior to or subsequent to administration of the population of CD4⁺ T cells or the pharmaceutical composition.
132. The method of Clause 131, wherein poly-donor CD4^{IL-10} cells or the pharmaceutical composition prevents or reduces severity of host rejection of the cell transplantation.
133. The method of any one of Clauses 87-113, further comprising the step of administering a recombinant AAV to the patient, either prior to or subsequent to administration of the poly-donor CD4^{IL-10} cells or the pharmaceutical composition.
134. The method of Clause 133, wherein the poly-donor CD4^{IL-10} cells or the pharmaceutical composition reduces immune responses against the recombinant AAV.
135. The method of any one of Clauses 87-113, wherein the patient has an excessive immune response against viral or bacterial infection.
136. The method of Clause 135, wherein the patient has a coronavirus infection.
137. The method of any one of Clauses 87-136, further comprising the step of detecting the selection marker in a biological sample obtained from the patient, thereby detecting presence or absence of poly-donor CD4^{IL-10} T cells.
138. The method of Clause 137, wherein the biological sample is a biopsy or blood from the patient.
139. A method of treating a patient with a malignancy, comprising:
   administering an allo-HSCT graft to the patient, and
   administering a therapeutically effective amount of poly-donor CD4^{IL-10} cells.
140. The method of Clause 139, wherein none of the donors of the CD4^{IL-10} cells in the poly-donor CD4^{IL-10} cells is the donor of the HSCT graft.
141. A method of treating a hematological cancer, comprising:
   administering to a hematological cancer patient an amount of poly-donor CD4^{IL-10} cells sufficient induce anti-cancer effect,
   wherein the poly-donor CD4^{IL-10} cells comprise CD4⁺ T cells obtained from at least three different T cell donors and genetically modified by vector-mediated gene transfer of the coding sequence of human IL-10 under control of a constitutive promoter.
142. The method of Clause 141, further comprising the step of administering allo HSCT graft to the patient prior to or subsequent to administration of the poly-donor CD4^{IL-10} cells.
143. The method of Clause 142, wherein the amount of poly-donor CD4^{IL-10} cells is further sufficient to suppress graft versus host disease (GvHD) without suppressing graft versus lekemia (GvL) or graft versus tumor (GvT) efficacy of the allo HSCT.
144. The method of any one of Clauses 141-143, wherein the hematological cancer is a myeloid leukemia.
145. The method of any one of Clauses141-142, wherein the poly-donor CD4^{IL-10} cells target and kill cancer cells that express CD13.
146. The method of any one of Clauses 141-145, wherein the poly-donor CD4^{IL-10} cells target and kill cancer cells that express HLA-class I.
147. The method of any one of Clauses 141-146, wherein the myeloid leukemia is acute myeloid leukemia (AML).
148. The method of any one of Clauses 141-147, wherein the allo-HSCT graft is obtained from a related or unrelated donor with respect to the recipient.
149. The method of any one of Clauses 141-148, wherein the poly-donor CD4^{IL-10} cells are non-autologous to the recipient.
150. The method of any one of Clauses 141-148, wherein the poly-donor CD4IL-10 cells are allogeneic to the recipient.
151. The method of any one of Clauses 141-148, wherein the poly-donor CD4^{IL-10} cells are not anergized to host allo-antigens prior to administration to the host.
152. The method of any one of Clauses 141-148, wherein the poly-donor CD4^{IL-10} cells are Tr1-like cells.
153. The method of any one of Clauses 141-148, wherein the poly-donor CD4^{IL-10} cells are polyclonal.
154. The method of any one of Clauses 141-148, wherein the poly-donor CD4^{IL-10} cells are polyclonal and non-autologous to the recipient.
*155.* The method of any one of Clauses 141-148, wherein the poly-donor CD4^{IL-10} cells are isolated from at least three donors prior to being genetically modified.
156. The method of Clause 155, wherein none of the at least three donors is the same donor as the allo-HSCT donor.
157. The method of any one of Clauses 141-156, wherein the allo-HSCT graft is obtained from a matched or mismatched donor with respect to the recipient.
158. The method of any one of Clauses 141-157, wherein the poly-donor CD4^{IL-10} cells target and kill cells that express CD54.
159. The method of any one of Clauses 141-158, wherein the poly-donor CD4^{IL-10} cells target and kill cancer cells that express HLA-class I and CD54.
160. The method of any one of Clauses 141-159, wherein the poly-donor CD4^{IL-10} cells target and kill cancer cells that express CD112.
161. The method of any one of Clauses 141-160, wherein the poly-donor CD4^{IL-10} cells target and kill cancer cells that express CD58.
162. The method of any one of Clauses 141-161, wherein the poly-donor CD4^{IL-10} cells target and kill cancer cells in the host.
163. A method of treating a hematological cancer by allogeneic hematopoietic stem cell transplant (allo-HSCT), comprising:
   administering allo-HSCT graft to a subject (host);
   administering to the allo-HSCT recipient (host) an amount of poly-donor CD4^{IL-10} cells sufficient to suppress graft-versus-host disease (GvHD) without suppressing graft-versus-leukemia (GvL) or graft-versus-tumor (GvT) efficacy of the allo-HSCT graft,
   wherein the poly-donor CD4^{IL-10} cells comprise CD4⁺ T cells obtained from at least three different T cell donors and genetically modified by vector-mediated gene transfer of the coding sequence of human IL-10 under control of a constitutive promoter;
   wherein the poly-donor CD4^{IL-10} cells are non-autologous to the recipient and non-autologous to the allo-HSCT donor;
   wherein the poly-donor CD4^{IL-10} cells are not anergized to host allo-antigens prior to administration to the host; and
   wherein the poly-donor CD4^{IL-10} cells are polyclonal and Tr1-like.
164. A method of treating a hematological cancer by allogeneic hematopoietic stem cell transplant (allo-HSCT), comprising:
   administering allo-HSCT graft to a subject (host);
   administering to the allo-HSCT recipient (host) an amount of poly-donor CD4^{IL-10} cells sufficient to suppress graft-versus-host disease (GvHD) without suppressing graft-versus-leukemia (GvL) or graft-versus-tumor (GvT) efficacy of the allo-HSCT graft,
   wherein the poly-donor CD4^{IL-10} cells comprise CD4⁺ T cells obtained from at least three different T cell donors and genetically modified by vector-mediated gene transfer of the coding sequence of human IL-10 under control of a constitutive promoter;
   wherein the poly-donor CD4^{IL-10} cells target and kill cancer cells in the host;
   wherein the wherein the poly-donor CD4^{IL-10} cells are not anergized to host allo-antigens prior to administration to the host; and
   wherein the poly-donor CD4^{IL-10} cells are non-autologous to the recipient, and polyclonal, and are Tr1-like.

## Claims

1. A population of CD4⁺ T cells that have been genetically modified to comprise an exogenous polynucleotide encoding IL-10, wherein:
(a) the CD4⁺ T cells were obtained from at least three different T cell donors (poly-donor CD4^{IL-10} cells); and
(b) the exogenous polynucleotide is integrated into the nuclear genomes of the population of CD4⁺ T cells, wherein the exogenous polynucleotide comprises an IL-10-encoding polynucleotide segment operably linked to an expression control element.

2. The population of CD4⁺ T cells of claim 1, wherein:
(a) the CD4⁺ T cells in the population collectively have six, seven, eight, nine, ten, eleven, twelve, or more different HLA haplotypes; or
(b) the CD4⁺ T cells in the population have:
(i) at least 5/10, 6/10, 7/10, 8/10, or 9/10 match at the HLA-A, HLA-B, HLA-C, HLA-DRB1, and HLA-DQB1 loci to each other;
(ii) at least 4/8, 5/8, 6/8, 7/8, or 8/8 match at the HLA-A, HLA-B, HLA-C, and HLA-DRB1 loci to each other;
(iii) 2/2 match at the HLA-A locus to each other;
(iv) 2/2 match at the HLA-B locus to each other;
(v) 2/2 match at the HLA-C locus to each other; and/or
(vi) all the CD4⁺ T cells in the population have at least 3/4 or 4/4 match at the HLA-DRB1 and HLA-DQB 1 loci with each other.

3. The population of CD4⁺ T cells of claim 1 or claim 2, wherein the exogenous polynucleotide further comprises:
(a) a sequence encoding a selection marker, optionally wherein the selection marker is ΔNGFR, optionally wherein at least 90%, at least 95%, or at least 98% of the CD4⁺ T cells within the population express the selection marker from the exogenous polynucleotide; and/or
(b) lentiviral vector sequences.

4. The population of CD4⁺ T cells of any one of the above claims, wherein:
(a) at least 90% of the CD4⁺ T cells within the population express IL-10
(b) the genetically modified CD4⁺ T cells constitutively express at least 100pg, 200pg, 500pg, 1ng, 5ng, 10ng, or 50ng IL-10 per 10⁶ of the CD4⁺ T cells/ml of culture medium;
(c) the genetically modified CD4⁺ T cells express at least 1ng, 2ng, 5ng, 10ng, 100ng, 200ng, or 500ng IL-10 per 10⁶ of the CD4⁺ T cells/ml after activation with anti-CD3 and anti-CD28 antibodies; and/or
(d) the genetically modified CD4⁺ T cells express CD49b, LAG-3, TGF-β, IFNγ, GzB, perforin, CD18, CD2, CD226, and/or IL-22.

5. The population of CD4⁺ T cells of any one of the above claims, wherein the CD4⁺ T cells are in:
(a) a frozen suspension; or
(b) a liquid suspension, optionally wherein the liquid suspension has previously been frozen.

6. A method of making poly-donor CD4^{IL-10} cells, comprising the steps of:
(i) modifying primary CD4⁺ T cells which have been obtained from at least three different T cell donors, wherein each donor's CD4⁺ T cells are separately modified by introducing an exogenous polynucleotide encoding IL-10 using a viral vector, and pooling the genetically modified CD4⁺ T cells; or
(ii) pooling primary CD4+ T cells obtained from at least three different T cell donors; and introducing an exogenous polynucleotide encoding IL-10.

7. The method of claim 6, further comprising the step of:
incubating the primary CD4⁺ T cells in the presence of:
(a) an anti-CD3 antibody, and anti-CD28 antibody; or
(b) anti-CD3 antibody and anti-CD28 antibody coated beads,
optionally wherein the method further comprises incubating the primary CD4⁺ T cells in the presence of IL-2.

8. The method of claim 6 or claim 7, wherein the exogenous polynucleotide further comprises a segment encoding a selection marker, optionally wherein the encoded selection marker is ΔNGFR;
optionally wherein the method further comprises the step of:
isolating the genetically-modified CD4⁺ T cells expressing the selection marker.

9. The method of any one of claims 6-8, wherein the primary CD4⁺ T cells are obtained from one or more frozen stocks.

10. The population of CD4⁺ T cells according to any one of claims 1-5 for use in a method of treating a patient in need of immune tolerization, wherein the method comprises the step of:
administering the poly-donor CD4^{IL-10} cells to the patient.

11. The population of CD4⁺ T cells for use according to claim 10, wherein the method further comprises the step of:
administering hematopoietic stem cells (HSC) of an HSC donor to the patient either prior to or subsequent to administration of the pooled donor CD4^{IL-10} cells.

12. The population of CD4⁺ T cells for use according to claim 10, wherein the patient has:
(a) an inflammatory or autoimmune disease, optionally wherein the inflammatory or autoimmune disease is selected from the group consisting of type-1 diabetes, Crohn's disease, autoimmune uveitis, rheumatoid arthritis, psoriasis, psoriatic arthritis, multiple sclerosis, systemic lupus, inflammatory bowel disease, Addison's disease, Graves' disease, Sjögren's syndrome, Hashimoto's thyroiditis, myasthenia gravis, autoimmune vasculitis, pernicious anemia, ulcerative colitis, bullous diseases, scleroderma, and celiac disease;
(b) a disease or disorder involving hyperactivity of NLPR3 inflammasome;
(c) type 2 diabetes, a neurodegenerative disease, a cardiovascular disease or inflammatory bowel disease;
(d) an allergic or atopic disease, optionally wherein the allergic or atopic disease is selected from the group consisting of: asthma, atopic dermatitis, and rhinitis; and/or
(e) Crohn's disease.

13. The population of CD4⁺ T cells for use according to claim 10, wherein the method further comprises the step of organ transplantation to the patient, either prior to or subsequent to administration of the population of CD4⁺ T cells.

14. The population of CD4⁺ T cells according to any one of claims 1-5 for use in a method of treating a hematological cancer, the method comprising:
administering to a hematological cancer patient an amount of poly-donor CD4^{IL-10} cells sufficient to induce anti-cancer effect,
wherein the poly-donor CD4^{IL-10} cells are genetically modified by vector-mediated gene transfer of the coding sequence of human IL-10 under control of a constitutive promoter.

15. The population of CD4⁺ T cells for use according to claim 14, wherein:
(a) the poly-donor CD4^{IL-10} cells are administered to the patient prior to or subsequent to administration of an allogenic hematopoietic stem cell transplant (allo HSCT), and the amount of poly-donor CD4^{IL-10} cells administered to the patient is sufficient to suppress GvHD without suppressing graft versus leukemia (GvL) or graft versus tumor (GvT) efficacy of the allo HSCT; and/or
(b) the hematological cancer is a myeloid leukemia.
